# EUROPEAN PATENT APPLICATION

(11) **EP 4 752 226 A1**
(43) Date of publication of application: **03.06.2026**
(21) Application number: 24216316.0
(22) Date of filing: 28.11.2024
(51) Int. Cl.: C12N 15/86

(54) **PLASMID SYSTEM FOR AAV PRODUCTION**

(71) Applicant: Ascend Advanced Therapies GmbH, 82152 München (DE)
(72) Inventor: Hörer, Markus, Munich (DE); Schulze, Andreas, Munich (DE); Sonntag, Florian, Munich (DE)
(74) Representative: Kosti, Vasiliki

(57) **Abstract**

The present invention relates to helper plasmids and two-plasmid systems for producing recombinant AAV (rAAV) vectors. The invention further relates to methods of using, or uses of the helper plasmids and two-plasmid systems of the invention. The present invention also relates to a helper plasmid which does not comprise a cap gene encoding a functional set of Cap proteins and which does comprise at least one rep gene and at least one helper virus gene.

## Description

### FIELD OF THE INVENTION

The present invention relates to helper plasmids and two-plasmid systems for producing recombinant AAV (rAAV) vectors. The invention further relates to methods of using, or uses of the helper plasmids and two-plasmid systems of the invention.

### BACKGROUND

Recombinant adeno-associated virus (rAAV) vectors are a leading gene delivery platform, have remarkable potential for gene therapy due to their promising safety profile and their ability to transduce many tissues *in vivo,* and several rAAV-mediated therapies have recently been approved (Ameri; 2018; J. Curr. Ophthalmol. 30, 1-2; Ylä-Herttuala; 2012; Mol. Ther.20:1831-1832). However, despite these advances in the clinic, rAAV vector manufacturing still remains a challenge. As an example, a phase 1/2 trial for hemophilia B required over 400 ten-layer cell stacks to generate sufficient material for six patients (Allay et al; 2011; Hum. Gene Ther. 22: 595-604). Although the trial was successful, this study highlights the need for new methods to improve vector generation. Increased production efficiency will reduce manufacturing costs, improve patient access, and make this emerging modality more feasible for large disease indications. Higher gene therapy vector loads are crucial to account for larger patient sizes, systemic diseases, or diseases in less accessible body sites. Thus, beyond the efforts in improving safety, alternative host cells, and alternative viral helpers, an additional area of rAAV research has been in scale-up, moving production from laboratory scale to industrial scale (Clement; 2026; Mol Ther Methods Clin Dev; 3:16002). An important focus of the AAV vector development field has been to fine-tune the manufacturing process to augment the vector yield, purity, or its potency so that dose of vectors required per patient is low. AAV transduction requires a reasonable multiplicity of infection of ~10³ to 10⁵ vector genomes (vg) per cell depending on cell type. However, for a clinical trial, an estimated 10¹² to 10¹⁴ viral particles are essential to be efficacious during gene transfer. This high vector dose requirement in the clinical settings has underscored the need for optimizing vector production.

WO2020/208379 A1 describes a two-plasmid system for AAV production that uses a 'trans-split configuration', i.e. a plasmid configuration in which the rep and cap genes are not both present on a single plasmid. In this configuration the helper plasmid comprises at least one rep gene gene encoding a functional Rep 52 protein, at least one gene encoding a functional Rep 40 protein, and a gene encoding a functional Rep 68 protein, but does not encode a Rep78 protein. The vector plasmid of the system comprises a cap gene encoding at least one functional Cap protein; or at least one cap gene promoter, a cloning site operably linked to the cap gene promoter, and an expression cassette flanked on at least one side by an ITR, and the expression cassette comprises a transgene operably linked to at least one regulatory control element. Such a plasmid system appears to be particularly suitable for AAV production using adherent host cell lines.

WO 2022/079429 A1 describes a two-plasmid system for AAV production that uses a 'trans-split configuration'. In this system, the helper plasmid encodes a rep gene which expresses Rep 78 at a reduced level compared to a wild type rep gene, while the vector plasmid comprises a cap gene encoding at least one functional Cap protein; or at least one cap gene promoter, a cloning site operably linked to the cap gene promoter, and an expression cassette flanked on at least one side by an ITR, and the expression cassette comprises a transgene operably linked to at least one regulatory control element. Such a plasmid system appears to be also particularly suitable for AAV production using adherent host cell lines.

In order to satisfy the current demand of rAAV vector material for clinical trials and market supply, the following goals have yet to be achieved: (a) improved safety and quality profile of the rAAV vectors to meet the regulatory demands on vector quality; (b) improved economics of rAAV manufacture in terms of cost of starting materials for a given manufacturing campaign as well as cost of switching between campaigns; (c) high rAAV production yields in the large scale e.g in bioreactor cultures for large scale industrial production; and (d) control over the proportion of produced rAAV particles containing ("full") or lacking ("empty") a complete recombinant vector genome.

The inventors have designed rAAV vectors which meet the above needs. In particular, the inventors have created rAAV vectors that have an improved safety profile owing to low levels of impurities to as well as a decreased capacity for rcAAV (replication competent AAV) formation. The inventors have also achieved high yields of rAAV by reducing rep78 expression and at the same time allowing effective production in large scale suspension cell cultures e.g in bioreactor cultures.

### SUMMARY

The present invention relates to a helper plasmid for manufacturing rAAV, which does not comprise a cap gene and which does comprise a rep gene and which expresses Rep 78. This rep 78 positive helper plasmid allows for a high yield of rAAV expression, in particular during AAV production in suspension cells, and enhanced safety and quality attributes in addition to other surprising advantages as disclosed herein.

In a first aspect, the present invention provides a helper plasmid which does not comprise a cap gene encoding a functional set of Cap proteins and which does comprise at least one Adeno-associated virus (AAV) rep gene and at least one helper virus gene, wherein the at least one rep gene comprises a rep 78 cassette comprising a nucleotide sequence encoding a functional Rep 78 protein and a transcription regulatory element or a rep 78 cassette comprising a nucleotide sequence encoding a functional Rep 78 protein and a functional Rep 68 protein and a transcription regulatory element, and wherein said rep 78 cassette comprises an ATG start codon at the beginning of the nucleotide sequence encoding a Rep 78 protein or both a Rep 78 and a Rep 68 protein.

In a second aspect, the present invention provides a helper plasmid which does not comprise a cap gene encoding a functional set of Cap proteins and which does comprise at least one rep gene and at least one helper virus gene, wherein the at least one rep gene comprises a rep 78 cassette comprising a nucleotide sequence encoding a functional Rep 78 protein and a transcription regulatory element or a rep 78 comprising a nucleotide sequence encoding a functional Rep 78 protein and a functional Rep 68 protein and a transcription regulatory element, and wherein and said rep 78 cassette comprises an ATG start codon at the beginning of the nucleotide sequence encoding a functional Rep 78 protein or both a Rep 78 and a Rep 68 protein,characterised in that said helper plasmid promotes production of recombinant AAV at a higher yield in suspension cells compared to a helper plasmid wherein the at least one rep gene does not encode a Rep 78 protein and/or promotes production of recombinant AAV comprising a lower level of nucleic acid impurities compared to a helper plasmid comprising at least one rep gene which does not encode a functional Rep 78 protein.

In a third aspect, the present invention provides a two-plasmid system comprising the helper plasmid of the invention and a vector plasmid.

In a fourth aspect, the present invention provides a host cell comprising the helper plasmid of the invention or two-plasmid system of the invention.

In a fifth aspect, the present invention provides a use of the helper plasmid or the two-plasmid system of the invention for producing a recombinant AAV preparation.

In a sixth aspect, the present invention provides a method for producing a recombinant AAV preparation comprising:
(a) obtaining the helper plasmid or the two-plasmid system of the invention;
(b) transfecting a host cell with the helper plasmid or the two-plasmid system of the invention; and
(c) culturing the host cell in suspension under conditions suitable for recombinant AAV production.

In a seventh aspect, the present invention provides a use of the helper plasmid or the two-plasmid system of the invention for:
(i) increasing, optimising and/or maximising the yield of recombinant AAV produced during recombinant AAV production;
(ii) increasing the yield of recombinant AAV produced during recombinant AAV production compared to an equivalent use of a rep 78 negative helper plasmid or a two-plasmid system comprising a rep 78 negative helper plasmid;
(iii) controlling and/or maximising the ratio of full to total particles produced during recombinant AAV production;
(iv) reducing and/or minimising the level of nucleic acid impurities (normalised to vector genome level) produced during recombinant AAV production;
(v) reducing the level of nucleic acid impurities (normalised to vector genome level) produced during recombinant AAV production compared to an equivalent use of a rep 78 negative helper plasmid or a two-plasmid system comprising a rep 78 negative helper plasmid;
(vi) increasing the potency of recombinant AAV produced during recombinant AAV production; and/or
(vii) increasing the potency of recombinant AAV produced during recombinant AAV production compared to an equivalent use of a rep 78 negative helper plasmid or a two-plasmid system comprising a rep 78 negative helper plasmid.

In an eighth aspect, the present invention provides a method for:
(i) increasing, optimising and/or maximising the yield of recombinant AAV produced during recombinant AAV production;
(ii) increasing the yield of recombinant AAV produces during recombinant AAV production compared to an equivalent method using a rep 78 negative helper plasmid or a two-plasmid system comprising a rep 78 negative helper plasmid;
(iii) controlling and/or maximising the ratio of full to total particles produced during recombinant AAV production;
(iv) reducing and/or minimising the level of nucleic acid impurities (normalised to vector genome level) produced during recombinant AAV production;
(v) reducing the level of nucleic acid impurities (normalised to vector genome level) produced during recombinant AAV production compared to an equivalent method using rep 78 negative helper plasmid or a a two-plasmid system comprising a rep 78 negative helper plasmid;
(vi) increasing the potency of recombinant AAV produced during recombinant AAV production; and/or
(vii) increasing the potency of recombinant AAV produced during recombinant AAV production compared to an equivalent method using a two-plasmid system comprising rep 78 negative helper plasmid or a a rep 78 negative helper plasmid,
   comprising:
   a) obtaining the helper plasmid or the two-plasmid system of the invention;
   (b) transfecting a host cell with the helper plasmid or the two-plasmid system of the invention; and
   (c) culturing the host cell in suspension under conditions suitable for recombinant AAV production.

In a ninth aspect, the present invention provides a method for reducing and/or minimising the level of replication competent AAV (rcAAV) produced during recombinant AAV production comprising:
(a) obtaining the two-plasmid system, or the helper plasmid of the invention;
(b) transfecting a host cell with the two-plasmid system, or the helper plasmid of the invention; and
(c) culturing the host cell in suspension under conditions suitable for recombinant AAV production.

In a tenth aspect, the present invention provides a method for reducing and/or minimising the level of pseudo-wild type replication competent AAV (rcAAV) produced during recombinant AAV production comprising:
(a) obtaining the two-plasmid system, or the helper plasmid of the invention;
(b) transfecting a host cell with the two-plasmid system, or the helper plasmid of the invention; and
(c) culturing the host cell in suspension under conditions suitable for recombinant AAV production.

In an eleventh aspect, the present invention provides a method for producing recombinant AAV comprising a reduced and/or minimised level of replication competent AAV (rcAAV) or pseudo-wild type replication competent AAV (rcAAV).

In a twelfth aspect, the present invention provides a recombinant AAV preparation obtainable by the method of the invention.

In a thirteenth aspect, the present invention provides a recombinant AAV preparation obtained by the method of the invention.

### BRIEF DESCRIPTION OF THE DRAWINGS

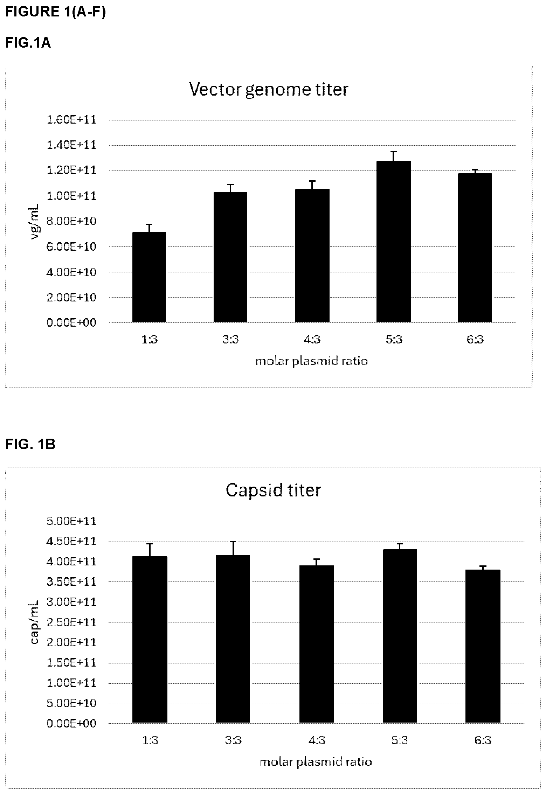

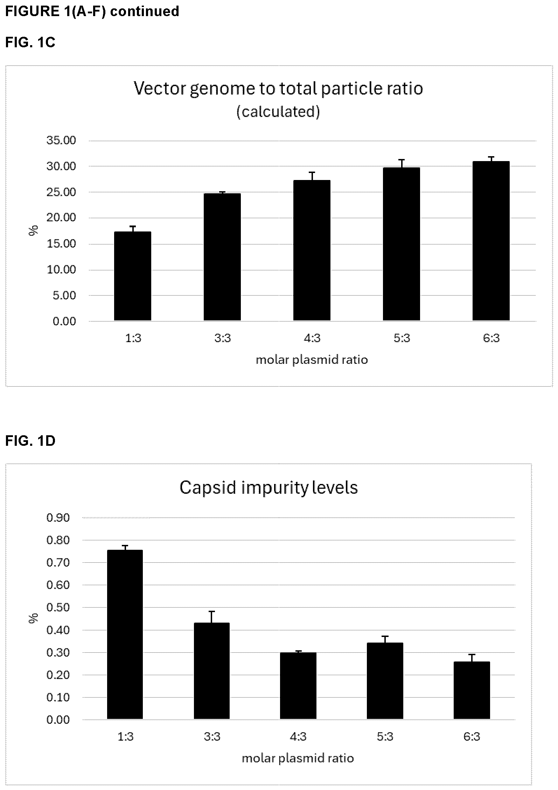

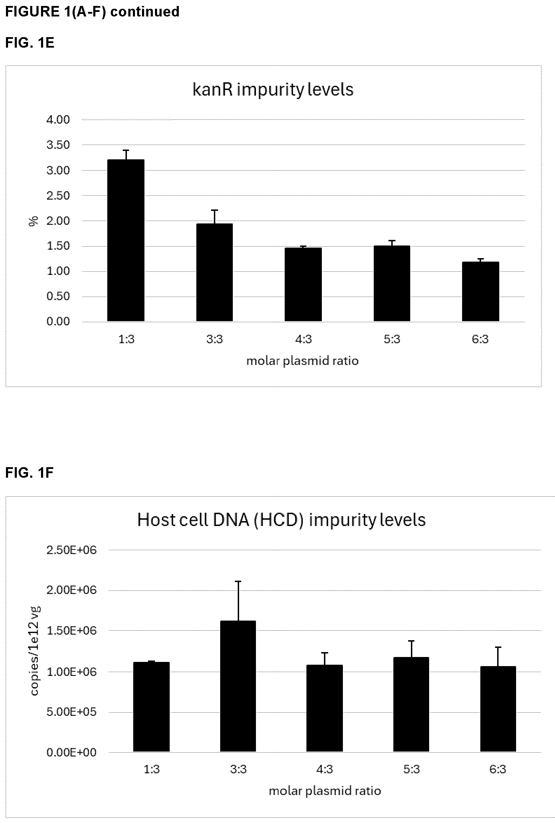

**Figure 1****(A-F)** provides the results of analysis of rAAV produced and assayed as described in Example 6. Fig. 1A: Vector genome yield was determined for rAAV produced using the Rep78pos two plasmid system using different molar helper:vector plasmid ratios (1:3, 3:3, 4:3, 5:3, and 6:3). The vector plasmid used comprises the engineered cap gene and the expression cassette encoding for a marker transgene. Vector genome (vg) titre per ml was measured by qPCR. The vector genome yield was determined 72 hrs after transfection. Fig. 1B: Yield of rAAV particles ("capsid yield") was determined for rAAV produced using the Rep78pos two plasmid system using different molar helper:vector plasmid ratios (1:3, 3:3, 4:3, 5:3, and 6:3). The number of rAAV particles per ml was measured by ELISA. The capsid yield was determined 72 h after transfection. Fig. 1C: Vector genome to total particle ratio was determined for rAAV produced using the Rep78pos two plasmid system using different molar helper:vector plasmid ratios (1:3, 3:3, 4:3, 5:3, and 6:3). The vector genome to total particle ratio was determined using the vector genome yield and capsid yield as determined for (A) to (B) above. The vector genome to total particle ratio was determined 72 h after transfection. The vector genome to total particle ratio is expressed as a fold change compared to the helper plasmid to vector plasmid ratio with the least helper plasmid, which is the molar plasmid ratio of 1:3 (helper:vector). For example, the vector genome to total particle ratio produced using the Rep78pos two plasmid system at a molar plasmid ratio of 3:3 (helper:vector) has been divided by the vector genome to total particle ratio produced using the Rep78pos two plasmid system at a molar plasmid ratio of 1:3 (helper:vector) to arrive at the fold change value plotted. Levels of capsid, KanR, and Host Cell DNA impurities were determined for rAAV produced using the Rep78pos two plasmid system 72h after transfection and are shown in Fig. 1D, Fig. 1E, Fig. 1F in different molar helper:vector plasmid ratios (1:3, 3:3, 4:3, 5:3, and 6:3).

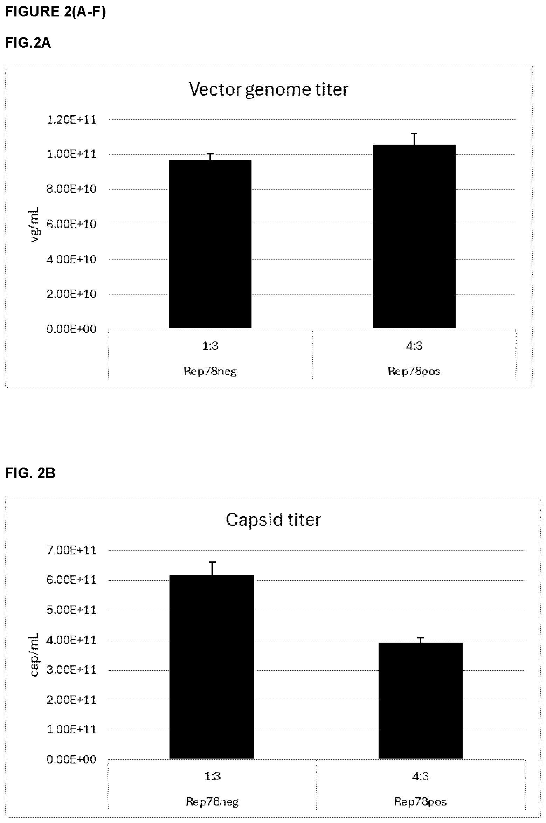

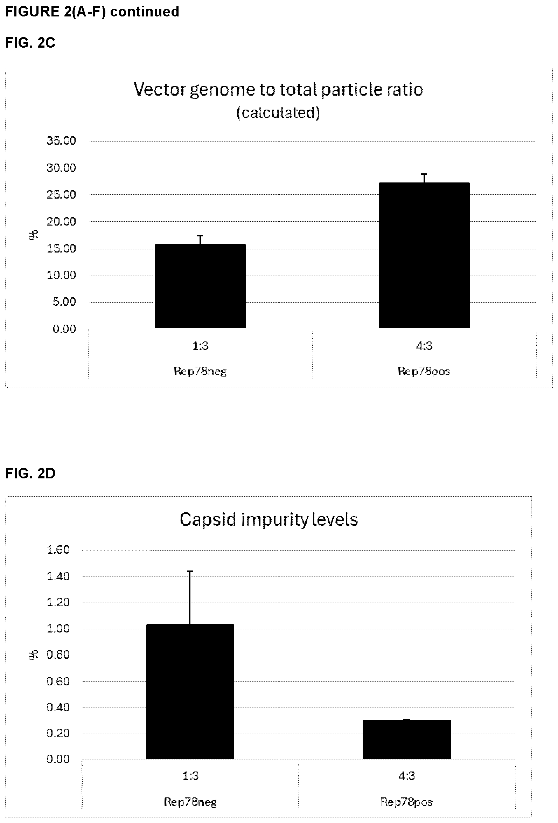

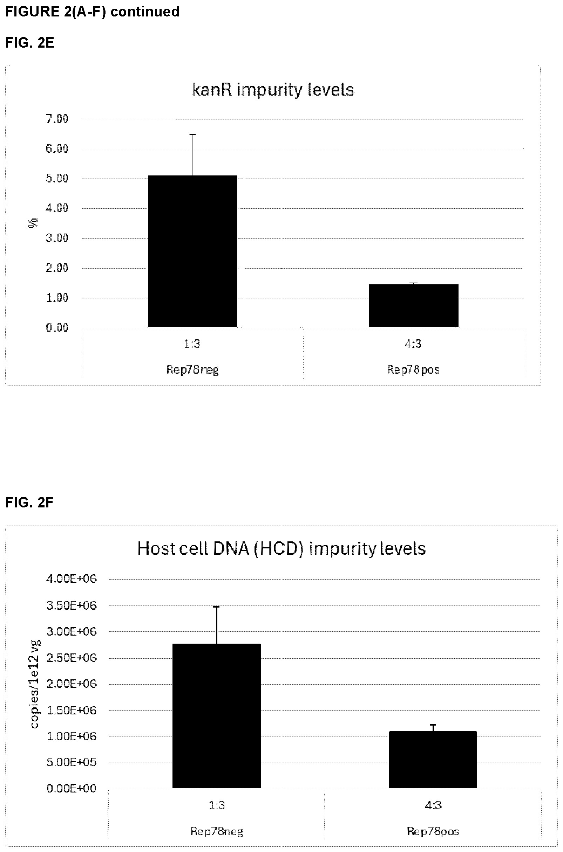

**Figure 2** **(A-F)** provides the results of analysis of rAAV produced and assayed as described in Example 7. Fig. 2A: Vector genome yield was determined for rAAV produced using the Rep78neg two plasmid system at a ratio of helper:vector plasmid of 1:3 or the Rep78pos two plasmid system at a ratio of helper:vector plasmid of 4:3. The vector plasmids used in both systems comprise the engineered cap gene and the expression cassette comprising encoding a marker transgene. transgene. Vector genome (vg) titre per ml was measured by qPCR. Fig. 2B: The yield of rAAV particles ("capsid yield") measured as the number of particles per ml ("cap/ml"), for the same samples of Fig. 2A. The number of particles per ml was measured by anti-capsid ELISA. Fig 2C: Vector genome to total particle ratio for the same samples of Fig. 2A. The vector genome to total particle ratio was determined using the vector genome yield and capsid yield as determined for (A) to (B) above. Levels of capsid, KanR and Host Cell DNA impurities were determined for rAAV produced using the Rep78neg two plasmid system at a ratio of helper:vector plasmid of 1:3 or the Rep78pos two plasmid system at a ratio of helper:vector plasmid of 4:3 are shown in Fig. 2D, Fig. 2E and Fig. 2F respectively. The vector plasmids used in both systems comprise the engineered cap gene and the expression cassette encoding for a marker transgene. The impurities were measured by qPCR amplifying a sequence of the cap gene, and the kanamycin resistance gene (kanR) or by ddPCR amplifying the 18S rRNA respectively. The percentage capsid impurity or percentage kanR impurity is the copy number of capsid or kanR impurity per ml (determined by qPCR) divided by the copy number of vector genomes per ml (determined by qPCR) x 100.

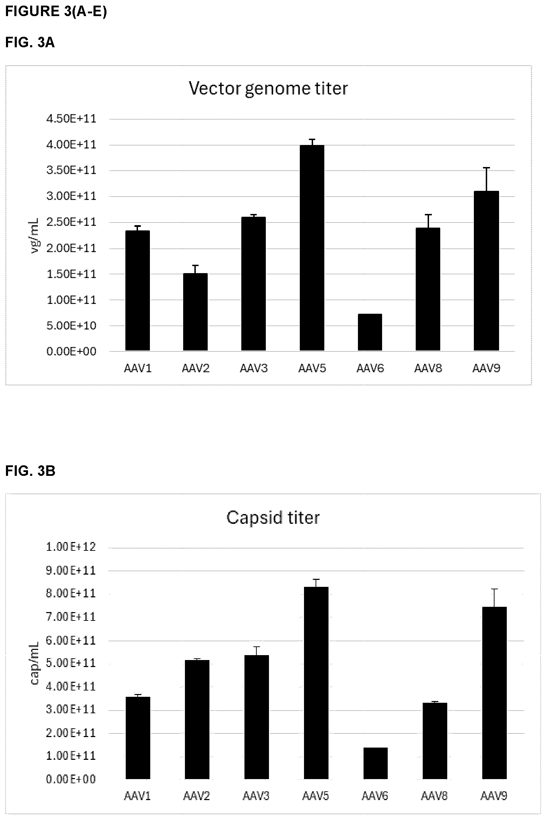

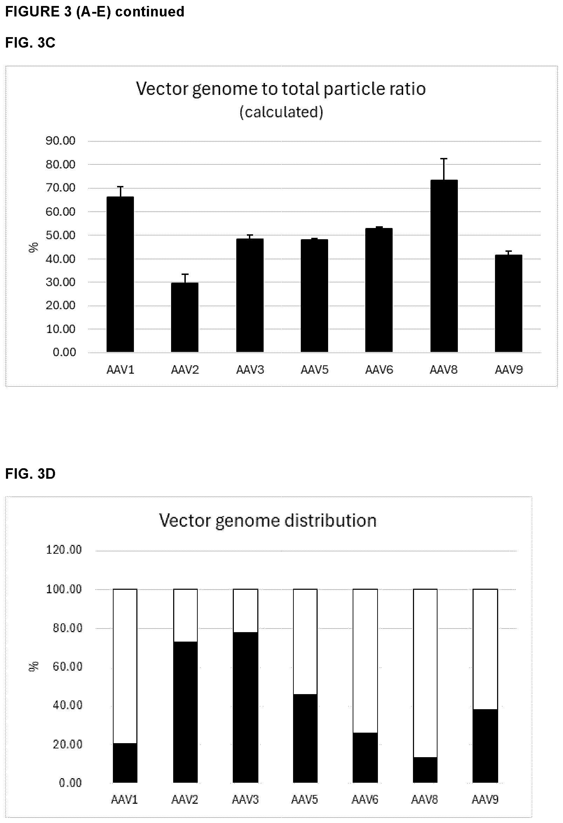

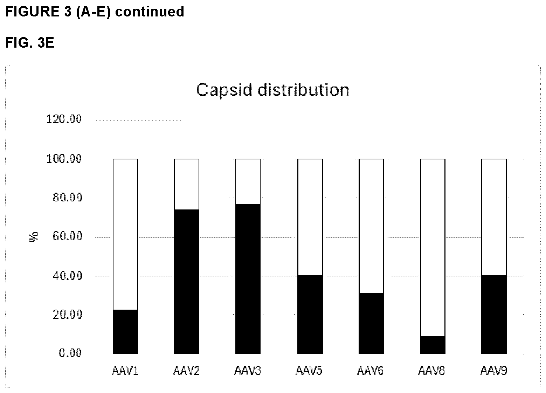

**Figure 3** **(A-E)** provides the results of analysis of rAAV produced and assayed as described in Example 8. Fig. 3A: Vector genome yield was determined for rAAV produced using the Rep78pos two plasmid system in different AAV serotypes (AAV1, AAV2, AAV3, AAV5, AAV6, AAV8, and AAV9) in molar helper:vector plasmid ratio of 4:3. Vector genome (vg) titre per ml was measured by SEAP ddPCR. The vector genome yield was determined 72 hrs after transfection. Fig. 3B: Yield of rAAV particles ("capsid yield") was determined for rAAV produced using the Rep78pos two plasmid system in different AAV serotypes (AAV1, AAV2, AAV3, AAV5, AAV6, AAV8, and AAV9) in molar helper:vector plasmid ratio of 4:3. The number of rAAV particles per ml was measured by a Gyrolab^{™} explore immunoassay. The capsid yield was determined 72 hrs post-transfection. Fig. 3C: Vector genome to total particle ratio for the same samples of Fig. 3A. The vector genome to total particle ratio was determined using the vector genome yield and capsid yield as determined for (A) to (B) above. Fig. 3D: Vector genome yield was determined for rAAV produced using the Rep78pos two-plasmid system system in different AAV serotypes (AAV1, AAV2, AAV3, AAV5, AAV6, AAV8, and AAV9) in molar helper:vector plasmid ratio of 4:3 in medium and lysate fraction of a differential harvest performed 72 hrs after transfection. The data is shown as percentage of the total vector genome yield for lysate (black) and medium (white) fraction. Fig. 3E: Capsid yield was determined for rAAV produced using the Rep78pos two-plasmid system system in different AAV serotypes (AAV1, AAV2, AAV3, AAV5, AAV6, AAV8, and AAV9) in molar helper:vector plasmid ratio of 4:3 in medium and lysate fraction of a differential harvest performed 72 hrs after transfection. The data is shown as percentage of the total vector genome yield for lysate (black) and medium (white) fraction.

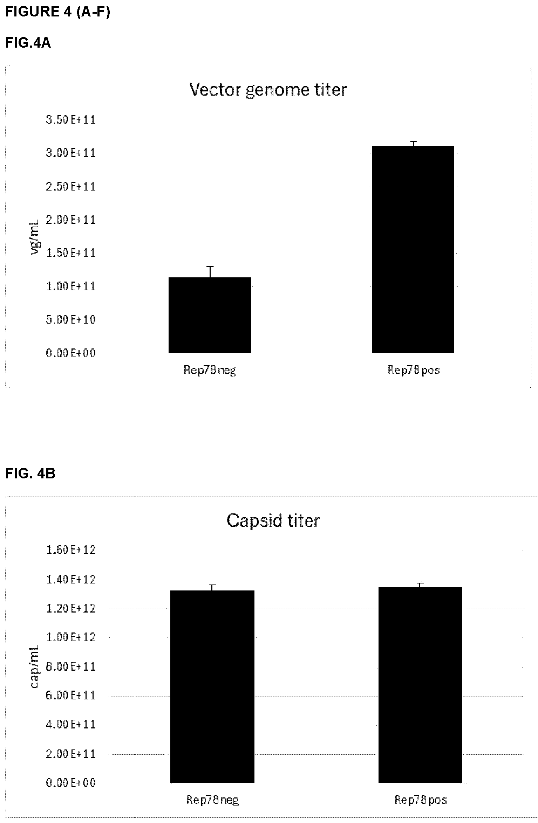

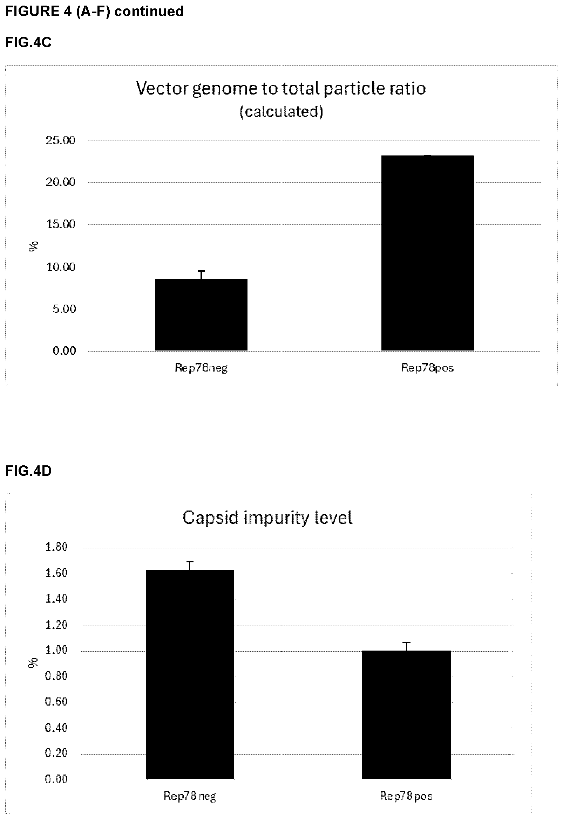

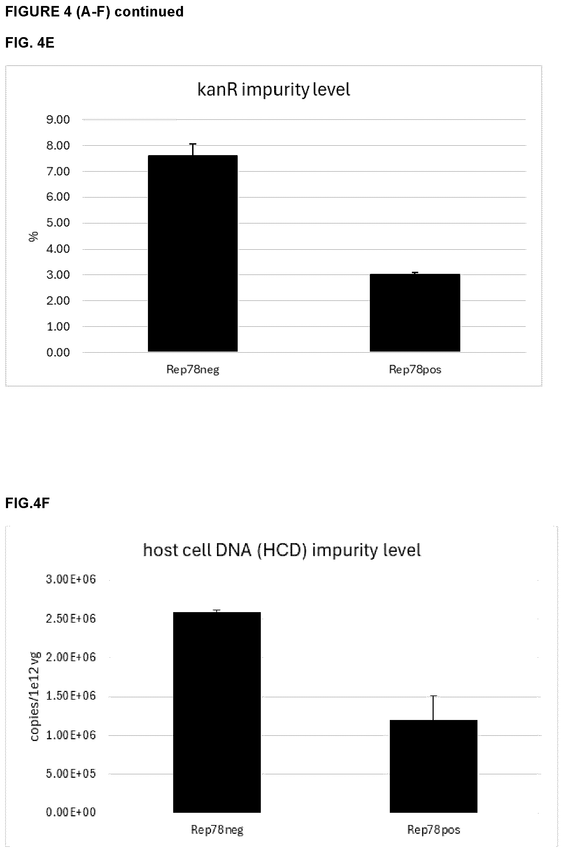

**Figure 4****(A-F)** provides the results of analysis of rAAV produced and assayed as described in Example 9. Fig. 4A: Vector genome yield was determined for rAAV produced using the Rep78pos two plasmid system using different expression cassettes encoding different size transgenes in a molar helper:vector plasmid ratio of 4:3. The control Rep78neg plasmid was used in a molar helper:vector plasmid ratio of 1: 3 accordingly. Vector genome (vg) titre per ml was measured by SEAP ddqPCR. The vector genome yield was determined 72 hrs after transfection. Fig. 3B: Yield of rAAV particles ("capsid yield") was determined for rAAV produced using the Rep78pos two plasmid system using different expression cassettes encoding different size transgenes in molar helper:vector plasmid ratio of 4:3. The control Rep78neg plasmid was used in a molar helper:vector plasmid ratio of 1: 3. The number of rAAV particles per ml was measured by a Gyrolab^{™} explore immunoassay. The capsid yield was determined 72 hrs post-transfection. Fig. 4C: The vector genome to total particle ratio was determined using the vector genome yield and capsid yield as determined for (A) to (B) above. The vector genome to total particle ratio was determined 72 h after transfection. Levels of capsid, KanR, and Host Cell DNA impurities were determined for rAAV produced using the Rep78pos two plasmid system using different size expression cassettes encoding different transgenes in molar helper:vector plasmid ratio of 4:3. The control Rep78neg plasmid was used in a molar helper:vector plasmid ratio of 1: 3. Measurements were performed 72h after transfection and are shown in Fig. 4D, Fig. 4E, Fig. 4F.

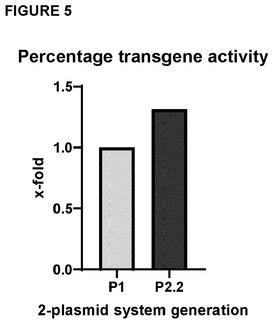

**Figure 5** provides the results of analysis following the transduction of HuH-7 cells with rAAV as described in Example 10. Transgene activity was determined following the transduction of Huh-7 cells with rAAV produced using the Rep78neg two plasmid system at a ratio of helper:vector plasmid of 1:3 (white bar) or the Rep78pos two plasmid system at a ratio of helper:vector plasmid of 4:3 (black bar). The vector plasmids used in both systems comprise the engineered cap gene and the expression cassette comprising the transgene.

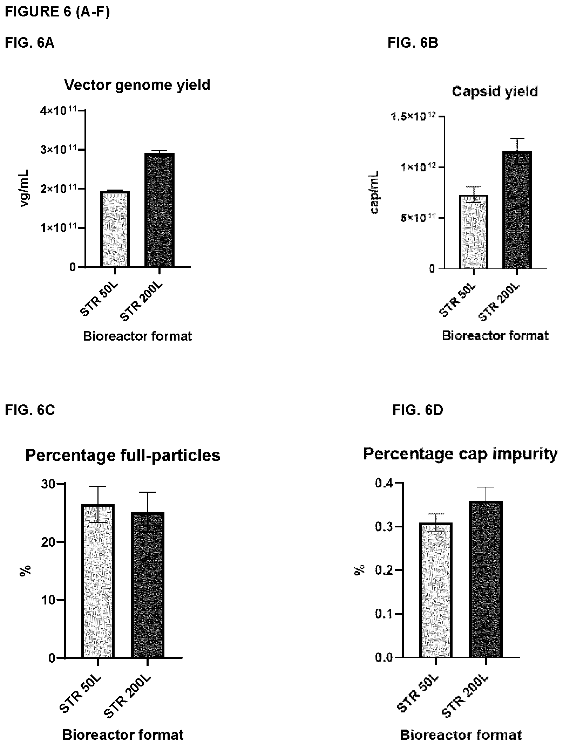

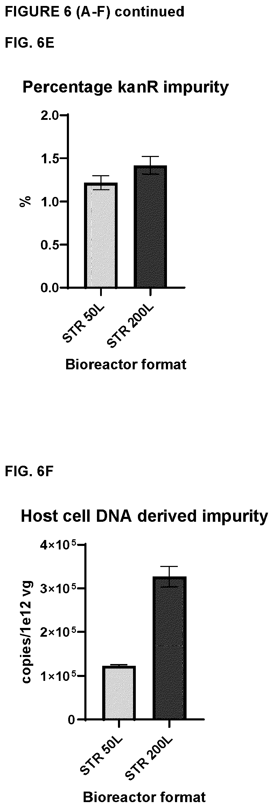

**Figure 6** **(A-F)** provides the results of analysis of rAAV produced and assayed as described in Example 11. Fig. 6A: Vector genome yield was determined for rAAV produced using the Rep78pos two plasmid system in a molar helper:vector plasmid ratio of 4:3. Vector genome (vg) titre per ml was measured by SEAP ddqPCR. The vector genome yield was determined 72 hrs after transfection. Fig. 6B: Yield of rAAV particles ("capsid yield") was determined for rAAV produced using the Rep78pos two plasmid system in molar helper:vector plasmid ratio of 4:3. The number of rAAV particles per ml was measured by a Gyrolab^{™} explore immunoassay. The capsid yield was determined 72 hrs post-transfection. Fig. 6C: The vector genome to total particle ratio was determined using the vector genome yield and capsid yield as determined for (A) to (B) above. The vector genome to total particle ratio was determined 72 h after transfection. Levels of capsid, KanR, and Host Cell DNA impurities were determined for rAAV produced using the Rep78pos two plasmid system in molar helper:vector plasmid ratio of 4:3. Measurements was were performed 72 hrs after transfection and are shown in Fig. 6D, Fig. 6E, Fig. 6F.

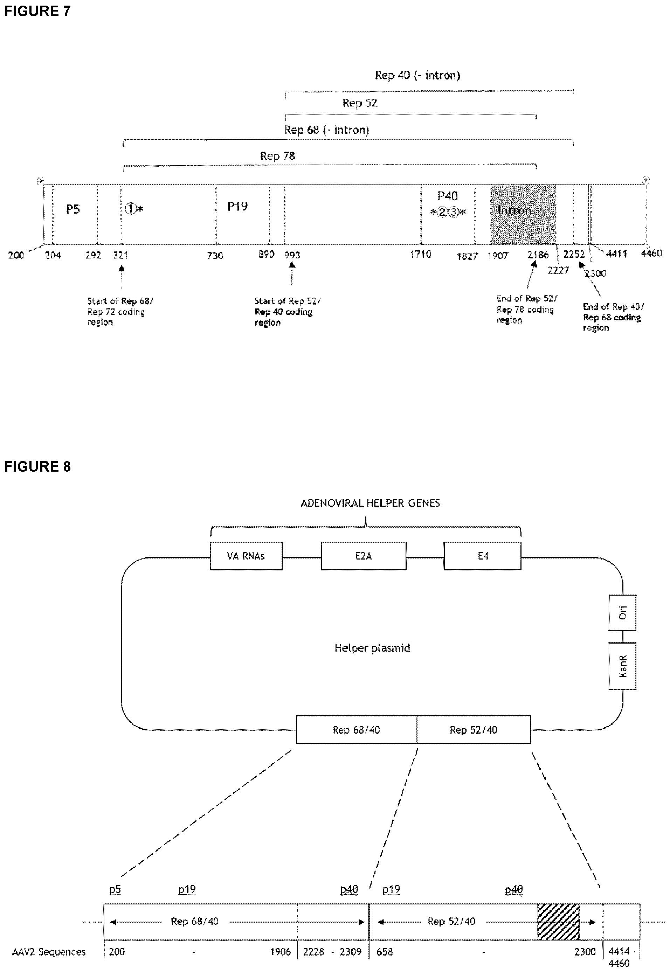

**Figure 7** shows a schematic of a suitable Rep78pos helper plasmid comprising at least one rep gene. The numbers indicate the corresponding position of each sequence element in the AAV2 genome. Hatched area in the rep 52/40 gene indicates the presence of intron sequence which is spliced into rep 52 transcript but spliced out of rep 40 transcript. Note the respective rep gene features are not shown to scale. The 4 Rep proteins are encoded by overlapping genes, with the Rep 40 protein being encoded by nucleotides 993-1907 and 2227 to 2252 of the AAV2 genome, the Rep 52 protein being encoded by nucleotides 993 to 2186 of the AAV2 genome, the Rep 68 protein being encoded by nucleotides 321 to 1907 and 2227 to 2252 of the AAV2 genome, and the Rep 78 protein being encoded by nucleotides 321 to 2186 of the AAV2 genome. Expression of Rep 78 and Rep 68 starts at the start codon in positions 321 to 323 of the AAV2 genome and is controlled by the p5 promoter. Expression of Rep 40 and Rep 52 starts at the start codon in positions 993 to 995 of the AAV2 genome, and is controlled by the p19 promoter. The p40 promoter is involved in controlling expression of Cap proteins in native AAV2.
**Figure 8** provides a schematic of the Rep78neg helper plasmid constructed as described in Example 1, with inset showing rep genes including p5, p19 and p40 promoters. The crosses through "p40" indicate that these promoters have been rendered non-functional. Hatched area in the rep 52/40 gene indicates the presence of intron sequence which is spliced into rep 52 transcript but spliced out of rep 40 transcript. Ori = bacterial origin of replication. KanR = kanamycin resistance gene. Note the respective plasmid features are not shown to scale.

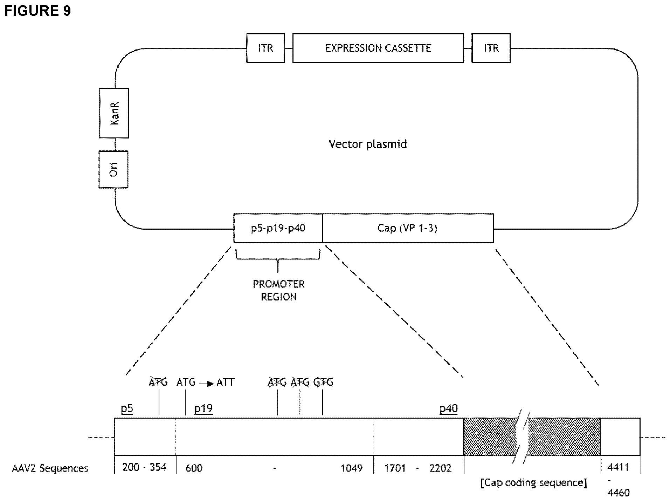

**Figure 9** provides a schematic of vector plasmid constructed as described in Example 2, with inset showing cap gene and upstream promoter region including p5, p19 and p40 promoters. The crosses through "ATG" and "GTG" indicate that these potential translation initiation codons have been deleted. Ori = bacterial origin of replication. KanR = kanamycin resistance gene. ITR = inverted terminal repeat. Note the respective plasmid features are not shown to scale.

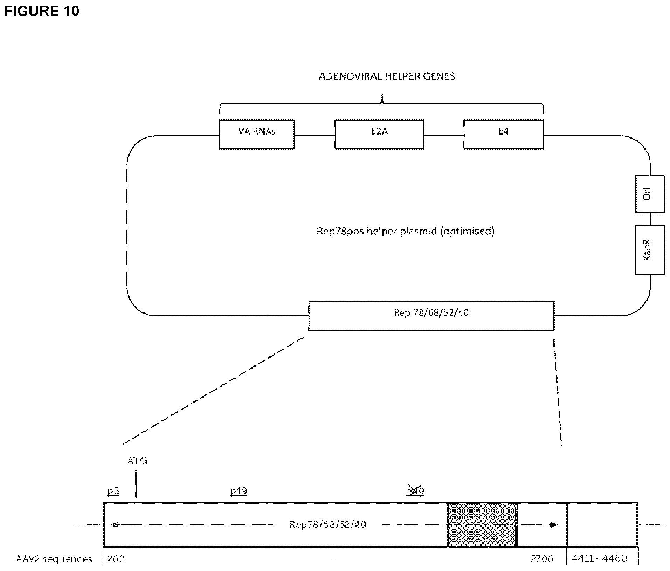

**Figure 10** provides a schematic of Rep78pos helper plasmid constructed as described in Example 3, with inset showing rep genes including p5, p19 and p40 promoters. The cross through "p40" indicates that this promoter has been rendered non-functional. Hatched area indicates the presence of intron sequence which is spliced into rep 52 and rep 78 transcripts but spliced out of rep 40 and rep 68 transcripts. Ori = bacterial origin of replication. KanR = kanamycin resistance gene. Note the respective plasmid features are not shown to scale.

### DETAILED DESCRIPTION

Unless defined otherwise, technical and scientific terms used herein have the same meaning as commonly understood by a person skilled in the art to which this invention belongs. All publications, patent applications, patents and other references mentioned herein are incorporated by reference in their entirety.

In some embodiments, the term *"around"* in relation to a reference numerical value and its grammatical equivalents as used herein can include the numerical value itself and a range of values plus or minus 10% from that numerical value. For example, in these embodiments the term *"around* 10" includes 10 and any amounts from 9 to 11. For example, in one the term *"around"* in relation to a reference numerical value can also include a range of values plus or minus 10%, 9%, 8%, 7%, 6%, 5%, 4%, 3%, 2%, or 1% from that value.

In general, the term *"comprising"* is intended to mean including but not limited to. For example, the phrase "a *helper plasmid comprising at least one rep gene"* should be interpreted to mean that the helper plasmid has at least one rep gene, but the helper plasmid may comprise further components such as further genes.

In some embodiments of the invention, the word *"comprising"* is replaced with the phrase *"consisting of'* or the phrase *"consisting essentially of'.* The term *"consisting of'* is intended to be limiting. For example, the phrase "a *helper plasmid consisting of one rep gene"* should be understood to mean that the helper plasmid has one rep gene and no other genetic material. The phrase *"A helper plasmid which comprises at least one rep gene wherein the at least one rep gene consists of a rep 78 cassette"* should be understood to mean that the helper plasmid includes a rep gene and other genetic material, but that the rep 78 cassette is the only portion of the helper plasmid that encodes Rep 78. Similarly, the phrase "a *helper plasmid consisting essentially of one rep gene"* should be understood to mean that the helper plasmid has one rep gene and comprises no additional components that materially affect the function of the helper plasmid. For example, a helper plasmid consisting essentially of a rep gene will not contain any other genes but could contain other genetic material such as spacers.

The terms *"protein"* and *"polypeptide"* are used interchangeably herein, and are intended to refer to a polymeric chain of amino acids of any length.

For the purpose of this invention, in order to determine the percent identity of two sequences (such as two polynucleotide or two polypeptide sequences), the sequences are aligned for optimal comparison purposes (e.g., gaps can be introduced in a first sequence for optimal alignment with a second sequence). The nucleotides or amino acids at each position are then compared. When a position in the first sequence is occupied by the same amino acid or nucleotide as the corresponding position in the second sequence, then the amino acids or nucleotides are identical at that position. The percent identity between the two sequences is a function of the number of identical positions shared by the sequences (i.e., % identity = number of identical positions /total number of positions in the reference sequence x 100).

Typically, the sequence comparison is carried out over the length of the reference sequence. For example, if the user wished to determine whether a given ("*test*") sequence is 95% identical to SEQ ID NO: 1, SEQ ID NO: 1 would be the reference sequence. To assess whether a sequence is at least 80% identical to SEQ ID NO: 1 (an example of a reference sequence), the skilled person would carry out an alignment over the length of SEQ ID NO: 1, and identify how many positions in the test sequence were identical to those of SEQ ID NO: 1. If at least 80% of the positions are identical, the test sequence is at least 80% identical to SEQ ID NO: 1. If the sequence is shorter than SEQ ID NO: 1, the gaps or missing positions should be considered to be non-identical positions.

The skilled person is aware of different computer programs that are available to determine the homology or identity between two sequences. For instance, a comparison of sequences and determination of percent identity between two sequences can be accomplished using a mathematical algorithm. In an embodiment, the percent identity between two amino acid or nucleic acid sequences is determined using the Needleman and Wunsch (1970) algorithm which has been incorporated into the GAP program in the Accelrys GCG software package (available at http://www.accelrys.com/products/gcg/), using either a Blosum 62 matrix or a PAM250 matrix, and a gap weight of 16, 14, 12, 10, 8, 6, or 4 and a length weight of 1, 2, 3, 4, 5, or 6.

Herein, the term *"plasmid"* is intended to refer to a nucleic acid molecule that can replicate independently of a cell chromosome. The term *"plasmid"* is intended to cover circular nucleic acid molecules and linear nucleic acid molecules. Furthermore, the term *"plasmid"* is intended to cover bacterial plasmids, but also cosmids, minicircles (*Nehlsen, K., Broll S., Bode, J. (2006), Gene Ther. Mol. Biol., **10:** 233*-*244*; *Kay, M.A., He, C.-Y, Chen, Z.-H. (2010), Nature*

*Biotechnology, **28:** 1287-1289)* and ministrings (Nafissi N, Alqawlaq S, Lee EA, Foldvari M, Spagnuolo PA, Slavcev RA. (2014), Mol Ther Nucleic Acids, 3:e165). Optionally, the plasmid is a circular nucleic acid molecule. Optionally, the plasmid is a nucleic acid molecule that is of bacterial origin.

The term *"helper"* is not intended to be limiting. Accordingly, a *"helper plasmid"* is any plasmid that comprises at least one rep gene encoding at least one functional Rep protein and does not comprise a cap gene encoding a functional set of Cap proteins.

The term *"vector plasmid"* is not intended to be limiting. Accordingly, a *"vector plasmid"* is any plasmid that:
(i) is suitable for use alongside a helper plasmid in a two-plasmid system of the invention; and/or
(ii) comprises:
   (a) a cap gene encoding at least one functional Cap protein; or
   (b) at least one cap gene promoter, a cloning site operably linked to the cap gene promoter, and an expression cassette flanked on at least one side by an ITR;
wherein the vector plasmid does not comprise a rep gene encoding a functional Rep protein and the expression cassette comprises a transgene operably linked to at least one regulatory control element.

The term *"nucleic acid molecule"* refers to a polymeric form of nucleotides of any length. The nucleotides may be deoxyribonucleotides, ribonucleotides or analogues thereof. Preferably, the plasmid is made up of deoxyribonucleotides or ribonucleotides. Even more preferably, the plasmid is made up of deoxyribonucleotides, i.e. the plasmid is a DNA molecule. In all instances herein, the term *"nucleic acid sequence"* may be replaced by the term *"polynucleotide".*

The terms *"wild type"* and *"native"* are synonymous and refer to genes present in the genome of a strain/serotype of AAV or adenovirus, or to proteins encoded by genes present in the genome of a strain/serotype of AAV or adenovirus.

### A helper plasmid

The present invention provides a helper plasmid which does not comprise a cap gene encoding a functional set of Cap proteins and which does comprise at least one Adeno-associated virus (AAV) rep gene and at least one helper virus gene, wherein the at least one rep gene comprises a rep 78 cassette comprising a nucleotide sequence encoding a functional Rep 78 protein and a transcription regulatory element or a rep 78 cassette comprising a nucleotide sequence encoding a functional Rep 78 protein and a functional Rep 68 protein and a transcription regulatory element, and wherein said rep 78 cassette comprises an ATG start codon at the beginning of the nucleotide sequence encoding a functional Rep 78 protein or both a functional Rep78 and a functional Rep68 protein.

The present invention also provides a helper plasmid which does not comprise a cap gene encoding a functional set of Cap proteins and which does comprise at least one Adeno-associated virus (AAV) rep gene and at least one helper virus gene, wherein the at least one rep gene comprises a rep 78 cassette comprising a nucleotide sequence encoding a functional Rep 78 protein and a transcription regulatory element, and wherein said rep 78 cassette comprises an ATG start codon at the beginning of the nucleotide sequence encoding a functional Rep 78 protein

The present invention also provides a helper plasmid which does not comprise a cap gene encoding a functional set of Cap proteins and which does comprise at least one rep gene and at least one helper virus gene, wherein the at least one rep gene comprises a rep 78 cassette comprising a nucleotide sequence encoding a functional Rep 78 protein and a transcription regulatory element or a rep 78 cassette comprising a nucleotide sequence encoding a functional Rep 78 protein and a functional Rep 68 protein and a transcription regulatory element, and wherein said rep 78 cassette comprises an ATG start codon at the beginning of the nucleotide sequence encoding a functional Rep 78 protein or both a functional Rep78 and a functional Rep68 protein, and wherein said plasmid promotes production of recombinant AAV at a higher yield compared to a helper plasmid wherein the at least one rep gene does not encode a Rep 78 protein.

The present invention also provides a helper plasmid which does not comprise a cap gene encoding a functional set of Cap proteins and which does comprise at least one rep gene and at least one helper virus gene, wherein the at least one rep gene comprises a rep 78 cassette comprising a nucleotide sequence encoding a functional Rep 78 protein and a transcription regulatory element, and wherein said rep 78 cassette comprises an ATG start codon at the beginning of the nucleotide sequence encoding a functional Rep 78 protein, and wherein said plasmid promotes production of recombinant AAV at a higher yield compared to a helper plasmid wherein the at least one rep gene does not encode a Rep 78 protein.

The present invention also provides a helper plasmid which does not comprise a cap gene encoding a functional set of Cap proteins and which does comprise at least one rep gene and at least one helper virus gene, wherein the at least one rep gene comprises a rep 78 cassette comprising a nucleotide sequence encoding a functional Rep 78 protein and a transcription regulatory element or a rep 78 comprising a nucleotide sequence encoding a functional Rep 78 protein and a functional Rep 68 protein and a transcription regulatory element, and wherein said rep 78 cassette comprises an ATG start codon at the beginning of the nucleotide sequence encoding a functional Rep 78 protein or both a functional Rep78 and a functional Rep68 protein, and wherein said plasmid promotes production of recombinant AAV at a higher yield compared to a helper plasmid wherein the at least one rep gene does not encode a Rep 78 protein, and/or said helper plasmid promotes production of recombinant AAV comprising a lower level of nucleic acid impurities compared to a helper plasmid comprising at least one rep gene which does not encode a Rep 78 protein.

The present invention also provides a helper plasmid which does not comprise a cap gene encoding a functional set of Cap proteins and which does comprise at least one rep gene and at least one helper virus gene, wherein the at least one rep gene comprises a rep 78 cassette comprising a nucleotide sequence encoding a functional Rep 78 protein and a transcription regulatory element, and wherein said rep 78 cassette comprises an ATG start codon at the beginning of the nucleotide sequence encoding a functional Rep 78 protein, and wherein said plasmid promotes production of recombinant AAV at a higher yield compared to a helper plasmid wherein the at least one rep gene does not encode a Rep 78 protein, and/or said helper plasmid promotes production of recombinant AAV comprising a lower level of nucleic acid impurities compared to a helper plasmid comprising at least one rep gene which does not encode a Rep 78 protein.

The present invention also provides a helper plasmid which does not comprise a cap gene encoding a functional set of Cap proteins and which does comprise at least one rep gene and at least one helper virus gene, wherein the at least one rep gene comprises a rep 78 cassette comprising a nucleotide sequence encoding a functional Rep 78 protein and a transcription regulatory element or a rep 78 cassette comprising a nucleotide sequence encoding a functional Rep 78 protein and a functional Rep 68 protein and a transcription regulatory element, and wherein said rep 78 cassette comprises an ATG start codon at the beginning of the nucleotide sequence encoding a functional Rep 78 protein or both a functional Rep78 and a functional Rep68 protein, and wherein said plasmid promotes production of recombinant AAV at a higher yield compared to a helper plasmid wherein the at least one rep gene does not encode a Rep 78 protein, and/or said helper plasmid promotes production of recombinant AAV comprising a lower level of nucleic acid impurities compared to a helper plasmid comprising at least one rep gene which does not encode a Rep 78 protein.

The present invention also provides a helper plasmid which does not comprise a cap gene encoding a functional set of Cap proteins and which does comprise at least one rep gene and at least one helper virus gene, wherein the at least one rep gene comprises a rep 78 cassette comprising a nucleotide sequence encoding a functional Rep 78 protein and a transcription regulatory element, and wherein said rep 78 cassette comprises an ATG start codon at the beginning of the nucleotide sequence encoding a functional Rep 78 protein, and wherein said plasmid promotes production of recombinant AAV at a higher yield compared to a helper plasmid wherein the at least one rep gene does not encode a Rep 78 protein, and/or said helper plasmid promotes production of recombinant AAV comprising a lower level of nucleic acid impurities compared to a helper plasmid comprising at least one rep gene which does not encode a Rep 78 protein.

Optionally, the rep 78 cassette consists of a nucleotide sequence encoding a functional Rep 78 protein, a transcription regulatory element and a short non-rep coding nucleotide sequence, wherein the short non-rep encoding nucleotide sequence is optionally between the nucleotide sequence encoding the functional Rep 78 protein and the transcription regulatory element. A nucleotide sequence is a non-rep encoding nucleotide sequence if it does not encode amino acids that form part of a Rep protein sequence. The short non-rep encoding nucleotide sequence may be non coding (i.e. it may not encode a portion of any protein), or it may encode part of a protein such as a Cap protein. Optionally, the short nucleotide sequence between the functional Rep 78 protein and the transcription regulatory element is less than 100 nucleotides, less than 80 nucleotides, less than 60 nucleotides, less than 50 nucleotides, less than 40 nucleotides or less than or around 30 nucleotides in length. Optionally, the at least one rep gene consists essentially of the rep 78 cassette and a polyA sequence. Optionally, the at least one rep gene consists of the rep 78 cassette, a polyA sequence and a short non-rep encoding nucleotide sequence. Optionally, the rep 78 cassette consists of the rep 78 cassette, a poly A sequence, a short non-rep encoding nucleotide sequence and nucleotides corresponding to nucleotides 2186-2252 of SEQ ID NO: 1. The short non-rep encoding nucleotide sequence may be between the rep 78 cassette and the polyA sequence. The short non-rep encoding nucleotide sequence may be less than 370, less than 330, less than 150, less than 100, less than 90, less than 80, or less than 70 nucleotides in length.

The helper plasmid may be useful for producing rAAV. Optionally, the helper plasmid is suitable for use in producing rAAV. Optionally, the helper plasmid is for producing rAAV. Optionally, the helper plasmid is suitable for producing rAAV suitable for use in gene therapy. Optionally, the helper plasmid is for producing rAAV for use in gene therapy.

### Helper plasmid does not comprise a cap gene encoding a functional set of Cap proteins

The helper plasmid of the invention does not comprise a cap gene encoding a functional set of Cap proteins, which means that it cannot be used to produce rAAV in isolation. However, the helper plasmid of the invention may be used to produce rAAV in a system that provides the cap function. For example, the helper plasmid of the invention may be used to produce rAAV in a multi plasmid system, such as a two-plasmid system of the invention. Accordingly, the helper plasmid may be useful for producing rAAV as part of a two-plasmid system. Optionally, the helper plasmid is suitable for use in producing rAAV as part of a two-plasmid system. Optionally, the helper plasmid is for producing rAAV as part of a two-plasmid system. Optionally, the helper plasmid is suitable for producing rAAV suitable for use in gene therapy as part of a two-plasmid system. Optionally, the helper plasmid is for producing rAAV for use in gene therapy as part of a two-plasmid system.

As the helper plasmid of the invention does not comprise a cap gene encoding a functional set of Cap proteins, it can be used as part of a multi plasmid system that uses a 'trans-split configuration', *i.e*. a plasmid configuration in which the rep and cap genes are not both present on a single plasmid. Ensuring that the helper plasmid does not comprise both a rep gene and a cap gene is difficult to do, as the native AAV cap and rep genes are overlapping and so separating them whilst maintaining sufficient genetic material to allow for production of the complete protein is difficult. However, splitting the rep genes and the cap genes between a helper plasmid and a vector plasmid is advantageous, as it increases the number of recombination events required to form rcAAV.

### At least one rep gene

The helper plasmid comprises at least one rep gene encoding at least one functional Rep protein.

AAV comprises a rep gene region which encodes four Rep proteins (Rep 78, Rep 68, Rep 52 and Rep 40); this is described in Figure 7. The rep gene region is under the control of the p5 and p19 promoters. When the p5 promoter is used, a gene that encodes Rep 78 and Rep 68 is transcribed. Rep 78 and Rep 68 are two alternative splice variants (Rep 78 comprises an intron that is excised in Rep 68). Similarly, when the p19 promoter is used, a gene that encodes Rep 52 and Rep 40 is transcribed. Rep 52 and Rep 40 are alternative splice variants (Rep 52 comprises an intron that is excised in Rep 40).

The four Rep proteins are known to be involved in replication and packaging of the viral genome, and are, therefore, useful in rAAV production.

It is not necessary for all four Rep proteins to be present. In the context of the present disclosure, however, the at least one rep gene encodes a large Rep protein (Rep 78, or a combination of a Rep 78 and a Rep 68) and a small Rep protein (Rep 52 or Rep 40).

### "functional" Rep protein

A "functional" Rep protein is one which allows for production of AAV particles. In particular, Rep 78 or Rep 68 (the large Rep proteins) are believed to be involved in replication of the AAV genome, and Rep 52 and Rep 40 (the small Rep proteins) are believed to be involved in packaging of the AAV genome into a capsid. It is within the abilities of the skilled person to determine whether a given Rep protein is functional. The skilled person merely needs to determine whether the Rep protein supports AAV production using an AAV production assay as described below. In this case, the test two-plasmid system will comprise a helper plasmid that comprises the Rep protein whose "functionality" is to be determined, and otherwise the test two-plasmid system used will be identical to the reference two-plasmid system.

### Rep 78 cassette

The term *"a rep 78 cassette" should* be understood to mean a nucleic acid sequence that comprises a portion encoding Rep 78, a transcription regulatory element that is operably linked to the portion encoding Rep 78, and any nucleotides positioned between the transcription regulatory element and the portion encoding Rep 78. For example, the region from position 204-2186 or 204-2252 of SEQ ID NO: 1 can be considered to be a rep 78 cassette; see Figure 7. Any nucleotides in the plasmid that do not encode a Rep protein, do not encode transcription regulatory elements and are not positioned between the Rep 78 encoding portion and the transcription regulatory element should not be considered to be part of the *"rep 78 cassette".* For the avoidance of doubt, a *"polyA sequence"* will not be considered to be part of the *"rep 78 cassette",* but the helper plasmid of the invention may comprise a polyA sequence, for example, a polyA sequence may be positioned 3' of the rep 78 cassette. Optionally, the polyA sequence corresponds to all or a portion of nucleotides 4411-4460 of SEQ ID NO: 1. The rep 78 cassette may also encode Rep 52 and at least a substantial portion of Rep 68 and Rep 40. The rep 78 cassette may comprise nucleotides corresponding to nucleotides 204-2252 of the AAV2 genome (SEQ ID NO: 1) or corresponding nucleotides in another strain of AAV. Nucleotides 204-2252 of SEQ ID NO: 1 correspond to a Rep78 encoding region (corresponding to nucleotides 321-2186 of SEQ ID NO: 1), a p5 promoter (which is a transcription regulatory element corresponding to nucleotides 204-292 of SEQ ID NO: 1), and nucleotides positioned between the p5 promoter and the transcription regulatory element (corresponding to nucleotides 293-320 of SEQ ID NO: 1). Optionally, the Rep 78 cassette shall be understood to mean a nucleic acid sequence wherein the intron allowing for encoding a Rep78 protein is in some cases excised and thus comprises a portion encoding Rep 68, a transcription regulatory element that is operably linked to the portion encoding Rep 68, and any nucleotides positioned between the transcription regulatory element and the portion encoding Rep68.

### Production of recombinant AAV at a higher yield

When rep 78 is expressed or both rep78 and rep68 are expressed utilising an ATG start codon at the beginning of the nucleotide sequence encoding a functional Rep 78 protein or both a functional Rep 78 and a functional Rep 68 protein, such an arrangement appears to particularly beneficial for AAV production in suspension cells.

### AAV production assay

In an AAV production assay, the user can determine whether a given *"test"* plasmid or two-plasmid system is effective to produce rAAV at a similar level to a *"reference"* plasmid or two-plasmid system as follows.

The user provides a *"reference"* two-plasmid system that comprises a *"reference helper plasmid"* and a *"reference vector plasmid".*

For example, the user provides a reference helper plasmid comprising wild type Adenovirus 5 helper genes encoding E2A, E4 and VA RNA I and II, i.e. the adenovirus helper genes comprised within SEQ ID NO: 2. Details of the nucleic acid positions in SEQ ID NO: 2 which encode these genes are set out in more detail below under the heading *"at least one virus helper gene".* The helper plasmid also comprises a wild type rep gene encoding Rep 40, Rep 52, Rep 68 and Rep 78 and the rep promoters p5, p19 and p40, i.e. the sequences comprised within nucleotides 200-2252 of SEQ ID NO: 1. Such a helper plasmid is described in WO 2020/208379 A1 and is incorporated herein by reference.

The user provides a reference vector plasmid comprising a wild type cap gene operably linked to a wild type cap gene promoter comprising p5, p19 and p40, i.e. the cap gene comprised within SEQ ID NO: 1 (nucleotides 5961-8171 of SEQ ID NO: 1). The vector plasmid further comprises a transgene flanked by two AAV2 ITRs, i.e. the ITRs comprised within nucleotides 1-145 and 4535-4679 of SEQ ID NO: 1. Such a helper plasmid is described in WO 2020/208379 A1 and is incorporated herein by reference.

The user then provides a "*test*" two-plasmid system comprising a *"test helper plasmid"* and a *"test vector plasmid"* that is based on the reference two-plasmid system, but has a single change relating to a characteristic that the user wishes to test. For example, if the user wishes to see whether a given Rep protein is functional, the user could swap out the rep gene of the *"reference helper plasmid"* and replace it with the test rep protein to provide a *"test helper plasmid".*

The user then compares the ability of the reference two-plasmid system and the test two-plasmid system to allow for production of rAAV. To do this, the user can transfect a first set of suitable host cells in suspension culture (such as HEK293T cells which express the E1A/B gene so that the helper plasmid does not need to comprise an E1A/B gene) with the reference two-plasmid system, and a second set of identical host cells with the test two-plasmid system and incubate the host cells for a period of time suitable for the AAV production to occur. The yield of recombinant AAV produced from the reference two-plasmid system and the test two-plasmid system may then be harvested and measured using qPCR to quantify the number of vector genomes. For example, qPCR may be used to determine the number of instances of nucleic acid molecules comprising a component of the vector genome, such as a promoter sequence, that are produced in the host cells transfected with the test two-plasmid system compared to the host cells transfected with the reference two-plasmid system. Alternatively, the comparative yield of particles may be determined, for example by an anti-capsid ELISA or using an immunoassay preferably a sandwich immunoassay such a Gyrolab^{®}Xplore.

### Production of recombinant rAAV comprising a lower level of nucleic acid impurities

Surprisingly, the Examples demonstrate that helper plasmids that express functional Rep 78 utilising an ATG start codon for the expression of said functional Rep 78 can be used to produce AAV e.g. AAV2 comprising a lower level of nucleic acid impurities compared to helper plasmids that do not contain a nucleic acid sequence encoding functional Rep 78.

Whether or not a helper plasmid that express functional Rep 78 under the control of a wild type p5 promoter at or utilising an ATG start codon for the expression of said functional Rep 78 can produce AAV e.g. AAV2 comprising a lower level of nucleic acid impurities compared to a helper plasmid that does not contain a nucleic acid sequence encoding functional Rep 78 may be determined using an *"impurity level"* assay based on the *"AAV production"* assay specific for the rep 78 gene.

### The term "level of nucleic acid impurities" is discussed in more detail in the section entitled "lower level of AAV comprising impurities".

The impurity level assay should be based on the general AAV production assay discussed above. In the case of an impurity level assay the *"reference helper plasmid"* does not contain a nucleic acid sequence encoding a functional Rep 78 (*"an AAV2 reference"* helper plasmid). The "*test*" helper plasmid is identical, except that it comprises a nucleic acid sequence encoding a functional Rep78 protein.. Rather than measuring the number of instances of nucleic acid molecules comprising a component of the vector genome, the user measures the number of nucleic acid impurities. For example, the user may use ddPCR or qPCR to measure the level of nucleic acid impurities as described in the section entitled *"lower level of AAV comprising impurities".*

### Start codon

Start codons are present at the beginning of genes, and represent the first codon of the messenger RNA transcript. The start codon effectively provides an instruction to the cell machinery to begin transcription. A variety of start codons are available. For example, a variety of start codons are used for transcription initiation in eukaryotic cells. Different start codons may be more or less efficient, i.e. transcription will initiate at different start codons at different levels. Genes that are initiated using efficient start codons will be transcribed more frequently compared to genes that are initiated using less efficient start codons.

ATG is the most common start codon used in eukaryotic DNA, and other start codons will generally be less efficient than ATG at starting transcription in human cells (such as host cells that may be used to produce recombinant AAV). For example, alternative start codons include ACG, ATC, AAG, AGG, CTG, GTG, ATT, ATA, and TTG. Optionally, therefore, the start codon that is less efficient that ATG is selected from the group consisting of ACG, ATC, AAG, AGG, CTG, GTG, ATT, ATA, and TTG. Optionally, the start codon that is less efficient than ATG is an ACG start codon.

One may determine whether a given start codon is less efficient than ATG by performing "a *start codon efficiency assay".* The start codon efficiency assay is very similar to the rep 78 cassette expression level assay set out above. However, in this case the "*test*" helper plasmid comprises a rep 78 cassette comprising the start codon whose efficiency is to be assayed, e.g. a start codon other than ATG, and the *"reference"* helper plasmid is identical to the "*test*" helper plasmid, except that the start codon is ATG. If the amount of Rep 78 expression level of the "*test*" helper plasmid is lower than the Rep 78 expression level of the *"reference"* helper plasmid, then the start codon is less efficient than ATG.

The start codon is positioned at the beginning of the nucleotide sequence encoding a functional Rep 78 protein. A start codon is *"at the beginning of the nucleotide sequence encoding a Rep 78 protein"* if it encodes the first amino acid of the functional Rep 78 protein. In wild type AAV2 the functional Rep 78 encoding portion begins at positions 321 to 323. However, the start position may differ in different AAV strains. Optionally, the start codon at the beginning of the nucleotide sequence encoding a functional Rep 78 protein is a start codon in a position corresponding to nucleotides 321 to 323 of SEQ ID NO: 1

### Transcription regulatory elements

Transcription regulatory elements are nucleotide sequences which effect the level of expression of a gene, and include, for example, promoters, enhancers, introns, untranslated regions, and transcriptional terminators. Some transcription regulatory elements promote greater levels of transcription compared to others (stronger transcription regulatory elements). For example, some promoters are known to promote transcription at a higher level than others. Generally, a promoter will be a stronger promoter if it comprises a sequence that allows for strong binding to the transcription complex. Promoters which are known to be generally strong promoters in human cells include viral promoters. Promoters that are generally believed to be strong promoters in human cells include the EF1A, CMV, CAG and SV40 promoters. Promoters that are generally believed to be weak promoters in human cells include UBC and PGK promoters.

The rep 78 cassette comprises a nucleotide sequence encoding a functional Rep 78 protein. A *"functional"* Rep 78 protein is one which allows for production of AAV particles. Rep 78 is believed to be involved in replication of the AAV genome, so a functional Rep 78 protein is one that provides increased levels of AAV genome replication. Optionally, the functional Rep 78 protein is a protein of SEQ ID NO: 15.

One can determine whether a Rep 78 protein is functional, by carrying out a *"Rep* 78 *protein functionality"* assay. A two-plasmid system can be used to produce recombinant AAV even if it does not encode Rep 78 (as long as it encodes the other three Rep proteins - Rep 68, Rep 52 and Rep 40). Thus, the Rep 78 protein functionality assay is very similar to the *"AAV production"* assay described above, except that the "*test*" helper plasmid and the *"reference"* helper plasmid both have identical transcription regulatory elements and both have an ATG start codon, but the "*test*" helper plasmid comprises a nucleotide sequence encoding the Rep 78 whose function is being tested as well as the other three Rep proteins (Rep 68, Rep 52 and Rep 40) and the *"reference"* helper plasmid lacks a nucleotide sequence encoding Rep 78 (but encodes Rep 68, Rep 52 and Rep 40). If the "*test*" helper plasmid produces a higher yield of recombinant AAV compared to the *"reference"* helper plasmid, then the rep 78 cassette encodes a Rep 78 protein that is functional. Optionally, the Rep 78 protein is functional if it improves yield of recombinant AAV production by at least 1. 03, at least 1.04, at least, 1.05, at least 1.1, at least 1.2 fold, at least 1.4 fold, at least 1.8 fold, at least 2 fold, between 1.2 fold and 10 fold, between 1.4 fold and 10 fold, between 1.8 fold and 25 fold, or between 2 fold and 50 fold higher, i.e. the Rep 78 protein is functional if the "*test*" helper plasmid produces an at least 1.2 fold, at least 1.4 fold, at least 1.8 fold, at least 2 fold, between 1.2 fold and 10 fold, between 1.4 fold and 10 fold, between 1.8 fold and 25 fold, or between 2 fold and 50 fold higher yield compared to the *"reference"* plasmid.

Optionally, the Rep 78 protein encoded by the nucleotide sequence of the rep 78 cassette is functional. Optionally, the nucleotide sequence encoding a functional Rep 78 protein has at least 90%, at least 95%, at least 98%, at least 99%, or 100% identity to the full length or a fragment of at least 1500, at least 1600, at least 1700, at least 1800, or at least 1850 nucleotides of SEQ ID NO: 8. Optionally, the nucleotide sequence encoding a functional Rep 78 protein has at least 90%, at least 95%, at least 98%, at least 99%, or 100% identity to the full length or a fragment of at least 1500, at least 1600, at least 1700, at least 1800, or at least 1850 nucleotides of SEQ ID NO: 8 and the Rep 78 protein is functional.

Optionally, the nucleotide sequence encoding a functional Rep 78 protein has at least 98% identity to a fragment of at least 1800 nucleotides of SEQ ID NO: 8. Optionally, the nucleotide sequence encoding a functional Rep 78 protein has at least 99% identity to a fragment of at least 1850 nucleotides of SEQ ID NO: 8. Optionally, the nucleotide sequence encoding a functional Rep 78 protein has at least 98%, or at least 99% identity to SEQ ID NO: 8. Optionally, the nucleotide sequence encoding a functional Rep 78 protein encodes a protein that has at least 90%, at least 95%, at least 98%, at least 99%, or 100% identity to the full length or a fragment of at least 500, at least 550, at least 560, at least 570, or at least 575 amino acids of SEQ ID NO: 22. Optionally, the nucleotide sequence encoding a functional Rep 78 protein encodes a protein that has at least 98%, at least 99%, or 100% identity to the full length or a fragment of at least 570 amino acids of SEQ ID NO: 15. Optionally, the nucleotide sequence encoding a functional Rep 78 protein encodes a protein that has at least 98% identity to SEQ ID NO: 15. Optionally, the rep 78 cassette is identical to SEQ ID NO: 8.

Optionally, the rep 78 cassette comprises a nucleotide sequence that has at least 90%, at least 95%, at least 98%, at least 99%, or 100% identity to the full length or a fragment of at least 1500, at least 1600, at least 1700, at least 1800, or at least 1900 nucleotides of SEQ ID NO: 9,. Optionally, the rep 78 cassette comprises a nucleotide sequence that has at least 99%, or 100% identity to the full length or a fragment of or at least 1900 nucleotides of SEQ ID NO: 9. Optionally, the rep 78 cassette comprises a nucleotide sequence that has at least 98%, at least 99%, or 100% identity to SEQ ID NO: 9.

As discussed above, the wild type rep encoding region of AAV encodes all of Rep 40, Rep 52, Rep 68, and Rep 78, and the genes encoding these 4 proteins overlap (see Figure 7). SEQ ID NO: 1 provides the sequence of the genome of wild type AAV2, and nucleotides 321-2252 of SEQ ID NO: 1 encode the four Rep proteins. The full length rep gene (nucleotides 321-2252) encodes all four Rep proteins (Rep 78 and Rep 68 from the p5 promoter and Rep 52 and Rep 40 from the p19 promoter). A shorter stretch of the rep gene downstream of the p19 promoter (nucleotides 993-2252) encodes Rep 52 and Rep 40 only (i.e. this stretch of the rep gene reaches from the end of the p19 promoter to the end of the gene). Nucleotides 1907-2227 of SEQ ID NO: 1 correspond to an intron. Rep 78 and Rep 52 comprise amino acids encoded by the intron, but Rep 68 and Rep 40 are alternative splice variants that do not comprise amino acids encoded by the intron. The relationship between the rep gene and the four Rep proteins is shown in Figures 7. SEQ ID NO: 9 corresponds to nucleotides 321-2252 of SEQ ID NO: 1. Optionally, the rep 78 cassette comprises a nucleotide sequence that has at least 90%, at least 95%, at least 98%, at least 99%, or 100% identity to the full length or a fragment of at least 1500, at least 1600, at least 1700, at least 1800, or at least 1900 nucleotides of SEQ ID NO: 9 and encodes a functional Rep 52 protein, a functional Rep 40 protein and a functional Rep 68 protein. Optionally, the rep 78 cassette comprises a nucleotide sequence that has at least 99%, or 100% identity to the full length or a fragment of at least 1900 nucleotides of SEQ ID NO: 9 and encodes a functional Rep 52 protein, a functional Rep 40 protein and a functional Rep 68 protein. Optionally, the rep 78 cassette comprises a nucleotide sequence that has at least 98%, at least 99%, or 100% identity to SEQ ID NO: 9 and encodes a functional Rep 52 protein, a functional Rep 40 protein and a functional Rep 68 protein.

It is not necessary for all four Rep proteins to be present. Accordingly, the helper plasmid may further comprise at least one rep gene encoding (in addition to Rep 78):
(a) a functional Rep 52 protein;
(b) a functional Rep 40 protein; and/or
(c) a functional Rep 68 protein.

A *"functional"* Rep 52 protein, Rep 40 protein and/or Rep 68 protein is one which allows for production of AAV particles. In particular, Rep 68 and Rep 78 (the large Rep proteins) are believed to be involved in replication of the AAV genome, and Rep 52 and Rep 40 (the small Rep proteins) are believed to be involved in packaging of the AAV genome into a capsid. It is within the abilities of the skilled person to determine whether a given Rep protein is functional. The skilled person merely needs to determine whether the Rep protein supports AAV production using an AAV production assay as described above. In this case, the test two-plasmid system will comprise a helper plasmid that comprises the Rep protein whose *"functionality"* is to be determined, and otherwise the test two-plasmid system used will be identical to the reference two-plasmid system.

In an embodiment, the at least one rep gene of the helper plasmid encodes a *"functional"* Rep protein if the Rep protein supports rAAV production at a level at least 25%, at least 40%, at least 50%, at least 70%, at least 80%, at least 90%, or at least 95% of the level supported by the wild type Rep protein, i.e. if the yield of rAAV vector genomes produced is at least 25%, at least 40%, at least 50%, at least 70%, at least 80%, at least 90% or at least 95% of the yield of rAAV vector genomes produced using the reference two-plasmid system. Preferably, the at least one rep gene of the helper plasmid is functional if it supports rAAV production at a level at least 80% of the level supported by the wild type Rep protein.

In general, a Rep protein will only be able to support rAAV production if it is compatible with the ITR(s) surrounding the genome of the AAV to be packaged. Some Rep proteins may only be able to package genomic material (such as an expression cassette) when it is flanked by ITR(s) of the same serotype as the Rep protein. Other Rep proteins are cross-compatible, meaning that they can package genomic material that is flanked by ITR(s) of a different serotype. For example, in the case of a two-plasmid system, it is preferred that the Rep protein is able to support replication and packaging of an expression cassette comprised within the vector plasmid, and such a Rep protein will be compatible with the at least one ITR flanking the expression cassette (i.e. able to replicate and package the expression cassette flanked on at least one side by an ITR).

Optionally, the at least one rep gene comprises a gene encoding a functional Rep 52 protein, a gene encoding a functional Rep 40 protein, and a gene encoding a functional Rep 68 protein.

The at least one rep gene (and therefore the helper plasmid) may comprise only a single rep gene in the form of the rep 78 cassette, because, as discussed above, the rep 78 cassette may comprise nucleotides corresponding to all or a portion of nucleotides 204-2252 of AAV2 which encode at least a portion of all four rep proteins (Rep 40, Rep 52, Rep 68 and Rep 78). If there is only a single rep gene, the gene encoding a functional Rep 52 protein, the gene encoding a functional Rep 40 protein, the gene encoding a functional Rep 68 protein, and the gene encoding a functional Rep 78 protein all contain a common portion (i.e. are at least partially encoded by the same nucleotide sequence). For example, nucleotides 993-1710 of AAV2 is part of the coding sequence of all four rep proteins, and could correspond to a *"common portion".* Optionally, therefore, the common portion comprises a sequence having at least 90%, at least 95%, at least 98%, at least 99%, or 100% identity to the full length or a fragment of at least 500, at least 550, at least 600, at least 650, or at least 700 nucleotides of SEQ ID NO: 14. Optionally, the common portion comprises a sequence having at least at least 98%, at least 99%, or 100% identity to the full length or a fragment of at least 650, or at least 700 nucleotides of SEQ ID NO: 14. Optionally, the common portion comprises a sequence having at least 98%, at least 99%, or 100% identity to SEQ ID NOs: 14. Optionally, the common portion encodes an amino acid sequence having at least 90%, at least 95%, at least 98%, at least 99%, or 100% identity to the full length or a fragment of at least 150, at least 210, at least 220, at least 230, or at least 238 amino acids of SEQ ID NO: 16.

If the at least one rep gene comprises only a single rep gene in the form of the rep 78 cassette, the entire at least one rep gene will be relatively small in size. For example, a region of AAV2 from nucleotide 204 to nucleotide 2252 encodes all four rep proteins and includes the p5 promoter. Optionally, the rep 78 cassette comprises fewer than 2400, fewer than 2300, fewer than 2200, or fewer than 2100 nucleotides, and encodes a functional Rep 52 protein, a functional Rep 40 protein, a functional Rep 68 protein and a functional Rep 78 protein. Optionally, the rep 78 cassette comprises fewer than 2200, or fewer than 2100 nucleotides, and encodes a functional Rep 52 protein, a functional Rep 40 protein, a functional Rep 68 protein and a functional Rep 78 protein. Optionally, the at least one rep gene comprises fewer than 2400, fewer than 2300, fewer than 2200, or fewer than 2100 nucleotides, and encodes a functional Rep 52 protein, a functional Rep 40 protein, a functional Rep 68 protein and a functional Rep 78 protein. Optionally, the at least one rep gene comprises fewer than 2200, or fewer than 2100 nucleotides, and encodes a functional Rep 52 protein, a functional Rep 40 protein, a functional Rep 68 protein and a functional Rep 78 protein.

Optionally, the rep 78 cassette has at least 90%, at least 95%, at least 98%, at least 99%, or 100% identity to the full length or a fragment of at least 1500, at least 1600, at least 1700, at least 1800, at least 1900, or at least 2000 nucleotides of SEQ ID NO: 7. Optionally, the rep 78 cassette has at least 98%, at least 99%, or 100% identity to the full length or a fragment of at least 1900, or at least 2000 nucleotides of SEQ ID NO: 7. Optionally, the rep 78 cassette has at least 98%, at least 99%, or 100% identity to SEQ ID NO: 7.

Alternatively, the at least one rep gene may comprise two or more genes encoding functional Rep 68, Rep 58 and/or Rep 40 proteins. For example, the helper plasmid comprises a Rep 78 cassette that is modified to, for example, express Rep 78 at a reduced level. If the rep 78 cassette encodes Rep 68 and Rep 78 as overlapping genes (as in the wild type rep gene), reducing Rep 78 expression using a weak start codon will also reduce Rep 68 expression. For that reason, the user may wish to include an additional copy of Rep 68 to increase the Rep 68 expression level. For example, the rep gene may comprise a Rep 78 cassette and a second rep gene that could encode Rep 68 (for example using the p5 promoter or a different promoter situated near the normal position of the p5 promoter in the rep gene) and Rep 40 (for example using the p19 promoter or a different promoter situated near the normal position of the p19 promoter). In these embodiments, the second rep gene does not comprise an intron, and therefore cannot express rep 78. As indicated in Figure 7, the intron forms part of the Rep 78 and Rep 52 coding sequences.

Optionally, the helper plasmid comprises a gene encoding a functional Rep 52 protein, and the gene encoding a functional Rep 52 protein comprises a nucleic acid sequence having at least 95%, at least 98%, at least 99%, or 100% identity to the full length or a fragment of at least 800, at least 900, at least 1000, or at least 1100 nucleotides in length of nucleotides 993-2186 of SEQ ID NO: 1, or to a corresponding stretch of nucleotides in a different serotype of AAV.

It is within the abilities of the person skilled in the art to determine whether a particular (test) stretch of nucleotides is a *"corresponding stretch of nucleotides in a different serotype of"* AAV. All that is required is that the person skilled in the art align the test stretch of nucleotides with the genome of the reference serotype (i.e. SEQ ID NO: 1). If the test stretch of nucleotides has greater than 90% identity with a contiguous stretch of nucleotides of the same length in SEQ ID NO: 1, the contiguous stretch is a corresponding stretch of nucleotides in a different serotype of AAV. The same applies in the case of adenovirus sequences (except here the reference serotype is SEQ ID NO: 2).

Optionally, the at least one rep gene comprises at least one gene encoding a functional Rep 40 protein, and the gene encoding a functional Rep 40 protein comprises a nucleic acid sequence having at least 95%, at least 98%, at least 99%, or 100% identity to the full length or to a fragment of at least 900, at least 1000, at least 1100, or at least 1200 nucleotides in length of nucleotides 993-2252 of SEQ ID NO: 1, or to a corresponding stretch of nucleotides in a different serotype of AAV. Nucleotides 993-2252 of SEQ ID NO: 1 comprises an intron which does not encode part of the Rep 40 protein, but for the present purposes nucleotides 993-2252 will still be considered to encode the Rep 40 protein.

Optionally, the at least one rep gene comprises a gene encoding a functional Rep 68 protein, and the gene encoding a functional Rep 68 protein comprises a nucleic acid sequence having at least 95%, at least 98%, at least 99%, or 100% identity to the full length or to a fragment of at least 1000, at least 1400, at least 1500, or at least 1600 nucleotides in length of a stretch of nucleotides corresponding to nucleotides 321-2252 of SEQ ID NO: 1, or to corresponding stretches of nucleotides in a different serotype of AAV. Nucleotides 321-2252 of SEQ ID NO: 1 comprises an intron which does not encode part of the Rep 68 protein, but for the present purposes nucleotides 321-2252 will still be considered to encode the Rep 68 protein.

Optionally, the rep 78 cassette comprises a nucleic acid sequence having at least 95%, at least 98%, at least 99%, or 100% identity to the full length or to a fragment of at least 1700, at least 1800, or at least 1900 nucleotides in length of a stretch of nucleotides corresponding to nucleotides 321-2252 of SEQ ID NO: 1, or to corresponding stretches of nucleotides in a different serotype of AAV.

In some embodiments, the at least one rep gene does not comprise a functional internal p40 promoter. The native rep/cap gene comprises a p40 promoter (D.J. Pereira and N. Muzyczka (1997), J. Virol. 71 :1747-1756). The p40 promoter drives expression of the cap gene, but is not required for expression of rep genes. A functional p40 promoter is one that is capable to drive expression of the cap gene.

Whether or not a given p40 promoter is functional may be determined by testing its ability to drive expression of a protein (using an expression assay). For example, a user may prepare a *"reference"* vector comprising a native p40 promoter (such as that of nucleotides 1710-1827 of SEQ ID NO: 1) upstream of a reporter protein such as GFP. The user may then prepare a "*test*" vector that is identical to the reference vector except that the native p40 promoter is replaced by the p40 promoter whose functionality is to be tested (the "*test*" p40 promoter). The two vectors may be transfected into suitable host cells, and the host cells incubated under conditions suitable for expression of the reporter protein. The level of reporter protein expressed in the host cells could be measured by, for example, fluorescence spectroscopy. The level of expression from the reference vector (corresponding to the level of expression driven by the native p40 promoter) can then be compared to the level of expression from the test vector (corresponding to the level of expression driven by the test promoter).

A p40 promoter will be considered to be functional if it drives expression at a level at least 40% of the expression driven by the native p40 promoter. Optionally, a functional p40 promoter drives expression at a level at least 60%, at least 70%, at least 80%, at least 90%, or at least 95% of the expression driven by the native p40 promoter. Optionally, the at least one rep gene does not comprise a functional internal p40 promoter if it does not comprise a sequence corresponding to the p40 promoter (for example nucleotides 1710-1827 of SEQ ID NO: 1) that drives expression at a level at least 60%, at least 70%, at least 80%, at least 90%, or at least 95% of the expression driven by the native p40 promoter.

P40 promoters lacking a *"TATA box"* are non-functional. The p40 promoter comprises a TATA box starting at a position corresponding to position 1823 of SEQ ID NO: 1. Thus, the helper plasmid that does not comprise a functional internal p40 promoter may comprise one or more p40 promoters in which the TATA boxes are mutated to render the p40 promoter non-functional.

Optionally, the at least one rep gene does not comprise a T nucleotide at a position corresponding to position 1823 of SEQ ID NO: 1. Optionally, the at least one rep gene comprises a C nucleotide at a position corresponding to position 1823 of SEQ ID NO: 1. Optionally, the at least one rep gene does not comprise AAG at positions corresponding to positions 1826-1828 of SEQ ID NO: 1. Optionally, the at least one rep gene comprises CTC at positions corresponding to positions 1826-1828 of SEQ ID NO: 1.

### At least one helper virus gene

The helper plasmid may comprise at least one helper virus gene. AAV is only able to propagate in the presence of a helper virus. Examples of helper viruses include adenoviruses and herpes viruses.

However, as set out above, growing AAV in the presence of a helper virus is not advantageous as helper viruses can be lytic to cells, including host cells used to grow AAV. Furthermore, if helper viruses are used in the production of rAAV products, such as gene therapy vectors, the helper virus may contaminate the product. As an alternative to co-infection with a helper virus such as adenovirus, the requisite genes of the helper virus can be provided on a transfected plasmid. For host cells expressing the adenoviral E1A/B genes (such as HEK293T cells) the remaining required helper genes are adenoviral E4, E2A and VA RNA I and II (a VA nucleic acid). While these genes are distributed across a long stretch of the adenoviral genome, the present inventors have determined that large stretches of non-coding nucleotides separating the genomic genes can be removed without affecting expression of the genes. The inventors have, therefore, designed a minimal helper gene region, which is SEQ ID NO: 4. SEQ ID NO: 4 contains the VA nucleic acid, and the reverse complement (as present in a double stranded plasmid) of an E2A gene and an E4 gene. Using such a minimal helper gene region in a plasmid for the production of rAAV is advantageous as it allows the user to use a smaller plasmid which is less costly to produce and easier to transfect into the cell.

In some embodiments, the helper plasmids of the invention comprise at least one helper virus gene. Preferably, the helper plasmids of the invention comprise sufficient helper genes to allow for AAV replication and packaging. Whether or not a helper plasmid comprises sufficient helper genes to facilitate AAV production can be assessed using an AAV production assay as described above. In this case, the test two-plasmid system will comprise a helper plasmid that comprises the helper genes whose ability to facilitate AAV production is to be tested, and otherwise the test two-plasmid system used will be identical to the reference two-plasmid system. In one embodiment, the helper gene products will be considered to facilitate AAV production if they support rAAV production at a level at least 25%, at least 40%, at least 50%, at least 60%, at least 70%, at least 80%, at least 90%, or at least 95% of the level supported by adenoviral helper genes encoding E4, E2A and VA RNA I and II, *i.e.* if the yield of rAAV produced is at least 25%, at least 40%, at least 50%, at least 60%, at least 70%, at least 80%, at least 90%, or at least 95% of the yield of rAAV produced using the reference two-plasmid system. Preferably, the helper gene products will be considered to facilitate AAV production if they support rAAV production at a level at least 70%, at least 80%, at least 90%, or at least 95% of the level supported by adenoviral helper genes encoding E4, E2A and VA RNA I and II.

Since the helper plasmids encode the genes required to allow for efficient AAV production, the addition of a helper virus is not required.

Optionally, the at least one helper virus gene is an adenovirus gene. Adenovirus is a virus which is known to aid propagation of AAV (Xiao et al (1998), J. Virol, 72:2224-2232). Optionally, the at least one helper virus gene is an Adenovirus 5 gene or an Adenovirus 2 gene. The genome of Adenovirus 5 is set out in SEQ ID NO: 2, and the genome of Adenovirus 2 is set out in SEQ ID NO: 3. Accordingly, the helper genes may comprise a stretch of nucleotides present in SEQ ID NO: 2 and/or SEQ ID NO: 3, or a corresponding stretch of nucleotides in another serotype of adenovirus.

The helper genes of adenoviruses encode E1A, E1B, E4, E2A and VA RNA I and II.

For example, E1A is encoded by nucleotides 560-1545 of the Adenovirus 5 genome (for example the genome of SEQ ID NO: 2). Nucleotides 560-1545 contain an intron, from nucleotide 1113 to nucleotide 1228. This intron is not essential, and so an E1A gene comprising nucleotides 560-1112 and 1229-1545 of SEQ ID NO: 2 would encode a functional E1A protein.

E1B is actually two proteins E1B 19K and E1B 55K, which work together to block apoptosis in adenovirus-infected cells. E1B is encoded by nucleotides 1714-2244 (E1B 19K), and by nucleotides 2019-3509 (E1B 55K) of the Adenovirus 5 genome (for example the genome of SEQ ID NO: 2).

E4 is encoded by a number of different open reading frames (ORFs) of the Adenovirus 5 genome (for example the genome of SEQ ID NO: 2). E4 ORF 6/7 is encoded by nucleotides 32914-34077, which comprises an intron between nucleotides 33193 and 33903. This intron is not essential, and so an E4 ORF 6/7 comprising nucleotides 32914-33192 and 33904-34077 of SEQ ID NO: 2 is sufficient. E4 34K is encoded by nucleotides 33193-34077 of the Adenovirus 5 genome (for example the genome of SEQ ID NO: 2). E4 ORF 4 is encoded by nucleotides 33998-34342 of the Adenovirus 5 genome (for example the genome of SEQ ID NO: 2). E4 ORF 3 is encoded by nucleotides 34353-34703 of the Adenovirus 5 genome (for example the genome of SEQ ID NO: 2). E4 ORF B is encoded by nucleotides 34700 to 35092 of the Adenovirus 5 genome (for example the genome of SEQ ID NO: 2). E4 ORF 1 is encoded by nucleotides 35140-35526 of the Adenovirus 5 genome (for example the genome of SEQ ID NO: 2).

A functional E4 protein may only comprise amino acids encoded by ORFs 6 and 7, as only the amino acids encoded by ORFs 6 and 7 are required for activity. Optionally, therefore, the functional E4 protein comprises a polypeptide sequence encoded by all or a significant portion of ORFs 6 and 7. In an embodiment the E4 gene comprises all or a significant portion of ORFs 6 and 7, for example at least 90%, at least 95%, at least 98% or 100% of ORFs 6 and 7. Optionally, the functional E4 protein does not comprise polypeptide sequence encoded by all or a portion of ORFs 1-4 and 34K. However, the amino acids encoded by ORFs 1-3 and 34K do improve the activity of the E4 protein, and so in some embodiments the functional E4 protein comprises amino acids encoded by ORFs 1-7.

The E2 (E2A) gene is encoded by nucleotides 22443-24032 of the Adenovirus 5 genome (for example the genome of SEQ ID NO: 2).

The VA RNA I and II is encoded by nucleotides 10589-11044 of the Adenovirus 5 genome (for example the genome of SEQ ID NO: 2).

E1B and E4 are believed to enhance AAV mRNA accumulation, and E2A and VA RNA I and II are believed to enhance AAV mRNA splicing and translation. E1B, E4 and E2A are proteins encoded by genes present in the adenovirus genome, whereas the VA nucleic acid encodes two RNA transcripts known as VA RNA I and VA RNA II. The transcripts themselves are functional in the cell, and are never translated into amino acid sequences. It will be appreciated, therefore, that the VA nucleic acid does not encode a protein, but does *"encode"* or *"correspond to"* an RNA, i.e. whilst the term *"encode"* is used, the VA nucleic acid is a non-translated nucleic acid sequence.

Of the five adenovirus genes, it is possible not to include E1A or E1B in the helper plasmid, as some host cell lines (such as HEK293 cells) express one or more of E1A or E1B constitutively. Optionally, therefore, the helper plasmid does not comprise a gene encoding a functional adenoviral E1A/B protein.

In one embodiment, the at least one helper virus gene comprises:
(a) a VA (viral associated) nucleic acid encoding functional VA RNA I and II;
(b) an E2A gene encoding a functional E2A protein; and/or
(c) an E4 gene encoding a functional E4 protein.

Optionally, the at least one helper virus gene comprises a VA nucleic acid, an E2A gene and an E4 gene.

A *"functional"* VA RNA I and II, E2A protein or E4 protein is able to facilitate production of AAV. It is within the abilities of the skilled person to determine whether a given VA RNA I and II, E2A protein or E4 protein is functional. The skilled person merely needs to determine whether the VA RNA I and II, E2A protein or E4 protein supports AAV production using an AAV production assay as described above. In this case, the test two-plasmid system will comprise a helper plasmid that comprises the VA RNA I and II, E2A protein or E4 protein whose *"functionality"* is to be determined, and otherwise the test two-plasmid system used will be identical to the reference two-plasmid system. In an embodiment, the VA RNA I and II, E2A protein or E4 protein will be considered to be *"functional"* if it supports rAAV production at a level at least 25%, at least 40%, at least 50%, at least 70%, at least 80%, at least 90%, or at least 95% of the level supported by the wild type (for example as found in native Adenovirus 5; SEQ ID NO: 2) VA RNA I and II, E2A protein or E4 protein, i.e. if the yield of rAAV produced is at least 25%, at least 40%, at least 50%, at least 70%, at least 80%, at least 90%, or at least 95% of the yield of rAAV produced using the reference two-plasmid system. Preferably, the E4 protein will be considered to be *"functional"* if it supports rAAV production at a level at least 70%, at least 80%, at least 90%, or at least 95% of the level supported by the wild type E4 protein.

Optionally, the E4 gene is not located between the VA nucleic acid and the E2A gene, i.e. the sequence of the plasmid is such that E4 gene sequence does not appear in the plasmid between the VA nucleic acid sequence and the E2A gene sequence. Optionally, the E2A gene is located between the VA nucleic acid and the E4 gene.

Optionally, the E4 gene encodes an E4 protein having an amino acid sequence that is at least 90%, at least 95%, at least 98%, at least 99% or 100% identical to SEQ ID NO: 17. SEQ ID NO: 17 corresponds to the amino acid sequence encoded by ORFs 6/7 (nucleotides 32914-33192 and 33904-34077 of SEQ ID NO: 2). Optionally, the E4 gene encodes an E4 protein having an amino acid sequence that is at least 98% identical to SEQ ID NO: 17.

Optionally, the E2A gene encodes an E2A protein having an amino acid sequence that is at least 90%, at least 95%, at least 98%, at least 99% or 100% identical to SEQ ID NO: 18. SEQ ID NO: 18 corresponds to the amino acid sequence of the AAV2 E2A protein encoded by nucleotides 22443-24032 of SEQ ID NO: 2. Optionally, the E2A gene encodes an E2A protein having an amino acid sequence that is at least 98% identical to SEQ ID NO: 18.

The helper plasmid is double stranded, and any of the genes comprised within the helper plasmid may be present in a *"forward'* or "reverse" orientation. For example, the E4 gene may comprise nucleotides 22443-24032 of the Adenovirus 5 genome (for example the genome of SEQ ID NO: 2) or may comprise the reverse complement of nucleotides 22443-24032. Thus, in all instances of the application where reference is made to a plasmid *"comprising"* a certain nucleic acid sequence, the references should be interpreted as encompassing embodiments where the plasmid comprises the reverse complement of the nucleic acid sequence.

The present inventors have demonstrated that removing all or part of the stretch of nucleotides present at positions 10595-10619 in the adenovirus genome (for example the genome of SEQ ID NO: 2) significantly reduces (by about 50%) the activity of the VA RNA I and II encoded by the VA nucleic acid. Avoiding this reduction in activity is advantageous.

Accordingly, in one embodiment, the VA nucleic acid has an activity level which has at least 75%, at least 80%, at least 90%, at least 95%, or between 95% and 100% of the activity of a wild type VA nucleic acid from Adenovirus 5. Optionally, the activity level of the VA nucleic acid is determined by measuring rAAV yield, for example using the AAV production assay described above. In an embodiment, the VA nucleic acid comprises a contiguous sequence at least 95%, at least 98%, or 100% identical to a stretch of at least 15 nucleotides, at least 20 nucleotides, or 25 nucleotides of nucleotides 10595-10619 of SEQ ID NO: 2, or a corresponding stretch of nucleotides in a different serotype of adenovirus.

Optionally, the E2A gene is operably linked to a promoter which comprises a contiguous sequence at least 96%, at least 98%, or 100% identical to a stretch of at least 60, at least 70, at least 80, or 100 nucleotides of nucleotides 27037-27136 of SEQ ID NO: 2, or a corresponding stretch of nucleotides in a different serotype of adenovirus.

Expression of the E4 gene is driven by a promoter (designated *"E4 promoter"* herein), corresponding to nucleotides 35585-35848 of SEQ ID NO: 2. Nucleotides corresponding to 35793-35848 of the Adenovirus 5 genome (such as a genome of SEQ ID NO: 2), are required for the promoter to be fully active. Optionally, therefore, the E4 gene is operably linked to an E4 promoter that has at least 50%, at least 70%, or at least 90% of the activity of a wild type promoter from Adenovirus 5. The activity of the E4 promoter may be determined by testing its ability to drive expression of a protein.

Whether or not a given E4 promoter is able to drive E4 gene expression may be determined using an AAV production assay as described above. Specifically, if the E4 promoter can drive E4 gene expression it will facilitate AAV production. In this case, the test two-plasmid system will comprise a vector plasmid that comprises the E4 gene and an E4 gene promoter whose ability to facilitate AAV production is to be tested, and otherwise the test two-plasmid system used will be identical to the reference two-plasmid system. In one embodiment, the E4 promoter will be considered to have at least 25%, at least 40%, at least 50%, at least 70%, or at least 90% of the activity of a wild type E4 promoter from Adenovirus 5 if it supports rAAV production at a level at least 25%, at least 40%, at least 50%, at least 60%, at least 70%, or at least 90% of the level supported by a wild type E4 gene promoter, i.e. if the yield of rAAV produced is at least 50%, at least 70%, or at least 90% of the yield of rAAV produced using the reference two-plasmid system. Preferably, the E4 promoter will be considered to facilitate AAV production if it supports rAAV production at a level at least 70%, at least 80%, at least 90% or at least 95% of the yield of rAAV produced using the reference two-plasmid system.

Optionally, the E4 promoter comprises a sequence of at least 30, at least 40, or 55 nucleotides corresponding to nucleotides 35793-35848 of SEQ ID NO: 2, or a corresponding stretch of nucleotides in a different serotype of adenovirus.

### Reducing the size of the helper plasmid

The present inventors have determined that there is a significant amount of non-helper gene nucleic acid sequence between the different helper gene sequences in native Adenovirus 5, and that a substantial portion of this nucleic acid sequence can be removed without significantly impacting the expression level and function of the proteins encoded by the helper gene sequences. Accordingly, the inventors have succeeded in defining a helper gene region with a reduced size. Having a reduced size helper gene region (corresponding to the at least one helper virus gene) is advantageous as it allows the overall size of the helper plasmid to be reduced. Smaller plasmids are cheaper to produce and easier to introduce into host cells.

Accordingly, the helper plasmid may be less than 20000 bp, less than 15000 bp, less than 14000 bp, less than 13000bp, between 10000 bp and 20000 bp, between 11000 bp and 15000 bp, between 12000 bp and 13000 bp, around 12941 bp, or around 12668 bp in length.

Similarly, the at least one helper virus gene comprises a VA nucleic acid, an E2A gene and an E4 gene, and the VA nucleic acid, the E2A gene and the E4 gene are comprised within a contiguous stretch of nucleotides on the helper plasmid of fewer than 15000, fewer than 12000, fewer than 10000, fewer than 9000, or fewer than 8500 nucleotides.

An example of a helper gene region with a reduced size is set out in SEQ ID NO: 4. The helper gene region is a contiguous region of the plasmid starting at the first nucleotide encoding a helper gene and ending at the last nucleotide encoding a helper gene. Optionally, the helper gene region comprises a sequence having at least 95%, at least 98%, at least 99%, or 100% identity to the full length or to a fragment at least 6000 nucleotides, at least 7000 nucleotides, or at least 8000 nucleotides in length of SEQ ID NO: 4, i.e. the at least one helper virus gene is comprised on a contiguous stretch of the plasmid having at least 95%, at least 98%, at least 99%, or 100% identity to the full length or to a fragment at least 6000 nucleotides, at least 7000 nucleotides, or at least 8000 nucleotides in length, of SEQ ID NO: 4. Optionally, the helper gene region consists of a sequence having at least 95%, at least 98%, at least 99%, or 100% identity to the full length or to a fragment at least 6000 nucleotides, at least 7000 nucleotides, or at least 8000 nucleotides in length of SEQ ID NO: 4, i.e. the at least one helper virus gene is comprised on a contiguous stretch of the plasmid consisting of a sequence at least 95%, at least 98%, at least 99%, or 100% identical to the full length or to a fragment at least 6000 nucleotides, at least 7000 nucleotides, or at least 8000 nucleotides in length of SEQ ID NO: 4.

The present inventors have identified specific regions of the Adenovirus 5 genome that can be excluded from the helper plasmid.

In one embodiment, the helper plasmid does not comprise a contiguous sequence of at least 2000, at least 2500, at least 3000, or 3427 nucleotides of a contiguous stretch of nucleotides of equivalent length comprised within nucleotides 194-3620 of SEQ ID NO: 2, or a corresponding stretch of nucleotides in a different serotype of adenovirus. In one embodiment, the helper plasmid does not comprise a contiguous sequence of at least 50, at least 60, or 69 nucleotides of a contiguous stretch of nucleotides of equivalent length comprised within nucleotides 4032-4100 of SEQ ID NO: 2, or a corresponding stretch of nucleotides in a different serotype of adenovirus.

In an embodiment, the helper plasmid does not comprise a contiguous sequence of at least 15000, at least 20000, at least 22000, or 22137 nucleotides of a contiguous stretch of nucleotides of equivalent length comprised within nucleotides 10619-32755 of SEQ ID NO: 2, or a corresponding stretch of nucleotides in a different serotype of adenovirus.

In an embodiment, the helper plasmid does not comprise a contiguous sequence of at least 15000, at least 20000, at least 21000, or 21711 nucleotides of a contiguous stretch of nucleotides of equivalent length comprised within nucleotides 11045-32755 of SEQ ID NO: 2, or a corresponding stretch of nucleotides in a different serotype of adenovirus.

Similarly, the inventors have identified regions of the AAV2 genome that do not need to be included in the helper plasmid and may accordingly be excluded in the interests of minimising the size of the helper plasmid. Accordingly, in one embodiment, the helper plasmid does not comprise a contiguous sequence of at least 200, at least 300, at least 350, or 363 nucleotides of a contiguous stretch of nucleotides of equivalent length comprised within nucleotides 4051-4413 of SEQ ID NO: 1, or a corresponding stretch of nucleotides in a different serotype of AAV. In one embodiment, the helper plasmid does not comprise a contiguous sequence of at least 200, at least 300, at least 350, or 361 nucleotides of a contiguous stretch of nucleotides of equivalent length comprised within nucleotides 4051-4411 of SEQ ID NO: 1, or a corresponding stretch of nucleotides in a different serotype of AAV. Similarly, in one embodiment, the helper plasmid does not comprise a contiguous sequence of at least 400, at least 500, at least 600, or 647 nucleotides of a contiguous stretch of nucleotides of equivalent length comprised within nucleotides 2301-2947 of SEQ ID NO: 1, or a corresponding stretch of nucleotides in a different serotype of AAV.

### Artificial rep binding sites

Rep proteins bind to a rep binding site (RBS), which is a nucleic acid sequence having a consensus sequence of GCTCGCTCGCTCGCTC (SEQ ID NO: 6) (McCarty, D. M. et al. (1994), J. Virol., 68(8): 4988-4997)). Accordingly, a rep binding site is a nucleic acid sequence having homology to GCTCGCTCGCTCGCTC (SEQ ID NO: 6). Determining whether a nucleic acid sequence (test nucleic acid sequence) comprises a rep binding site, for example, determining whether a nucleic acid sequence comprises a sequence having homology to GCTCGCTCGCTCGCTC (SEQ ID NO: 6), can be carried out using an electrophoretic mobility shift assay (EMSA). The test nucleic acid sequence is applied to a first well of a gel suitable for use in electrophoresis, and a mixture of the test nucleic acid sequence and Rep68/Rep78 proteins is applied to a second well of the gel. If the test nucleic acid sequence comprises a rep binding site, this will cause a shift in the mobility of the nucleic acid sequence in the well comprising the Rep68/Rep78 proteins compared to the well that lacked the Rep68/Rep78 proteins.

Binding of Rep proteins to aberrant RBSs might result in Rep-mediated non-homologous recombination between different sequences comprising RBSs, resulting in undesired fusion sequences. For example, Rep proteins could bind the RBS in AAV ITRs and any RBS present on a bacterial plasmid backbone resulting in the fusion of plasmid backbone sequences to an ITR. This could result in enhanced replication and packaging of such undesired fusion sequences.

Accordingly, it is preferred that the only RBSs present in the plasmids of the invention correspond to RBSs present in wild type AAV (allowing the Rep proteins to carry out their normal function). However, when a plasmid is constructed it is possible that an RBS will be present, either in the plasmid backbone or elsewhere in the plasmid in non-AAV-derived sequences, and such sequences are considered to be *"artificial"* or *"aberrant"* RBSs, and should be avoided/removed.

A common source of artificial RBSs in plasmids is from the plasmid backbone. The plasmid backbone is the bacterial sequence needed to amplify the plasmids in bacterial host cells. The plasmid backbone may be the region of the plasmid that is not derived from adenovirus or AAV or is not situated between two AAV-derived ITRs. Optionally, the helper plasmid backbone encompasses any nucleotides except the at least one helper gene and associated adenovirus-derived regulatory elements, the at least one rep gene and associated AAV-derived regulatory elements, and one or more promoters operably linked to the at least one rep gene. In one embodiment, the helper plasmid comprises a backbone, and the plasmid backbone does not comprise an artificial RBS.

Accordingly, in one embodiment, the helper plasmid does not comprise an artificial RBS. Preferably, the helper plasmid do not comprise an artificial RBS. Optionally, there are no RBSs in the helper plasmid, except within any ITR(s), p5 promoter(s) and p19 promoter(s).

### Lack of a cap gene

A helper plasmid of the invention does not comprise a cap gene encoding a functional set of Cap proteins.

In native AAV (such as an AAV having a genome as set out in SEQ ID NO: 1), the cap and the rep genes are overlapping. In particular, the p40 promoter, which drives expression of the cap gene is present within the rep gene. Thus, any helper plasmid that encodes Rep proteins will comprise nucleotides from the cap gene. However, a helper plasmid of the invention does not comprise a stretch of nucleotides encoding a functional set of Cap proteins. In particular, a helper plasmid of the invention may not comprise a significant stretch of nucleotide sequence that is exclusively cap gene sequence. The phrase *"exclusively cap gene sequence"* is intended to refer to gene sequence that encodes a portion of a Cap protein, but does not encode a portion of a Rep protein, for example nucleotides 2253-4410 of SEQ ID NO: 1 or 2300-4410 of SEQ ID NO: 1.

Cap proteins (VP1, VP2 and VP3) are proteins that assemble to form a capsid surrounding the viral genome. Thus, a gene encoding a functional set of Cap proteins is one that encodes Cap proteins capable of assembling to encapsidate a viral genome.

A *"functional"* set of Cap proteins is one which is sufficient for encapsidation of AAV. It is within the abilities of the skilled person to determine whether the product of a cap gene is functional. The skilled person merely needs to determine whether the encoded Cap protein(s) support AAV production using an AAV production assay as described above. In this case, the test two-plasmid system will comprise a vector plasmid that comprises the cap gene which encodes the protein(s) whose *"functionality"* is to be determined, and otherwise the test two-plasmid system used will be identical to the reference two-plasmid system. The helper plasmid does not comprise a cap gene encoding a functional set of Cap proteins if it does not comprise any genetic material corresponding to a cap gene, or if any cap gene present in the helper plasmid encodes protein(s) which support AAV production at a level below 10% of the level supported by the wild type cap gene product, i.e. if the yield of rAAV produced is less than 10% of the yield of rAAV produced using the reference two-plasmid system.

In general, a functional cap gene is greater than 400 nucleotides in length. Accordingly, the helper plasmid, which does not comprise a cap gene encoding a functional set of Cap proteins, may not comprise a contiguous stretch of exclusively cap gene sequence of more than 400, more than 250 nucleotides, more than 100 nucleotides, or more than 60 nucleotides. As discussed above, the cap gene and the rep gene in native AAV overlap. Optionally, the helper plasmid does not comprise a contiguous stretch of exclusively cap gene sequence of more than 60 nucleotides. Optionally the helper plasmid does not comprise a cap gene encoding a functional VP1 protein. Optionally, the helper plasmid comprises a portion of cap gene sequence, and the portion of cap gene sequence does not encode a set of functional Cap proteins. The helper plasmid can comprise a portion of cap gene sequence, so long as it does not encode a functional set of Cap proteins. So long as the portion of cap gene sequence is not functional, multiple recombination events would be required to provide a rcAAV.

### A two-plasmid system

The present invention further provides a two-plasmid system comprising the helper plasmid and a vector plasmid.

The two-plasmid system is useful for producing rAAV. Optionally, the two-plasmid system is suitable for use in producing rAAV. Optionally, the two-plasmid system is for producing rAAV. Optionally, the two-plasmid system is for producing rAAV suitable for use in gene therapy. Optionally, the two-plasmid system is for producing rAAV for use in gene therapy.

The phrase *"two-plasmid system"* refers to a system that comprises only two plasmids, and can be used without the need for additional plasmids to produce rAAV. Optionally, the two-plasmid system can be used to produce rAAV without the need for helper virus such as adenovirus. Optionally, the two-plasmid system can be used to produce rAAV without the need for genetic material originating from a host cell, optionally with the exception of a gene encoding E1A/B. However, the system may comprise additional non-plasmid components. Optionally, the two-plasmid system does not comprise a helper virus. Optionally, the two-plasmid system of the invention comprises all the necessary genetic information for the production of rAAV. For example, the two-plasmid system of the invention may comprise at least one rep gene, at least one cap gene and at least one helper gene. Optionally, the two-plasmid system of the invention comprises all the necessary genetic information required for the production of rAAV suitable for use in gene therapy. For example, the two-plasmid system of the invention may comprise at least one rep gene, at least one cap gene, at least one helper gene and an expression cassette comprising a transgene operably linked to at least one regulatory control element. However, in embodiments the two-plasmid system of the invention may lack a functional cap gene (required for the production of rAAV) and/or an expression cassette comprising a transgene operably linked to at least one regulatory control element (required for the production of rAAV suitable for use in gene therapy).

It is an advantage of the present invention that a cap gene and/or a transgene in the vector plasmid may be exchanged with another in order to treat different genetic disorders. Optionally, therefore, the two-plasmid system of the invention may comprise all the necessary genetic information for the production of rAAV except a functional cap gene, and in such embodiments the two-plasmid system of the invention may comprise a site suitable for cloning in a cap gene. Such a site may comprise a cloning site adjacent to a cap gene promoter. The site suitable for cloning in a cap gene will be present on the vector plasmid. Optionally, the two-plasmid system of the invention comprises all the necessary genetic information for the production of rAAV suitable for use in gene therapy except a functional cap gene and an expression cassette comprising a transgene and a regulatory control element, wherein said two-plasmid system comprises a site suitable for cloning in a cap gene (such as a cap gene promoter and a cloning site operably linked, i.e. adjacent, to the cap gene promoter) and a site suitable for cloning in an expression cassette (such as a cloning site flanked by one or more ITRs). Optionally, the two-plasmid system of the invention comprises all the necessary genetic information for the production of rAAV suitable for use in gene therapy except an expression cassette comprising a transgene and a regulatory control element, wherein said two-plasmid system comprises a site suitable for cloning in an expression cassette (such as a cloning site flanked by one or more ITRs). In such cases the site suitable for cloning in a cap gene and the site suitable for cloning in an expression cassette will be present on the vector plasmid. Optionally, the two-plasmid system of the invention comprises the following components split between the vector plasmid and the helper plasmid:
- at least one rep gene encoding at least one functional Rep protein suitably a Rep 78 protein;
- at least one helper virus gene;
- a cap gene encoding at least one functional capsid protein, or a cap gene promoter and a cloning site operably linked to the cap gene promoter;
- at least one ITR; and
- an expression cassette comprising a transgene operably linked to at least one regulatory control element, or a site suitable for cloning in an expression cassette flanked on at least one side by (i.e. adjacent to) an ITR.

The vector plasmid may comprise:
(a) a cap gene encoding at least one functional Cap protein; or
(b) at least one cap gene promoter, a cloning site operably linked to the cap gene promoter, and an expression cassette flanked on at least one side by an ITR;
wherein the vector plasmid does not comprise a rep gene encoding a functional Rep protein and the expression cassette comprises a transgene operably linked to at least one regulatory control element.

Optionally, the two-plasmid system comprises a molar excess of helper plasmid compared to vector plasmid. Optionally, the ratio of helper plasmid to vector plasmid is between 1:10 to 10:1, between 1:3 and 7:3, between 1:3 and 6:3, preferably around 1:3 or around 2:3 or around 3:3 or around 4:3 or around 5:3, or around 6:3

As set out in more detail below, altering the ratio of helper plasmid to vector plasmid can be used to control the ratio of full to total particles (or proportion of capsids/particles that are full) produced during rAAV production, and/or to increase the yield of rAAV produced during rAAV production.

By the phrase *"ratio of helper plasmid to vector plasmid",* is meant a molar ratio, i.e. the ratio of the number of moles of helper plasmid present to the number of moles of vector plasmid present. A given ratio of helper plasmid to vector plasmid may be provided by simply mixing the helper plasmid and the vector plasmid in an appropriate molar ratio.

### Vector plasmid

As discussed above, the two-plasmid system of the invention may comprise a vector plasmid.

The vector plasmid may comprise:
(a) a cap gene encoding at least one functional Cap protein; or
(b) at least one cap gene promoter, a cloning site operably linked to the cap gene promoter, and an expression cassette flanked on at least one side by an ITR;
wherein the vector plasmid does not comprise a rep gene encoding a functional Rep protein and the expression cassette comprises a transgene operably linked to at least one regulatory control element.

Optionally, the vector plasmid does not comprise an artificial RBS. Optionally, the vector plasmid backbone encompasses any nucleotides except the cap gene, a promoter (region) operably linked to the cap gene, ITRs and the expression cassette. In one embodiment, the vector plasmid comprises a backbone, and the plasmid backbone does not comprise an artificial RBS. Optionally, the helper plasmid and the vector plasmid each comprise a backbone, and neither plasmid backbone comprises an artificial RBS.

Accordingly, in one embodiment, the vector plasmid does not comprise an artificial RBS. Preferably, the helper plasmid and the vector plasmid do not comprise an artificial RBS. Optionally, there are no RBSs in the vector plasmid and/or the helper plasmid, except within any ITR(s), p5 promoter(s) and p19 promoter(s).

### Cap gene

The vector plasmid may comprise a cap gene. The cap gene encodes a functional Cap protein. The cap gene may encode a functional set of Cap proteins. AAV generally comprise three Cap proteins, VP1, VP2 and VP3. These three proteins form a capsid into which the AAV genome is inserted, and allow the transfer of the AAV genome into a host cell. All of VP1, VP2 and VP3 are encoded in native AAV by a single gene, the cap gene. The amino acid sequence of VP1 comprises the sequence of VP2. The portion of VP1 which does not form part of VP2 is referred to as VP1unique or VP1U. The amino acid sequence of VP2 comprises the sequence of VP3. The portion of VP2 which does not form part of VP3 is referred to as VP2unique or VP2U.

A *"functional"* set of Cap proteins is one which allows for encapsidation of AAV. As discussed above, it is within the abilities of the skilled person to determine whether a given Cap protein is or a set of Cap proteins are functional. The skilled person merely needs to determine whether the encoded Cap protein(s) support AAV production using an AAV production assay as described above. In this case, the test two-plasmid system will comprise a vector plasmid that comprises the cap gene which encodes the Cap protein(s) whose *"functionality"* is to be determined, and otherwise the test two-plasmid system used will be identical to the reference two-plasmid system. The Cap protein(s) will be considered to be *"functional"* if it/they support(s) rAAV production at a level at least 25%, at least 40%, at least 50%, at least 70%, at least 80%, at least 90%, or at least 95% of the level supported by the wild type cap gene product, i.e. if the yield of rAAV produced is at least 25%, at least 40%, at least 50%, at least 70%, at least 80%, at least 90%, or at least 95% of the yield of rAAV produced using the reference two-plasmid system. Preferably, the Cap protein(s) will be considered to be *"functional"* if it/they support(s) rAAV production at a level at least 70%, at least 80%, at least 90%, or at least 95% of the level supported by the wild type cap gene product.

Optionally, VP2 and/or VP3 proteins are *"functional"* if an AAV comprising the VP2 and/or the VP3 proteins is able to transduce Huh7 cells at a level at least 25%, at least 40%, at least 50%, at least 70%, at least 80%, at least 90%, or at least 95% of that of an equivalent AAV comprising a wild type VP2 and/or VP3 protein. The ability of an AAV particle to transduce Huh7 cells can be tested by adding a reporter protein such as green fluorescent protein (GFP) to the AAV particle, mixing the AAV particle with Huh7 cells, and measuring the fluorescence produced.

Optionally, the vector plasmid comprises a cap gene that encodes a VP1, a VP2 and/or a VP3 protein. Optionally, the VP1, VP2 and VP3 proteins are expressed from more than one cap gene. Optionally, the vector plasmid comprises a cap gene that encodes a VP1, a VP2 and a VP3 protein. Optionally, the vector plasmid comprises a cap gene encoding a functional VP1, *i.e.* a VP1 protein capable of assembling with other Cap proteins to encapsidate a viral genome.

Different serotypes of AAV have Cap proteins having different amino acid sequences. A cap gene encoding any (set of) Cap protein(s) is suitable for use in connection with the present invention. The Cap protein can be a native Cap protein expressed in AAV of a certain serotype. Alternatively, the Cap protein can be a non-natural, for example an engineered, Cap protein, which is designed to comprise a sequence different to that of a native AAV Cap protein. Genes encoding non-natural Cap proteins are particularly advantageous, as in the context of gene therapy applications it is possible that fewer potential patients have levels of antibodies that prevent transduction by AAV comprising non-natural Cap proteins, relative to native capsids.

Optionally, the cap gene encodes a Cap protein from a serotype selected from the group consisting of serotypes 1, 2, 3A, 3B, 4, 5, 6, 7, 8, 9, 10, 11, 12, or 13, or hybrids or chimeras thereof. Optionally, the cap gene encodes a Cap protein from a serotype selected from the group consisting of serotypes 2, 5, 8, and 9. Optionally, the cap gene encodes a Cap protein selected from the group consisting of LK03, rh74, rh10 and Mut C (WO 2016/181123; WO 2013/029030; WO 2017/096164). Optionally, the cap gene encodes a Cap protein selected from the group of AAV serotypes consisting of serotypes 2, 5, 8 or 9, and Mut C (SEQ ID NO: 3 from WO 2016/181123). Optionally, the cap gene encodes the Cap protein Mut C (SEQ ID NO: 3 from WO 2016/181123).

### Cap gene promoter

Optionally, the vector plasmid comprises a cap gene promoter. The cap gene promoter may be operably linked to a cap gene. Alternatively, the vector plasmid may not comprise a cap gene, but may comprise a cloning site operably linked (*i*.*e*. in close juxtaposition: 5'-[cap gene promoter]-[cloning site]-3') to the cap gene promoter. The cloning site may be a multiple cloning site (MCS, or polylinker). The user may wish to have the option to add a specific cap gene for a specific application. For example, if the vector plasmid is to be used to produce AAV for use in gene therapy, the user may wish the vector plasmid to lack a cap gene, but comprise a cloning site to allow a specific cap gene to be cloned in for a specific application. The vector plasmid could be used in connection with any transgene (in an expression cassette), and the user may find that for certain transgenes, encapsidation of such cassettes into capsids having properties such as liver tropism is advantageous, whereas for other transgenes capsids having different tropisms are advantageous. By designing a vector plasmid that comprises a cap gene promoter linked to a cloning site, the user can readily *'plug in'* an appropriate cap gene for a specific application (such as use of a specific transgene).

Optionally, the vector plasmid comprises an at least one cap gene promoter, which is a native cap gene promoter.

The native cap gene (i.e. the cap gene of a wild type AAV) is operably linked to a p40 promoter, a p5 promoter and a p19 promoter. Optionally, the at least one cap gene promoter comprises an AAV p40 promoter, an AAV p5 promoter, and/or an AAV p19 promoter. In one embodiment, the at least one cap gene promoter comprises an AAV p40 promoter, an AAV p5 promoter, and an AAV p19 promoter. Optionally, the at least one cap gene promoter comprises an AAV p40 promoter, a p5 promoter, and a p19 promoter. However, any suitable promoter that is able to drive cap gene expression can be used.

Whether or not a given cap gene promoter is able to drive cap gene expression may be determined using an AAV production assay as described above. Specifically, if the cap gene promoter can drive cap gene expression it will facilitate AAV production. In this case, the test two-plasmid system will comprise a vector plasmid that comprises the cap gene and a cap gene promoter whose ability to facilitate AAV production is to be tested, and otherwise the test two-plasmid system used will be identical to the reference two-plasmid system. In one embodiment, the cap gene promoter will be considered to facilitate AAV production if it supports rAAV production at a level at least 25%, at least 40%, at least 50%, at least 60%, at least 70%, at least 80%, at least 90%, or at least 95% of the level supported by a wild type p40 cap gene promoter, i.e. if the yield of rAAV vectors produced is at least 25%, at least 40%, at least 50%, at least 60%, at least 70%, at least 80%, at least 90%, or at least 95% of the yield of rAAV produced using the reference two-plasmid system. Preferably, the cap gene promoter will be considered to facilitate AAV production if it supports rAAV production at a level at least 70%, at least 80%, at least 90%, or at least 95% of the yield of rAAV produced using the reference two-plasmid system.

The p40 promoter is understood to drive expression of the cap gene. The native p40 promoter is contained within the native rep gene. The AAV2 p40 promoter has a sequence of nucleotides 1710-1827 of SEQ ID NO: 1. However, p40 promoters in other serotypes of AAV may comprise slightly different sequences. In some embodiments, the p40 promoter has a sequence at least 95%, at least 98%, or at least 99% identical to nucleotides 1710-1827 of SEQ ID NO: 1, or a corresponding sequence from another serotype of AAV. In some embodiments, the p40 promoter has a sequence at least 98% identical to nucleotides 1710-1827 of SEQ ID NO: 1.

The present inventors have found that an at least one cap gene promoter that comprises a p40 promoter, a p5 promoter and a p19 promoter is advantageous, as the p5 promoter and the p19 promoter play a role in regulating the p40 promoter, and the presence of the p40 promoter, the p5 promoter and the p19 promoter leads to timely regulated expression of the cap gene resulting in high yield/high quality rAAV. Accordingly, in some embodiments, the at least one cap gene promoter is comprised in a 'promoter region' comprising a p40 promoter, a p5 promoter and a p19 promoter. Optionally, the promoter region comprises a sequence at least 95%, at least 98%, at least 99%, or 100% identical to the full length or a fragment of at least 800, at least 900, at least 1000 or at least 1100 nucleotides in length of the following stretches of native AAV2 sequence (SEQ ID NO: 1) positioned in immediate juxtaposition from 5' to 3': 200-354; 600-1049; 1701-2202, or to corresponding juxtaposed stretches of nucleotides from a different serotype of AAV.

The native p5 promoter is upstream of the native rep gene. The AAV2 p5 promoter has a sequence of nucleotides 204-292 of SEQ ID NO: 1. However, p5 promoters in other serotypes of AAV may comprise slightly different sequences. In some embodiments, the p5 promoter has a sequence at least 95%, at least 98%, or at least 99% identical to nucleotides 204-292 of SEQ ID NO: 1, or a corresponding sequence from another serotype of AAV. In some embodiments, the p5 promoter has a sequence at least 98% identical to nucleotides 204-292 of SEQ ID NO: 1.

The p19 promoter is contained within the native rep gene. The AAV2 p19 promoter has a sequence of nucleotides 730-890 of SEQ ID NO: 1. However, p19 promoters in other serotypes of AAV may comprise slightly different sequences. In some embodiments, the p19 promoter has a sequence at least 95%, at least 98%, or at least 99% identical to nucleotides 730-890 of SEQ ID NO: 1, or a corresponding sequence from another serotype of AAV. In some embodiments, the p19 promoter has a sequence at least 98% identical to nucleotides 730-890 of SEQ ID NO: 1.

### An expression cassette flanked on at least one side by an ITR

The two-plasmid system of the invention may be used to produce AAV vector for use in gene therapy. A *"gene therapy"* involves administering AAV/viral particles of the invention that are capable of expressing a transgene (such as a Factor IX-encoding nucleotide sequence) in the host to which it is administered. In such cases, the vector plasmid will comprise an expression cassette.

Optionally, the vector plasmid comprises at least one ITR. Thus, optionally, the vector plasmid comprises at least one ITR, but, more typically, two ITRs (generally with one either end of the expression cassette, i.e. one at the 5' end and one at the 3' end). There may be intervening sequences between the expression cassette and one or more of the ITRs. The expression cassette may be incorporated into a viral particle located between two regular ITRs or located on either side of an ITR engineered with two D regions. Optionally, the vector plasmid comprises ITR sequences which are derived from AAV1, AAV2, AAV4 and/or AAV6. Preferably the ITR sequences are AAV2 ITR sequences.

Optionally, the vector plasmid comprises an expression cassette. As described herein, an expression cassette refers to a sequence of nucleic acids comprising a transgene and a promoter operably linked to the transgene. Optionally, the cassette further comprises additional transcription regulatory elements, such as enhancers, introns, untranslated regions, transcriptional terminators, etc.

The expression cassette should be considered to comprise the stretch of the vector plasmid between the ITRs that comprises any transgenes and transcription regulatory elements operably linked to the transgene. In embodiments where the vector plasmid comprises two ITRs, the expression cassette is considered to comprise the stretch of the vector plasmid between and including the ITRs. Optionally, the expression cassette is less than 5.0 kbp, less than 4.9 kbp, less than 4.8 kbp, less than 4.75 kbp, less than 4.7 kbp, or less than 4.5 kbp. Optionally, the expression cassette is less than 4.7kbp, less than 4.6kbp, less than 4.5kbp, less than 4.4 kbp, less than 4.3 kbp, less than 4.2 kbp, less than 4.1 kbp, less than 4.0 kbp, less than 3.9 kbp, less than 3.8 kbp, less than 3.7 kbp, less than 3.6 kbp, less than 3.5 kbp, less than 3.4 kbp, less than 3.3 kbp, less than 3.2 kbp, less than 3.1 kbp, less than 3.0 kbp, less than 2.9 kbp, less than 2.8 kbp, less than 2.7 kbp, less than 2.6 kbp, less than 2.5 kbp, less than 2.4 kbp, less than 2.3 kbp, less than 2.2 kbp, less than 2.1 kbp, less than 2.0 kbp, 1.9 kbp, less than 1.8 kbp, less than 1.7 kbp, less than 1.6 kbp, less than 1.5 kbp, less than 1.4 kbp, less than 1.3 kbp, less than 1.2 kbp, less than 1.1 kbp, less than 1.0 kbp, less than 0.9 kbp, less than 0.8 kbp, less than 0.7 kbp, less than 0.6 kbp, less than 0.5 kbp, less than 0.4 kbp, less than 0.3 kbp, less than 0.2 kbp, or less than 0.1 kbp. Optionally, the expression cassette is between less than 4.9kb and between less than 4.7kb. Optionally, the expression cassette is between 2.0 kbp and 5.5 kbp, between 2.0 kbp and 4.75 kbp, between 2.0 kbp and 4.7 kbp, between 2.1 kbp and 4.9 kbp, between 2.2 kbp and 4.8 kbp or between 2.3 kbp and 4.5 kbp.

Optionally, the expression cassette comprises a transcription regulatory element comprising the promoter element and/or enhancer element from HLP2, HLP1, LP1, HCR-hAAT, ApoE-hAAT, and/or LSP. These transcription regulatory elements are described in more detail in the following references: HLP2: WO16/075473; HLP1: McIntosh J. et al., Blood 2013 Apr 25, 121(17):3335-44; LP1: Nathwani et al., Blood. 2006 April 1, 107(7): 2653-2661; HCR-hAAT: Miao et al., Mol Ther. 2000;1: 522-532; ApoE-hAAT: Okuyama et al., Human Gene Therapy, 7, 637-645 (1996); and LSP: Wang et al., Proc Natl Acad Sci USA. 1999 March 30, 96(7): 3906-3910. Each of these transcription regulatory elements comprises a promoter, an enhancer, and optionally other nucleotides. If the polynucleotide is intended for expression in the liver, the promoter may be a liver-specific promoter. Optionally, the promoter is a human liver-specific promoter.

The transgene may be any suitable gene. If the vector plasmid is for use in gene therapy, the transgene may be any gene that comprises or encodes a protein or nucleotide sequence that can be used to treat a disease. For example, the transgene may encode an enzyme, a metabolic protein, a signalling protein, an antibody, an antibody fragment, an antibody-like protein, an antigen, or a non-translated RNA such as a miRNA, siRNA, snRNA, or antisense RNA.

Optionally, the vector plasmid comprises a cap gene and further comprises an expression cassette flanked on at least one side by an ITR.

### Dispensable translation initiation codons

Optionally, the vector plasmid does not comprise any dispensable translation initiation codons. Optionally, the vector plasmid may comprise at least two genes that must be capable of being transcribed and translated (the transgene (in an expression cassette) and the cap gene). Optionally, the transgene encodes a functional RNA which does not encode a translational protein or polypeptide. The transgene, if protein-encoding, and the cap gene will comprise start (ATG or GTG) codons to promote initiation of translation of the gene. However, the vector plasmid may comprise additional instances of ATG or GTG (either in-frame or out of frame with the reading frame of these genes), and it is possible that translation may initiate at one of these positions. Since there is no need for translation to be initiated at the site of these additional instances of ATG or GTG, the ATG or GTG codons may be considered to be *"dispensable translation initiation codons".* It is preferred that dispensable translation initiation codons are removed or rendered non-functional, in particular where they occur in the promoter region. The promoter region is a region of the vector plasmid that comprises one or more promoters operably linked to the cap gene. In some embodiments, the cap gene is operably linked to one or more of the p5, p19 and p40 promoters, and in such embodiments the promoter region comprises the p5, p19 and p40 promoters.

Optionally, the plasmid comprises a promoter region comprising one or more promoters, and the promoter region does not comprise ATG or GTG codons. Optionally, the promoter region comprises p5, p19 and/or p40 promoters, and wherein ATG or GTG codons at one or more positions corresponding to positions (a) 321-323 (Rep78/68 ATG start codon), (b) 766-768 (ATG codon), (c) 955-957 (ATG codon), (d) 993-995 (Rep52/40 ATG start codon) and (e) 1014-1016 (GTG codon) of SEQ ID NO: 1 are absent or mutated.

Optionally, in the promoter region:
(a) nucleotides corresponding to nucleotides 321-323 of SEQ ID NO: 1 are absent;
(b) nucleotides corresponding to nucleotides 766-768 of SEQ ID NO: 1 are not ATG and are optionally ATT;
(c) nucleotides corresponding to nucleotides 955-957 of SEQ ID NO: 1 are absent;
(d) nucleotides corresponding to nucleotides 993-995 of SEQ ID NO: 1 are absent; and/or
(e) nucleotides corresponding to nucleotides 1014-1016 of SEQ ID NO: 1 are absent.

Optionally, in the promoter region :
(a) nucleotides corresponding to nucleotides 321-323 of SEQ ID NO: 1 are absent;
(b) nucleotides corresponding to nucleotides 766-768 of SEQ ID NO: 1 are not ATG and are optionally ATT;
(c) nucleotides corresponding to nucleotides 955-957 of SEQ ID NO: 1 are absent;
(d) nucleotides corresponding to nucleotides 993-995 of SEQ ID NO: 1 are absent; and
(e) nucleotides corresponding to nucleotides 1014-1016 of SEQ ID NO: 1 are absent.

### Size of the vector plasmid

As discussed above in connection with the helper plasmid, it is advantageous that plasmids used in the production of rAAV are as small as possible. Smaller plasmids are more cost-effective to produce and are easier to transfect into cells.

The size of the vector plasmid will in part be defined by the size of the promoters, transcription regulatory elements, transgenes and cap genes that are included, and as discussed above, the nature of these features will be partly or fully defined by the application for which the rAAV is to be used.

However, the size of the vector plasmid can be reduced by ensuring that the size of the backbone that is used is small, and/or by ensuring that the vector plasmid does not comprise a spacer (a contiguous regions of non-coding nucleic acid sequences of greater than 200 nucleotides in length).

Optionally, the vector plasmid comprises a backbone of fewer than 4000 nucleotides, fewer than 3500 nucleotides, fewer than 3000 nucleotides, or fewer than 2500 nucleotides in length. Optionally, the vector plasmid does not comprise any spacers.

### Host cell

The present invention provides a host cell comprising the two-plasmid system, or the helper plasmid of the invention.

The host cell is preferably a host cell that can be used to produce rAAV. The host cell may therefore be a host cell suitable for the production of rAAV. In addition, the host cell may be for the production of rAAV.

In general a host cell that is suitable for the production of rAAV is a host cell that is derived from a eukaryotic cell line, preferably a vertebrate cell line, preferably a mammalian cell line, preferably a human cell line. Optionally, the host cell is a cell selected from the group consisting of a HEK293T cell, a HEK293 cell, a HEK293EBNA cell, a HEK293F cell, aHEK293S cell, a CAP cell, a CAP-T cell, an AGE1.CR cell, a PerC6 cell, a C139 cell, an EB66 cell, a BHK cell, a COS cell, a Vero cell, a HeLa cell, and an A549 cell. Preferably, the host cell is selected from the group consisting of a HEK293T cell, a HEK293 cell, a HEK293EBNA cell, a CAP cell, a CAP-T cell, an AGE1.CR cell, a PerC6 cell, a C139 cell, and an EB66 cell. Even more preferably, the host cell is selected from the group consisting of a HEK293T cell, a HEK293 cell, and a HEK293EBNA cell. For example, the host cell may be a HEK293T cell or a HEK293 cell. Optionally, the host cell is a cell that expresses a functional adenoviral E1A/B protein. For example, the host cell may comprise a chromosome comprising a gene encoding a functional adenoviral E1A/B protein. Functional adenoviral E1A/B proteins are discussed in the section entitled *"At least one helper virus gene".*

It is within the abilities of the skilled person to determine whether a host cell is suitable for the production of rAAV. The skilled person merely needs to determine whether the host cell supports AAV production using an AAV production assay as described above. In this case, the test two-plasmid system and the reference two-plasmid system that are used will be identical. However, the test two-plasmid system will be transfected into the host cell whose suitability for the production of recombinant AAV is to be tested, and the reference two-plasmid system will be transfected into HEK293cells. The host cell will be considered to be suitable for the production of recombinant AAV if it supports AAV production at a level at least 30%, at least 40%, at least 50%, at least 70%, at least 80%, at least 90%, or at least 95% of the level supported by HEK293T cells, i.e. if the yield of recombinant AAV produced is at least 30%, at least 40%, at least 50%, at least 70%, at least 80%, at least 90%, or at least 95% of the yield of recombinant AAV produced in HEK293T cells. It is particularly advantageous, in the context of the present invention that the host cell is used and/or maintained in suspension culture.

### Methods and uses

The present invention further provides a use of the helper plasmid or the two-plasmid system of the invention for producing a recombinant AAV preparation.

The present invention further provides a method for producing a recombinant AAV preparation comprising:
(a) obtaining the helper plasmid or the two-plasmid system of the invention;
(b) transfecting a host cell with the helper plasmid or the two-plasmid system of the invention; and
(c) culturing the host cell in suspension under conditions suitable for recombinant AAV production.

The present invention further provides a use of the helper plasmid or the two-plasmid system of the invention for:
(i) increasing, optimising and/or maximising the yield of recombinant AAV produced during recombinant AAV production;
(ii) increasing the yield of recombinant AAV produced during recombinant AAV production compared to an equivalent use of a two-plasmid system comprising a rep 78 negative helper plasmid;
(iii) controlling and/or maximising the ratio of full to total particles produced during recombinant AAV production;
(iv) reducing and/or minimising the level of nucleic acid impurities (normalised to vector genome level) produced during recombinant AAV production;
(v) reducing the level of nucleic acid impurities produced during recombinant AAV production (normalised to vector genome level) compared to an equivalent use of a two-plasmid system comprising a rep 78 negative helper plasmid;
(vi) increasing the potency of recombinant AAV produced during recombinant AAV production; and/or
(vii) increasing the potency of recombinant AAV produced during recombinant AAV production compared to an equivalent use of a two-plasmid system comprising a rep 78 negative helper plasmid.

The present invention further provides a method for:
(i) increasing, optimising and/or maximising the yield of recombinant AAV produced during recombinant AAV production;
(ii) increasing the yield of recombinant AAV produced during recombinant AAV production compared to an equivalent method using a two-plasmid system comprising a rep 78 negative helper plasmid;
(iii) controlling and/or maximising the ratio of full to total particles produced during recombinant AAV production;
(iv) reducing and/or minimising the level of nucleic acid impurities (normalised to vector genome level) produced during recombinant AAV production;
(v) reducing the level of nucleic acid impurities produced during recombinant AAV production (normalised to vector genome level) compared to an equivalent method using a two-plasmid system comprising a rep 78 negative helper plasmid;
(vi) increasing the potency of recombinant AAV produced during recombinant AAV production; and/or
(vii) increasing the potency of recombinant AAV produced during recombinant AAV production compared to an equivalent method using a two-plasmid system comprising a rep 78 negative helper plasmid,
comprising:
a) obtaining the helper plasmid or the two-plasmid system of the invention;
(b) transfecting a host cell with the helper plasmid or the two-plasmid system the invention; and
(c) culturing the host cell in suspension under conditions suitable for recombinant AAV production.

Optionally, the uses of the invention comprise transfecting a host cell with the two-plasmid system or helper plasmid of the invention and culturing the host cell in suspension under conditions suitable for recombinant AAV production.

### High (vector genome) yield

The present invention provides a use of the two-plasmid system or the helper plasmid of the invention or method for producing a recombinant AAV preparation at a high or desired yield.

The present invention also provides a use of the two-plasmid system or the helper plasmid of the invention or method for producing a recombinant AAV preparation at a higher yield than the yield obtained using an equivalent method or use using a two-plasmid system comprising a rep 78 negative helper plasmid.

The present invention also provides a use or method for increasing, optimising and/or maximising the yield of recombinant AAV produced during recombinant AAV production.

The term *"yield"* refers to the amount of AAV particles that are prepared in the methods or uses of the invention. The *"yield"* may be expressed as the number of vector genomes (vg) per ml of medium, as measured on bulk product prior to harvesting, purifying and/or concentrating the rAAV preparation. Optionally, the method or use comprises a step of transfecting a host cell with the helper plasmid or two-plasmid system of the invention and the *"yield"* is the yield measured approximately 72 or 120 hours post transfection. The number of vector genomes can be measured as discussed under the heading "

*Adjusting or selecting a ratio of helper plasmid to vector plasmid", i.e.* the yield of rAAV (such as rAAV particles) may be determined by using qPCR to quantify the number of nucleic acid sequences comprising a cassette comprising a promoter sequence (vg).

As used herein the term *"high yield"* generally refers to a yield that is at least 9x 10¹⁰ vg/ml or at least 1.1 fold greater than the yield achieved using an equivalent method or use using a rep 78 negative helper plasmid at a helper plasmid to vector plasmid ratio of 1:3. An *"equivalent method"* is a method that is identical, except that the helper plasmid is a rep 78 negative helper plasmid and the ratio of helper plasmid to vector plasmid is 1:3.

The present inventors have shown that modifying the ratio of helper plasmid to vector plasmid may be used to increase the yield (i.e. quantity of vgs or vg/ml produced) of a method that produces rAAV. Having the highest yield possible is clearly advantageous, as it reduces the amount of resources required to produce the rAAV. However, under some conditions increasing the yield to the highest possible level may result in a decrease in the ratio of full to total particles. Thus, if the user is producing the rAAV for an application where maximising the ratio of full to total particles is important, then he may opt to use a desired yield that is not the highest possible yield by using a helper plasmid to vector plasmid ratio that optimises in favour of the ratio of full to total (i.e. "%age full") particles.

As used herein the term *"maximising"* the yield refers to aiming for the highest possible yield within the limitations of the methods of the invention. As used herein the term *"optimising"* refers to increasing the yield, but aiming for a desired yield which may not be the maximum possible yield if, for example, the user is keen to ensure a high ratio of full to total particles (i.e. minimising the proportion of empty particles).

The present invention also provides a use or method for producing a recombinant AAV preparation at a higher yield than the yield obtained using an equivalent method or use using a two-plasmid system comprising a rep 78 negative helper plasmid.

Optionally, the higher yield is at least 1.03, at least 1.04, at least 1.05, at least 1.1 fold, at least 1.2 fold, at least 1.25 fold, at least 1.5 fold, at least 1.6 fold, at least 1.7 fold, at least 2 fold, between 1.1 fold and 5 fold, between 1.2 fold and 3 fold, or between 1.2 fold and 2.5 fold higher than the yield obtained using an equivalent method or use using a two-plasmid system comprising a rep 78 negative helper plasmid. Optionally, the higher yield is at least 1.7 fold or at least 2 fold higher than the yield obtained using an equivalent method or use using a two-plasmid system comprising a rep 78 negative helper plasmid. Optionally, the higher yield is between 1.2 fold and 3 fold or between 1.2 fold and 2.5 fold higher than the yield obtained using an equivalent method or use using a two-plasmid system comprising a rep 78 negative helper plasmid.

The present invention also provides a use or method for increasing the yield compared to an equivalent method using a two-plasmid system comprising a rep 78 negative helper plasmid. Optionally, the use or method is a use or method for increasing the yield compared to an equivalent use of a two-plasmid system comprising a rep 78 negative helper plasmid by at least 1.05, at least 1.1 fold, at least 1.2 fold, at least 1.25 fold, at least 1.5 fold, at least 1.6 fold, at least 1.7 fold, at least 2 fold, between 1.1 fold and 5 fold, between 1.2 fold and 3 fold, or between 1.2 fold and 2.5 fold. Optionally, the use or method is a use or method for increasing the yield compared to an equivalent use of a two-plasmid system comprising a rep 78 negative helper plasmid by at least 1.7 fold, or at least 2 fold. Optionally, the use or method is a use or method for increasing the yield compared to an equivalent use of a two-plasmid system comprising a rep 78 negative helper plasmid by between 1.2 fold and 3 fold, or between 1.2 fold and 2.5 fold.

A rep 78 negative helper plasmid is a helper plasmid that does not comprise a gene encoding Rep 78, but does comprise at least one gene encoding Rep 40, Rep 52 and Rep 68. Optionally, the rep 78 negative helper plasmid is identical to the helper plasmid of the invention, except that the rep 78 cassette is not present. Optionally, the rep 78 negative helper plasmid comprises an at least one rep gene that consists of nucleotides corresponding to the following nucleotides of SEQ ID NO: 1 position in immediate juxtaposition from 5' to 3': 200-1906; 2228-2309; and 658-2300, or to corresponding juxtaposed stretches of nucleotides from a different serotype of AAV. The rep 78 negative helper plasmid may be used at a ratio of helper plasmid to vector plasmid of 1:3, as methods or uses using a rep 78 negative helper plasmid at a ratio of helper plasmid to vector plasmid of 1:3 have improved properties compared to methods or uses using a rep 78 negative helper plasmid at other ratios of helper plasmid to vector plasmid.

The method may further comprise a step of harvesting the rAAV vector to provide an rAAV preparation, optionally wherein the recombinant AAV preparation comprises a high or desired yield of recombinant AAV or comprises a yield that is higher than the yield obtained using an equivalent method or use using a two-plasmid system comprising a rep 78 negative helper plasmid. As set out above, the *"yield"* may be expressed as the number of vector genomes (vg) per ml of medium, as measured on bulk product prior to harvesting, purifying and/or concentrating the rAAV preparation. Thus, the *"recombinant AAV preparation comprises a high yield or desired yield of recombinant AAV"* if the bulk AAV product produced by the method or use is at a high yield or desired yield of recombinant AAV.

The term *"capsid yield"* refers to the amount of AAV capsids that are prepared in the methods or uses of the invention. The *"capsid yield"* may be expressed as the number of capsids per ml of medium (as measured on bulk product prior to harvesting, purifying and/or concentrating the rAAV preparation). Optionally, the method or use comprises a step of transfecting a host cell with the helper plasmid or two-plasmid system of the invention and the *"capsid yield"* is the capsid yield measured approximately 72 post transfection. The number of capsids can be measured as discussed under the heading *"adjusting or selecting a ratio of helper plasmid to vector plasmid", i.e.* the capsid yield may be determined by using a capsid-specific ELISA.

The term *"high capsid yield"* generally refers to a capsid yield that is at least 1.3 x 10¹² cap/ml or 1.1 or 1.2 fold higher than the capsid yield achieved using an equivalent method or use using a rep 78 negative helper plasmid at a helper plasmid to vector plasmid ratio of 1:3. An *"equivalent method"* is a method that is identical, except that the helper plasmid is a rep 78 negative helper plasmid and the ratio of helper plasmid to vector plasmid is 1:3.

The present invention also provides a use or method for producing a recombinant AAV preparation at a higher capsid yield than the capsid yield obtained using an equivalent method or use using a two-plasmid system comprising a rep 78 negative helper plasmid.

Optionally, the higher capsid yield is at least 1.05, at least 1.1 fold, at least 1.2 fold, at least 1.4 fold, at least 1.5 fold, at least 1.6 fold, at least 1.7 fold, between 1.2 fold and 5 fold, between 1.4 fold and 3 fold, or between 1.4 fold and 2 fold higher than the capsid yield obtained using an equivalent method or use using a two-plasmid system comprising a rep 78 negative helper plasmid. Optionally, the higher capsid yield is at least 1.6 fold, or at least 1.7 fold higher than the capsid yield obtained using an equivalent method or use using a two-plasmid system comprising a rep 78 negative helper plasmid. Optionally, the higher capsid yield is between 1.4 fold and 3 fold, or between 1.4 fold and 2 fold higher than the capsid yield obtained using an equivalent method or use using a two-plasmid system comprising a rep 78 negative helper plasmid.

The present invention also provides a use or method for increasing the capsid yield compared to an equivalent method using a two-plasmid system comprising a rep 78 negative helper plasmid. Optionally, the use or method is a use or method for increasing the capsid yield compared to an equivalent use of a two-plasmid system comprising a rep 78 negative helper plasmid by at least 1.1 fold, at least 1.2 fold, at least 1.25 fold, at least 1.5 fold, at least 1.6 fold, at least 1.7 fold, at least 2 fold, between 1.1 fold and 5 fold, between 1.2 fold and 3 fold, or between 1.2 fold and 2.5 fold. Optionally, the use or method is a use or method for increasing the capsid yield compared to an equivalent use of a two-plasmid system comprising a rep 78 negative helper plasmid by at least 1.6 fold, or at least 1.7 fold. Optionally, the use or method is a use or method for increasing the capsid yield compared to an equivalent use of a two-plasmid system comprising a rep 78 negative helper plasmid by between 1.4 fold and 3 fold, or between 1.4 fold and 2 fold.

A rep 78 negative helper plasmid is a helper plasmid that does not comprise a gene encoding Rep 78, but does comprise at least one gene encoding Rep 40, Rep 52 and Rep 68. Optionally, the rep 78 negative helper plasmid is identical to the helper plasmid of the invention, except that the rep 78 cassette is not present. Optionally, the rep 78 negative helper plasmid comprises an at least one rep gene that consists of nucleotides corresponding to the following nucleotides of SEQ ID NO: 1 position in immediate juxtaposition from 5' to 3': 200-1906; 2228-2309; and 658-2300, or to corresponding juxtaposed stretches of nucleotides from a different serotype of AAV. Optionally, the *"equivalent"* method or use is identical to the method or use being compared, except that a rep 78 negative helper plasmid is used. The rep 78 negative helper plasmid may be used at a ratio of helper plasmid to vector plasmid of 1:3, as methods or uses using a rep 78 negative helper plasmid at a ratio of helper plasmid to vector plasmid of 1:3 have improved properties compared to methods or uses using a rep 78 negative helper plasmid at other ratios of helper plasmid to vector plasmid.

The method may further comprise a step of harvesting the rAAV vector to provide an rAAV preparation, optionally wherein the recombinant AAV preparation comprises a high and/or desired capsid yield or comprises a capsid yield that is higher than the capsid yield obtained using an equivalent method or use using a two-plasmid system comprising a rep 78 negative helper plasmid. As set out above, the *"capsid yield"* may be expressed as the number of capsids (vg) per ml of medium (as measured on bulk product prior to harvesting, purifying and/or concentrating the rAAV preparation). Thus, the *"recombinant AAV preparation comprises a high capsid yield or desired capsid yield of recombinant AAV"* if the bulk AAV product produced by the method is at a high capsid yield or desired capsid yield.

### Lower level of AAV comprising impurities

The AAV packaging machinery is not precise and sometimes it packages genetic material that was not intended for packaging (which packaged genetic material constitutes a nucleic acid impurity). It is possible that any genetic material available during rAAV production may become a nucleic acid impurity, such as all of the genetic material of the helper plasmid, the vector plasmid, and the host cells in which the rAAV is produced. The helper plasmids, two-plasmid systems, methods and uses of the invention may be used to provide recombinant AAV for use in gene therapy. Suitable recombinant AAV for use in gene therapy will comprises a vector genome that comprises a therapeutic transgene. It is advantageous to reduce the amount of these impurities that are packaged into recombinant AAV (particularly recombinant AAV that is to be used for gene therapy), as providing these impurities to a gene therapy patient may have inadvertent and non-therapeutic effects.

The present invention provides a use of the two-plasmid system or the helper plasmid of the invention or method for producing a recombinant AAV preparation with a low level of nucleic acid impurities (normalised to vector genome level).

The present invention also provides a use of the two-plasmid system or the helper plasmid of the invention or method for producing a recombinant AAV preparation with a lower level of nucleic acid impurities (normalised to vector genome level) than the level of nucleic acid impurities obtained using an equivalent method or use using a two-plasmid system comprising a rep 78 negative helper plasmid.

The present invention also provides a use or method for reducing or minimising the level of nucleic acid impurities (normalised to vector genome level).

The term *"nucleic acid impurities"* refers to genetic material that has been packaged into recombinant AAV and which was not intended to be packaged. For example, if the helper plasmid comprises two ITRs, the genetic material which was intended to be packaged into the recombinant AAV is any genetic material between the two ITRs of the vector plasmid, i.e. the *"nucleic acid impurities"* is any genetic material that is not the genetic material between the two ITRs. The level of nucleic acid impurities may be as measured on bulk product prior to harvesting, purifying and/or concentrating the rAAV preparation, or may be measured after the rAAV have been harvested, purified and/or concentrated.

The level of nucleic acid impurities may be measured using a similar method to the qPCR method for quantifying the number of vector genomes but using qPCR primers specific for the nucleic acid impurities of interest. This qPCR method will provide the copy number of the nucleic acid impurities of interest per ml. The level of nucleic acid impurities may be expressed as percentage of the copy number of vector genomes per ml (as determined using the qPCR method for quantifying the number of vector genomes), i.e. the percentage/level of recombinant AAV comprising nucleic acid impurities may be expressed as the copy number of nucleic acid impurities of interest per ml / the copy number of vector genomes (vg) per ml x 100. When the level of nucleic acid impurities is expressed as a percentage of the copy number of vector genomes, it will be considered to be normalised to vector genome level. Alternatively, the level of nucleic acid impurities may be measured digital droplet polymerase chain reaction (ddPCR) using qPCR primers specific for the nucleic acid impurities of interest. "Droplet digital PCR" (ddPCR) refers to a digital PCR assay that measures absolute quantities by counting nucleic acid molecules encapsulated in discrete, volumetrically defined, water-in-oil droplet partitions that support PCR amplification (i.e., a plurality of such compartments). Typically, a "droplet" refers to water-in-oil droplet (i.e., an oil droplet that may be generated by emulsifying a sample with droplet generator oil); an individual partition of the droplet digital PCR assay. A droplet supports PCR amplification of template molecule(s) using homogenous assay chemistries and workflows similar to those widely used for real-time PCR applications (Hinson et al (2011) Anal. Chem. 83:8604-8610; Pinheiro et al (2012) Anal. Chem. 84:1003-1011). A single ddPCR reaction may typically be comprised of at least 5000, at least 6000, at least 7000, at least 8000, at least 9000, at least 10,000, at least 12,000, at least 14,000, at least 16,000, at least 18,000 or at least 20,000 compartments.

Droplet digital PCR may be performed using any platform that performs a digital PCR assay that measures absolute quantities by counting nucleic acid molecules encapsulated in discrete, volumetrically defined, water-in-oil droplet partitions that support PCR amplification. The strategy for droplet digital PCR may be summarized as follows: a sample is diluted and partitioned into thousands to millions of separate reaction chambers (water-in-oil droplets) so that each contains one or no copies of the nucleic acid molecule of interest. The number of positive droplets detected, which contain the target amplicon (i.e., nucleic acid molecule of interest), versus the number of negative droplets, which do not contain the target amplicon (i.e., nucleic acid molecule of interest), may be used to determine the number of copies of the nucleic acid molecule of interest that were in the original sample. Examples of droplet digital PCR systems include the QX100^{™} Droplet Digital PCR System by Bio-Rad, which partitions samples containing nucleic acid template into 20,000 nanolitre-sized droplets.

Droplet digital PCR may thus be used to detect a single target in a sample, for example using a single primer pair. However, ddPCR may also be used to detect two different targets in a sample, for example using two primer pairs, each primer pair hybridising to a different target, i.e., duplexing of targets. Duplexing may be extended to look at more targets in a sample, such as at least 3, at least 4, at least 5, at least 6, at least 7, at least 8, at least 9, at least 10, at least 11 or at least 12 different targets in a sample, i.e., multiplexing of targets. Duplexing and multiplexing with ddPCR allows for improved sensitivity and precision, increased low level detection, and also the inference of the size of a nucleic acid. ddPCR may be quantitative.

As used herein the term *"low level of nucleic acid impurities"* generally refers to a level of nucleic acid impurities less than 8% (i.e. the copy number of nucleic acid impurities per ml/the copy number of vg per ml x 100 = less than 8), or a level lower than the level obtained using an equivalent method or use using a rep 78 negative helper plasmid at a helper plasmid to vector plasmid ratio of 1:3. An *"equivalent method"* is a method that is identical, except that helper plasmid is a rep 78 negative helper plasmid and the ratio of helper plasmid to vector plasmid is 1:3.

Optionally, the low or lower level of nucleic acid impurities is a low or lower level of vector plasmid or helper plasmid impurities, i.e. the nucleic acid impurities of interest and which are measured using the method described above are vector plasmid impurities. *"Vector plasmid impurities"* are impurities which correspond to nucleotides from the vector plasmid. For example, vector plasmid impurities may comprise cap gene and/or vector plasmid backbone impurities. *"Cap gene impurities"* are impurities which correspond to a portion of the cap gene; a *"cap gene impurity"* may comprise the entire cap gene, or just a portion of the cap gene. *"Helper plasmid impurities"* are impurities which correspond to nucleotides from the helper plasmid. For example, helper plasmid impurities may correspond to nucleotides from the at least one helper virus gene (*e.g*. all or a portion of an E2A gene, an E4 gene, and/or a VA nucleic acid), the at least one rep gene (e.g. all or a portion of the rep 78 cassette) or the helper plasmid backbone.

Optionally, the low or lower level of cap gene impurities is a level of cap gene impurities that is less than 0.8 fold, less than 0.7 fold, less than 0.65 fold, less than 0.55 fold, less than 0.5 fold, less than 0.25 fold, less than 0.2 fold, between 0.1 fold and 0.8 fold, between 0.15 fold and 0.7 fold, between 0.15 fold and 0.65 fold, between 0.15 fold and 0.55 fold, between 0.15 fold and 0.5 fold, or between 0.15 fold and 0.2 fold of the level of AAV comprising cap gene impurities produced using an equivalent method or use using a two-plasmid system comprising a rep 78 negative helper gene. Optionally, the low or lower level of cap gene impurities is a level of cap gene impurities that is less than 0.25 fold, or less than 0.2 fold of the level of cap gene impurities produced using an equivalent method or use using a two-plasmid system comprising a rep 78 negative helper gene. Optionally, the low or lower level of cap gene impurities is a level of cap gene impurities that is between 0.15 fold and 0.5 fold, or between 0.15 fold and 0.2 fold of the level of cap gene impurities produced using an equivalent method or use using a two-plasmid system comprising a rep 78 negative helper gene.

A rep 78 negative helper plasmid is a helper plasmid that does not comprise a gene encoding Rep 78, but does comprise at least one gene encoding Rep 40, Rep 52 and Rep 68. Optionally, the rep 78 negative helper plasmid is identical to the helper plasmid of the invention, except that the rep 78 cassette is not present. Optionally, the rep 78 negative helper plasmid comprises an at least one rep gene that consists of nucleotides corresponding to the following nucleotides of SEQ ID NO: 1 position in immediate juxtaposition from 5' to 3': 200-1906; 2228-2309; and 658-2300, or to corresponding juxtaposed stretches of nucleotides from a different serotype of AAV. The rep 78 negative helper plasmid may be used at a ratio of helper plasmid to vector plasmid of 1:3, as methods or uses using a rep 78 negative helper plasmid at a ratio of helper plasmid to vector plasmid of 1:3 have improved properties compared to methods or uses using a rep 78 negative helper plasmid at other ratios of helper plasmid to vector plasmid.

Optionally, the level of vector plasmid impurities or cap gene impurities is determined by qPCR using primers that are complementary to a portion of the cap gene, i.e. by determining the copy number of a cap gene sequence per mL using a similar method to the qPCR method for quantifying the number of vector genomes but using qPCR primers specific for the cap gene. As discussed above, the level of vector plasmid impurities or cap gene impurities may be expressed as a percentage of the copy number of vector genomes per ml (normalised to vector genome level).

Optionally, the low or lower level of plasmid backbone impurities is a level of plasmid backbone impurities that is less than 0.6 fold, less than 0.5 fold, less than 0.45 fold, less than 0.4 fold, less than 0.35 fold, less than 0.2 fold, less than 0.15 fold, between 0 fold and 0.6 fold, between 0.05 fold and 0.5 fold, between 0.1 fold and 0.45 fold, between 0.1 fold and 0.4 fold, between 0.1 fold and 0.35 fold, between 0.1 fold and 0.2 fold, or between 0.1 fold and 0.15 fold of the level of plasmid backbone impurities obtained using an equivalent method or use using a two-plasmid system comprising a rep 78 negative helper gene. Optionally, the low or lower level of plasmid backbone impurities is a level of plasmid backbone impurities that is less than 0.2 fold or less than 0.15 fold of the level of plasmid backbone impurities obtained using an equivalent method or use using a two-plasmid system comprising a rep 78 negative helper gene. Optionally, the low or lower level of plasmid backbone impurities is a level of plasmid backbone impurities that is between 0.1 fold and 0.2 fold, or between 0.1 fold and 0.15 fold of the level of plasmid backbone impurities obtained using an equivalent method or use using a two-plasmid system comprising a rep 78 negative helper gene.

Optionally, the level of vector plasmid impurities or vector plasmid backbone impurities is determined by qPCR using primers that are complementary to a portion of the vector plasmid backbone (such as an antibiotic resistance gene such as kanamycin R), *i.e.* by determining the copy number of a vector plasmid backbone impurity per mL using a similar method to the qPCR method for quantifying the number of vector genomes but using qPCR primers specific for the vector plasmid backbone, such as an antibiotic resistance gene. As discussed above, the level of vector plasmid impurities or vector plasmid backbone impurities may be expressed as a percentage of the copy number of vector genomes per ml.

Optionally, the low or lower level of nucleic acid impurities comprises a low or lower level of plasmid backbone impurities. Optionally, the low or lower level of nucleic acid impurities comprises a low or lower level of helper plasmid impurities. Optionally, the low or lower level of plasmid backbone impurities and/or helper plasmid impurities comprises a level of plasmid backbone impurities and/or helper plasmid impurities that is less than 0.6 fold, less than 0.5 fold, less than 0.45 fold, less than 0.4 fold, less than 0.35 fold, less than 0.2 fold, less than 0.15 fold, between 0 fold and 0.6 fold, between 0.05 fold and 0.5 fold, between 0.1 fold and 0.45 fold, between 0.1 fold and 0.4 fold, between 0.1 fold and 0.35 fold, between 0.1 fold and 0.2 fold, or between 0.1 fold and 0.15 fold of the level of plasmid backbone impurities and/or helper plasmid impurities obtained using an equivalent method or use using a two-plasmid system comprising a rep 78 negative helper gene. Optionally, the level of plasmid backbone impurities or helper plasmid impurities is determined by qPCR using primers that are complementary to a portion of the helper plasmid, optionally an antibiotic resistance gene such as kanamycin R.

Optionally, the low or lower level of nucleic acid impurities comprises a low or lower level of host cell derived impurities. *"Host cell derived impurities"* are impurities that correspond to nucleotides from the host cell genome. For example, if the host cell is a HEK293T or HEK293 cells, the host cell derived impurities may be derived from the genome of a HEK293 or HEK293T cell. Optionally, the low or lower level of host cell derived impurities comprises a low or lower level of 18S rRNA gene impurities. Optionally, the level of host cell-derived or 18S rRNA gene impurities is determined by ddPCR using primers and a probe that are complementary to a portion of the host cell genome, optionally an 18S rRNA gene. Host cell derived impurities can be measured, for example, using ddPCR or qPCR. For instance, host cell derived impurities can be measured in accordance with the section titled *"Quantification of host cell-derived impurities"* of the Examples.

### Increasing the potency of recombinant AAV produced during recombinant AAV production

The present invention provides a use of the two-plasmid system or the helper plasmid of the invention or method for producing a recombinant AAV preparation having high potency.

The present invention also provides a use of the two-plasmid system or the helper plasmid of the invention or method for producing a recombinant AAV preparation comprising recombinant AAV having higher potency compared to the potency of recombinant AAV obtained using an equivalent method or use using a two-plasmid system comprising a rep negative helper plasmid.

The present invention also provides a use or method for increasing the potency of recombinant AAV produced during recombinant AAV production.

The term *"potency"* refers to the ability of the recombinant AAV to transduce cells and deliver a transgene. The *"potency"* may be measured by transducing cells with recombinant AAV produced using the methods or uses of the invention which comprise a transgene and determining the activity of a polypeptide encoded by the transgene. For example, a user may determine the potency of recombinant AAV produced by a particular method or use by carrying out the particular method or use using a two-plasmid system of the invention comprising a transgene whose activity can be measured, for example a chromogenic/fluorogenic protein such as green fluorescent protein (GFP) or a blood clotting factor such as Factor VIII. The user may then measure the potency of the recombinant AAV by transducing host cells, such as Huh7 cells, with the recombinant AAV (for example at a pre-selected multiplicity of infection) and culturing the host cell under conditions suitable for expression of the transgene to occur. The user may then isolate the protein encoded by the transgene, and test its activity. In a preferred assay to measure *"potency",* the two-plasmid system comprises a Factor VIII (FVIII) transgene. The two-plasmid system is used to prepare recombinant AAV, the resulting recombinant AAV is used to transduce Huh7 cells after a 72 hour production phase, the supernatant is harvested and the FVIII activity of the supernatant is tested using a chromogenic assay. The potency assay may be an absolute potency assay.

For example, a suitable chromogenic assay is as follows. The Factor VIII polypeptide is mixed with human Factor X polypeptide and Factor IXa polypeptide, thrombin, phospholipids and calcium. The thrombin activates the Factor VIII polypeptide to form Factor Villa polypeptide. The thrombin-activated Factor VIII polypeptide forms an enzymatic complex with Factor IXa polypeptide, phospholipids and calcium, which enzymatic complex can catalyse the conversion of Factor X polypeptide to Factor Xa polypeptide. The activity of the Factor Xa polypeptide can catalyse cleavage of a chromogenic substrate (e.g. SXa-11) to produce pNA. The level of pNA generated can be measured by determining colour development at 405 nm (e.g. measured by absorbance). Factor X polypeptide, and therefore Factor Xa polypeptide, is provided in excess. Therefore the limiting factor is Factor Villa polypeptide. Thus, the level of pNA generated is proportional to the amount of the Factor Xa polypeptide generated by Factor FVllla polypeptide in the sample, which is proportional to the activity of Factor FVllla polypeptide in the sample. The activity of Factor FVllla polypeptide in the sample is a measure of the cofactor activity of the Factor FVIII polypeptide in the sample.

Optionally, *"potency"* of recombinant AAV is measured by transducing host cells with the recombinant AAV, culturing the host cells and measuring the activity of a polypeptide produced by the host cells, wherein the recombinant AAV comprise a transgene and the polypeptide is encoded by the transgene. Optionally, potency of recombinant AAV is measured by transducing Huh7 cells with the recombinant AAV, culturing the Huh7 cells and measuring the activity of a polypeptide produced by the host cells, wherein the recombinant AAV comprise a transgene and the polypeptide is encoded by the transgene. Optionally, the polypeptide whose activity is measured is Factor VIII, and the activity of the Factor VIII is measured using a chromogenic assay.

As used herein, the term *"high potency"* generally refers to a potency that is at least 1.1 fold higher than the potency achieved using an equivalent method or use using a rep 78 negative helper plasmid at a helper plasmid to vector plasmid ratio of 1:3. An *"equivalent method"* is a method that is identical, except that the helper plasmid is a rep 78 negative helper plasmid and the ratio of helper plasmid to vector plasmid is 1:3.

The present invention also provides a use or method for producing a recombinant AAV preparation having higher potency than the potency obtained using an equivalent method or use using a two-plasmid system comprising a rep 78 negative helper plasmid.

Optionally, the higher potency is at least 1.03, at least 1.04, at least 1.05, at least 1.1 fold, at least 1.15 fold, between 1.1 fold and 5 fold, or between 1.15 fold and 3 fold higher than the potency of recombinant AAV obtained using an equivalent method or use using a two-plasmid system comprising a rep 78 negative helper plasmid. Optionally, the higher potency is at least 1.15 fold higher than the potency of recombinant AAV obtained using an equivalent method or use using a two-plasmid system comprising a rep negative helper plasmid. Optionally, the higher potency is between 1.15 fold and 3 fold higher than the potency of recombinant AAV obtained using an equivalent method or use using a two-plasmid system comprising a rep negative helper plasmid.

The present invention also provides a use or method for increasing the potency of recombinant AAV produced during recombinant AAV production compared to an equivalent method using a two-plasmid system comprising a rep 78 negative helper plasmid. Optionally, the use or method is a use or method for increasing the potency of recombinant AAV produced during recombinant AAV production compared to an equivalent use of a two-plasmid system comprising a rep 78 negative helper plasmid by at least 1.05, at least 1.1 fold, at least 1.15 fold, between 1.1 fold and 5 fold, or between 1.15 fold and 3 fold. Optionally, the use or method is a use or method for increasing the potency of recombinant AAV produced during recombinant AAV production compared to an equivalent use of a two-plasmid system comprising a rep 78 negative helper plasmid by at least 1.1 fold. Optionally, the use or method is a use or method for increasing the potency of recombinant AAV produced during recombinant AAV production compared to an equivalent use of a two-plasmid system comprising a rep 78 negative helper plasmid by between 1.1 fold and 3 fold.

A rep 78 negative helper plasmid is a helper plasmid that does not comprise a gene encoding Rep 78, but does comprise at least one gene encoding Rep 40, Rep 52 and Rep 68. Optionally, the rep 78 negative helper plasmid is identical to the helper plasmid of the invention, except that the rep 78 cassette is not present. Optionally, the rep 78 negative helper plasmid comprises an at least one rep gene that consists of nucleotides corresponding to the following nucleotides of SEQ ID NO: 1 position in immediate juxtaposition from 5' to 3': 200-1906; 2228-2309; and 658-2300, or to corresponding juxtaposed stretches of nucleotides from a different serotype of AAV. The rep 78 negative helper plasmid may be used at a ratio of helper plasmid to vector plasmid of 1:3, as methods or uses using a rep 78 negative helper plasmid at a ratio of helper plasmid to vector plasmid of 1:3 have improved properties compared to methods or uses using a rep 78 negative helper plasmid at other ratios of helper plasmid to vector plasmid.

The method may further comprise a step of harvesting the rAAV vector to provide an rAAV preparation, optionally wherein the recombinant AAV of the recombinant AAV preparation have a high potency or a potency that is higher than the potency of recombinant AAV produced using an equivalent method or use using a two-plasmid system comprising a rep 78 negative helper plasmid.

### Adjusting or selecting a ratio of helper plasmid to vector plasmid

The ratio of helper plasmid to vector plasmid may be adjusted to:
(i) increase, optimise and/or maximise the yield of recombinant AAV produced during recombinant AAV production;
(ii) control and/or maximise the ratio of full to total particles produced during recombinant AAV production;
(iii) obtain a high or desired yield;
(iv) obtain a desired ratio of full to total particles; and/or
(v) achieve a balanced yield versus full to total particle ratio.

The use or method may comprise a step of selecting a ratio of helper plasmid to vector plasmid. Optionally, the step of selecting a ratio of helper plasmid to vector plasmid is carried out in order to:
(i) increase, optimise and/or maximise the yield of recombinant AAV produced during recombinant AAV production;
(ii) control and/or maximise the ratio of full to total particles produced during recombinant AAV production;
(iii) obtain a high or desired yield;
(iv) obtain a desired ratio of full to total particles; and/or
(v) achieve a balanced yield versus full to total particle ratio.

Optionally, the use or method is a use or method for producing a recombinant AAV preparation having a desired ratio of full to total particles.

Optionally, the method or use comprises a step of harvesting the recombinant AAV to provide a recombinant AAV preparation comprising a desired ratio of full to total particles.

Specifically, the inventors have determined that increasing the level of the vector plasmid relative to the helper plasmid decreases the ratio of full to total particles.

*"Full"* particles are rAAV particles comprising both a capsid and the intended vector genome, or at least a partial such genome as determined using the qPCR method described below. *"Empty"* particles (i.e. particles which are not full) comprise capsids but do not comprise a genome, or comprise only a partial genome (thereby not forming a complete rAAV particle) which is not detected using the qPCR method. However, the rAAV preparations may comprise both full particles and empty particles. Generally, a low or minimised proportion of empty particles is desired. For example, if the rAAV are to be used in gene therapy, any empty particles will not comprise the entire expression cassette of interest and so will not be effective in therapy. On the other hand, there are circumstances where the presence of empty particles could be desirable. In some instances, and in some patient groups, it may be the case that the empty particles behave as *"decoys"* to reduce the immune response in a patient to the administered rAAV particles (WO2013/078400). Furthermore, as will be discussed in more detail below, whilst decreasing the level of the vector plasmid relative to the helper plasmid decreases the ratio of full to total particles, it also effects the yield of rAAV produced (and thereby in some cases the total number of full particles that are produced). Thus, even if the user of the method believes that a high full to total particle ratio is desirable, the user may use a higher proportion of helper plasmid if this leads to a greater yield. Alternatively, if the user, for example, implements a process change during development of a given product, e.g. switches from bioreactor A to bioreactor B resulting in different full to total particle ratios when keeping the plasmid ratio constant, the plasmid ratio can be altered to compensate for or offset the effect of the process change to ensure consistent full to total particle ratio throughout development, as this ratio is a critical quality parameter of an rAAV batch that needs to be controlled and kept comparable from batch to batch.

Accordingly, an advantage of the methods and uses of the present invention is that they allow the user the flexibility to determine a desirable ratio of full to total particles and modify the ratio of helper plasmid to vector plasmid in order to achieve that desired ratio of full to total particles.

The ratio of full to total particles may be expressed herein as the percentage of the total number of particles (capsids) that notionally comprise a vector genome or at least a partial such genome (assuming one (partial) genome per capsid) as determined using the following qPCR assay. qPCR is carried out using a pair of primers that are able to amplify at least a region of the promoter of the expression cassette. Optionally, at least one of the primers is specific for (reverse and complementary to or identical to depending on whether the primer is a forward primer or a reverse primer) a region of at least 12, at least 14, at least 16, or at least 18 nucleotides of the promoter of the expression cassette. Optionally, one primer is specific for the start of the promoter (the first at least 12 nucleotides of the promoter) and the other primer is specific for a region of the expression cassette that is 150 base pairs from the binding site of the first primer.

The ratio of full to total particles (as measured as a percentage of the total number of particles that are full particles) may be determined using qPCR to determine the number of vector genomes (as discussed in the previous paragraph), and using a capsid-specific ELISA to measure the total number of particles. For example, the capsid-specific ELISA may comprise exposing the rAAV preparation to an antibody that binds to the capsid protein. If, for example, the vector plasmid comprises a cap gene that encodes a capsid from an AAV2 serotype, the antibody may be an antibody that binds to the AAV2 capsid. For example, the user may coat a plate with an antibody that is specific for the capsid. The user may then pass the rAAV preparation over the surface of the plate. The particles will bind to the antibody and be immobilised on the plate. The plate may then be washed to remove contaminants. The amount of particle present can then be detected by addition of a detection antibody that can bind to the capsid and is conjugated to a detection agent such as streptavidin peroxidase. The amount of particle present will be proportional to the colour change obtained when the streptavidin peroxidase is exposed to the chromogenic substrate TMB (tetramethylbenzidine).

Optionally, the desired ratio of full to total particles (expressed as a percentage of the total number of particles that notionally comprise a vector genome) is at least 2.5%, at least 5%, at least 7.5%, at least 8%, between 2.5% and 10%, between 5% and 9%, between 7.5% and 9%, or between 8% and 9% (as measured on bulk product prior to harvesting, purifying and/or concentrating the rAAV preparation). In many cases, increasing the full to total particle ratio is advantageous, and the present inventors have determined that aiming for a full to total particle ratio that is at least 2.5%, at least 5%, at least 7.5%, at least 8%, between 2.5% and 10%, between 5% and 9%, between 7.5% and 9%, or between 8% and 9% achieves a good balance between maintaining a high full to total particle ratio, whilst also achieving a good yield. Optionally, the desired ratio of full to total particles is a ratio of full to total particles that is at least 20% or at least 30% of the ratio of full to total particles achieved using an equivalent method with a ratio of helper plasmid to vector plasmid of 1:3. Optionally, the desired ratio of full to total particles is a ratio of full to total particles that is at least 40% or at least 45% of the ratio of full to total particles achieved using an equivalent method with a ratio of helper plasmid to vector plasmid of 1:3.

As discussed above, the ratio of full to total particles and the yield may be affected by altering the ratio of helper plasmid to vector plasmid used in the methods. Thus, the user may adjust the ratio of helper plasmid to vector plasmid to a suitable ratio for obtaining the desired full to total particle ratio. Optionally, the methods or uses may comprise a step of selecting a ratio of helper plasmid to vector plasmid. Altering the ratio of helper plasmid to vector plasmid, or selecting a ratio of helper plasmid to vector plasmid, may be achieved by simply mixing the helper plasmid and the vector plasmid in different relative proportions.

The user may need to carry out a test method to determine the ratio of helper plasmid to vector plasmid that is required to obtain the desired ratio of full to total particles, but this is not burdensome. For example, the skilled person may perform a small scale experiment to determine the required ratio of helper plasmid to vector plasmid, and then this ratio can be used in a larger scale production process. Optionally, the step of selecting a ratio of helper plasmid to vector plasmid may comprise performing small scale test runs of the method of the invention using different ratios of helper plasmid to vector plasmid.

Optionally, the ratio of helper plasmid to vector plasmid is selected or adjusted to a ratio that achieves a balanced yield versus full to total particle ratio. Optionally, the ratio of helper plasmid to vector plasmid is selected or adjusted to a ratio that achieves a maximum yield of rAAV (i.e. vgs) with the minimum yield of particles achievable at such maximum yield of rAAV, i.e. the user maximises the yield, and then determines the helper plasmid to vector plasmid ratio that achieves that yield while providing the highest ratio of full to total particles.

Optionally, the ratio of helper plasmid to vector plasmid is adjusted to obtain the desired ratio of full to total particles and/or the high or desired yield of recombinant AAV. Optionally, the ratio of helper plasmid to vector plasmid is selected or adjusted to a ratio that allows the user to obtain the desired ratio of full to total particles or the high or desired yield of recombinant AAV.

Optionally, the high or desired yield is a yield that is at least 1.2 fold, at least 1.4 fold, at least 1.6 fold, at least 1.8 fold or at least 2 fold higher than the yield achieved using an equivalent method or use with a ratio of helper plasmid to vector plasmid of 1:3.

Optionally, the desired ratio of full to total particles is a ratio of full to total particles that is at least 2 fold, at least 3 fold, or at least 3.5 fold higher than the ratio of full to total particles achieved using an equivalent method with a ratio of helper plasmid to vector plasmid of 1:3. Optionally, the ratio of helper plasmid to vector plasmid that is used, selected or adjusted to is between 1:10 to 10:1, between 1:3 and 7:3, between 1:3 and 6:3, preferably around 1:3 or around 2:3 or around 3:3 or around 4:3 or around 5:3, or around 6:3.

### A low level of replication competent AAV (rcAAV)

The present invention provides a method for reducing and/or minimising the level of replication competent AAV (rcAAV) produced during recombinant AAV (rAAV) production comprising:
(a) obtaining the two-plasmid system, or the helper plasmid of the invention;
(b) transfecting a host cell with the two-plasmid system, or the helper plasmid of the invention; and
(c) culturing the host cell in suspension under conditions suitable for rAAV production.

The method may further comprise a step of harvesting the rAAV to provide an rAAV preparation, optionally having a low level, a reduced level and/or a minimised level of rcAAV.

The present invention also encompasses a method for reducing and/or minimising the level of pseudo-wild type replication competent AAV (rcAAV) produced during recombinant AAV (rAAV) production comprising:
(a) obtaining the two-plasmid system, or the helper plasmid of the invention;
(b) transfecting a host cell with the two-plasmid system, or the helper plasmid of the invention; and
(c) culturing the host cell in suspension under conditions suitable for rAAV production.

The method may further comprise a step of harvesting the rAAV to provide an rAAV preparation, optionally having a low level of pseudo-wild type rcAAV.

*"RAAV"* or *"recombinantAAV"* refers to AAV particles, i.e. particles comprising an AAV genome (such as a vector genome) and an AAV capsid.

For the purposes of the present invention, the terms *"rcAAV"* or *"replication competent AAV"* is intended to refer to rAAV particles that comprise a genome which comprises a rep gene or a genome which comprises a cap gene. Whilst, for the purposes of the present invention, the term *"rcAAV"* is intended to refer to rAAV particles that comprise a genome comprising a rep gene or a cap gene, it is understood that the rAAV particles that comprise a genome comprising a rep gene but not a cap gene (herein so-called *"cap-deficient rcAAV"*) or a genome comprising a cap gene but not a rep gene (herein so-called *"rep-deficient rcAAV"*) are not replication competent in isolation. Rather, in order to replicate (in the presence of the necessary helper virus functions, but without any requirement for AAV functions provided *in trans*) an AAV particle must comprise a rep gene and a cap gene (so-called *"pseudo-wild type rcAAV*")*.* However, an AAV particle that comprises a rep gene but does not comprise a cap gene can replicate when co-infected in a host cell with an AAV particle that comprises a cap gene, and an AAV particle that comprises a cap gene but does not comprise a rep gene can replicate when co-infected in a host cell with an AAV particle that comprises a rep gene. Thus, the term *"rcAAV"* encompasses *"cap-deficient rcAAV",* but also *"pseudo-wild type rcAAV"* and *"rep-deficient rcAAV".* Pseudo-wild type rcAAV may (in the presence of helper virus functions) be able to replicate in a host cell without co-infection by another AAV particle, and produce progeny virus. Pseudo-wild type rcAAV may comprise a rep gene and a cap gene flanked by ITR sequences.

The term *"rep-rcAAV"* encompasses *"pseudo-wild type rcAAV"*and *"cap-deficient rcAAV".* The term *"cap-rcAAV"* encompasses *"pseudo-wild type rcAAV"* and *"rep-deficient rcAAV".*

All species of rcAAV are undesirable in an rAAV preparation. Cap-deficient rcAAV may replicate when co-infected with cap-containing AAV particles. Rep-deficient rcAAV may replicate when co-infected with rep-containing AAV particles, and pseudo-wild type rcAAV may replicate without AAV co-infection. If the rcAAV replicates, it can cause significant side effects. Accordingly, reducing and/or minimising the level of rcAAV produced is advantageous. As used herein, the term *"reducing the level of rcAAV"* refers to decreasing the number of rcAAV that are produced. As used herein the term *"minimising the level of rcAAV"* refers to reducing the level of rcAAV to the lowest level possible given the constraints of the method.

Using the methods of the invention, the rAAV genome that is packaged should not comprise either a rep gene or a cap gene as, in general, neither the rep gene nor the cap gene is linked (on the same plasmid, in close proximity) to an ITR. However, single recombination events could result in the generation of cap-deficient or rep-deficient rcAAV. To generate pseudo-wild-type rcAAV, two recombination events must occur, and this is highly unlikely.

Whether or not an rAAV particle comprises a genome comprising a rep gene, or a preparation of rAAV particles comprises a particle comprising a genome comprising a rep gene, may be determined using qPCR. Optionally, the amount of rcAAV is measured by determining the copy number of rep produced. Optionally, the amount of rcAAV is measured by determining the copy number of rep per cell (i.e. host cell) using qPCR.

A primer (or two primers) specific for the rep gene should be used in the qPCR. Optionally, the (or both) primer(s) is/are specific for (reverse and complementary to or identical to depending on whether the primer is a forward primer or a reverse primer) a region of at least 12, at least 14, at least 16, or at least 18 of nucleotides 321-2252 of SEQ ID NO: 1, which encode the four Rep proteins. Optionally, the primer is specific for rep 40. Optionally, the primer is specific for rep 52. Optionally, the primer is specific for rep 68.

To determine the copy number of rep per cell using qPCR a second pair of primers should be used in the qPCR, which is specific for an endogenous gene (such as an endogenous housekeeping gene) in the host cell such as human albumin in HEK293 cells. Optionally, each primer or pair of primers is specific for (complementary to) a region of at least 8, at least 10, at least 12 or at least 15 nucleotides of the genomic sequence of human albumin. The copy number per cell may then be determined as the ratio of the copy number of rep to the copy number of the endogenous gene.

Alternatively, the above method to detect/measure rcAAV (pseudo-wild type and/or cap-deficient rcAAV) may be performed by cap gene-specific qPCR which can be used to detect rep-deficient rcAAV.

The methods or uses may provide an rAAV preparation. In such embodiments, the rAAV preparation preferably comprises a low level, a reduced level and/or a minimised level of rcAAV. A low level, a reduced level and/or a minimised level of rcAAV is generally a level of rcAAV that is lower than 1 rcAAV in 10⁷ rAAV, or is lower than that in an rAAV preparation that is produced using a method equivalent to that of a method of the invention, except that the vector plasmid comprises both at least one rep gene and at least one cap gene or the two-plasmid system is a rep 78 negative two-plasmid system. The level of rAAV can be determined by using qPCR to determine the number of vector genomes, as described above in the section entitled *"high yield".*

Optionally, the low level, reduced level and/or minimised level of rcAAV is less than 1 rcAAV in 10⁷ recombinant AAV, less than 1 rcAAV in 10⁹ recombinant AAV, or less than 1 rcAAV in 10¹⁰ recombinant AAV. Optionally, the level of rcAAV is less than the level of rcAAV produced using an equivalent method except that the vector plasmid comprises both the at least one rep gene and at least one cap gene or the two-plasmid system is a rep 78 negative two-plasmid system. Optionally, the level of rcAAV is measured using the qPCR assay above. Optionally, the level of rcAAV is measured using the qPCR assay after two or three generations of culturing. Optionally, the rcAAV is pseudo-wild type rcAAV. Optionally, the rcAAV is cap-deficient rcAAV. Optionally, the rcAAV is rep-deficient rcAAV. Optionally, the rcAAV comprises pseudo-wild type rcAAV, cap-deficient rcAAV and rep-deficient rcAAV.

The phrase *"method equivalent to that of a method of the invention"* is intended to refer to a method that is identical, except that the vector plasmid comprises both the at least one rep gene and at least one cap gene or the two-plasmid system is a rep 78 negative two-plasmid system.

Optionally, the low level of rcAAV comprises a low level of rep-rcAAV. When the rcAAV *"comprises a low level, a reduced level and*/*or a minimised level of rep-rcAAV",* then the rcAAV present in the preparation has at most a small proportion of rep-rcAAV. Thus, the phrase *"low level, reduced level and*/*or minimised level of rcAAV comprises a low level of rep-rcAAV"* as used herein means that the low level, reduced level and/or minimised level of rcAAV present in a preparation has at most a small proportion of rep-rcAAV. Optionally, the low level, reduced level and/or minimised level of rcAAV comprises a lower level of rep-rcAAV compared to the level of rep-rcAAV produced using a two-plasmid system comprising a plasmid comprising both at least one rep gene and at least one cap gene or a two-plasmid system which is a rep 78 negative two-plasmid system (using a ratio of helper plasmid to vector plasmid of 1:3).

The two-plasmid system comprising a plasmid comprising both at least one rep gene and at least one cap gene or that is a rep 78 negative two-plasmid system is also referred to herein as *"the two-plasmid system used for comparison".* The two-plasmid system used for comparison may be in a conventional "non-split" configuration. The plasmid comprising both at least one rep gene and at least one cap gene (of the two-plasmid system used for comparison) may comprise all four rep genes (e.g. the AAV2 rep cassette). The plasmid comprising both at least one rep gene and at least one cap gene (of the two-plasmid system used for comparison) may comprise the same cap gene sequence as the two-plasmid system of the invention. The plasmid comprising both at least one rep gene and at least one cap gene (of the two-plasmid system used for comparison) may comprise the same rep gene sequence as the two-plasmid system of the invention. The two-plasmid system used for comparison may comprise a plasmid comprising the same AdV (adenovirus) helper and vector genome sequences (e.g. expression cassette) as the two-plasmid system of the invention. For example, the two-plasmid system used for comparison may comprise a plasmid which comprises the same AdV helper and vector genome sequences (e.g. expression cassette) as the two-plasmid system of the invention, and the plasmid comprising both at least one rep gene and at least one cap gene (of the two-plasmid system used for comparison) comprises the same cap gene sequence and the same rep gene sequence as the two-plasmid system of the invention. As a further example, the two-plasmid system used for comparison may comprise a plasmid which comprises the same AdV helper and vector genome sequences (e.g. expression cassette) as the two-plasmid system of the invention, and the plasmid comprising both at least one rep gene and at least one cap gene (of the two-plasmid system used for comparison) comprises the same cap gene sequence as two-plasmid system of the invention and an AAV (e.g. AAV2) rep cassette comprising all four rep genes. Optionally, the plasmids of the two-plasmid system used for comparison are transfected in a molar plasmid ratio of 1.8:1 (AdV helper-vector genome plasmid: rep-cap plasmid).

Optionally, the low level, reduced level and/or minimised level of rcAAV comprises an at least 5 times, at least 10 times, at least 25 times, at least 30 times, or at least 35 times excess of rep-rcAAV or cap-rcAAV. Optionally, the low level, reduced level and/or minimised level of rcAAV comprises an at least 5 times, at least 10 times, at least 25 times, at least 30 times, or at least 35 times excess of cap-rcAAV, i.e. the rcAAV may comprise at least 5 times, at least 10 times, at least 25 times, at least 30 times, or at least 35 times more cap sequences than rep sequences. Optionally, the low level, reduced level and/or minimised level of rcAAV comprises between an at least 5 times and an at least 100 times, or between an at least 25 times and an at least 50 times excess of cap-rcAAV, i.e. the low level, reduced level and/or minimised level of rcAAV may comprise between at least 5 times and at least 100 times, or between at least 25 times and at least 50 times more cap sequences than rep sequences. Where the level of cap-rcAAV is in excess, then the level of rep-rcAAV must be lower. A lower level of rep-rcAAV indicates a low level, reduced level and/or minimised level of pseudo wild-type rcAAV (because pseudo-wild type rcAAV comprises the rep gene).

Optionally, the low level, reduced level and/or minimised level of rcAAV comprises an at least 5 times, at least 10 times, at least 25 times, at least 30 times, or at least 35 times excess of rep-rcAAV, i.e. the rcAAV may comprise at least 5 times, at least 10 times, at least 25 times, at least 30 times, or at least 35 times more rep sequences than cap sequences. Optionally, the low level, reduced level and/or minimised level of rcAAV comprises between an at least 5 times and an at least 100 times, or between an at least 25 times and an at least 50 times excess of rep-rcAAV, i.e. the low level of rcAAV may comprise between at least 5 times and at least 100 times, or between at least 25 times and at least 50 times more rep sequences than cap sequences. Where the level of rep-rcAAV is in excess, then the level of cap-rcAAV must be lower. A lower level of cap-rcAAV indicates a low level of pseudo wild-type rcAAV (because pseudo-wild type rcAAV comprises the cap gene).

The likelihood of whether a rep or cap gene is packaged into an AAV particle is increased if the plasmid comprising the rep or cap gene also comprises at least one ITR sequence. For example, if one plasmid comprises the rep gene and does not comprise at least one ITR sequence, and another plasmid comprises the cap gene and at least one ITR sequence, then there is likely to be more cap gene packaged than rep gene. As a further example, if one plasmid comprises the cap gene and does not comprise at least one ITR sequence, and another plasmid comprises the rep gene and at least one ITR sequence, then there is likely to be more rep gene packaged than cap gene.

Optionally, the low level, reduced level and/or minimised level of rcAAV comprises a proportion of less than 1/5 rep-rcAAV, less than 1/10 rep-rcAAV, less than 1/25 rep-rcAAV, less than 1/30 rep-rcAAV, or less than 1/35 rep-rcAAV. As an example, where the rcAAV comprises a proportion less than 1/5 rep-rcAAV, then at least 4/5 of the rcAAV must be cap-rcAAV.

Optionally, the low level, reduced level and/or minimised level of rcAAV comprises a proportion of rep-rcAAV that is less than the proportion produced using a two-plasmid system comprising a plasmid comprising both at least one rep gene and at least one cap gene or a two-plasmid system that is a rep 78 negative two-plasmid system. Optionally, the proportion of rep-rcAAV is less than 90%, less than 75%, less than 60%, less than 50%, less than 25%, less than 20%, less than 17%, or less than 15% of the proportion produced using the two-plasmid system comprising a plasmid comprising both at least one rep gene and at least one cap gene. The two-plasmid system comprising a plasmid comprising both at least one rep gene and at least one cap gene, or that is a rep 78 negative two-plasmid system is also referred to herein as the two-plasmid system used for comparison.

Optionally, the low level, reduced level and/or minimised level of rcAAV comprises a low level of pseudo-wild type rcAAV. Optionally, the low level, reduced level and/or minimised level of rcAAV comprises less than 1/5 pseudo-wild type rcAAV, less than 1/10 pseudo-wild type rcAAV, less than 1/25 pseudo-wild type rcAAV, less than 1/30 pseudo-wild type rcAAV, or less than 1/35 pseudo-wild type rcAAV.

In the rcAAV produced, where the quantities of rep and cap sequences are not equal, the lower amount between the quantities of rep and cap sequences is an indicator of the maximum possible number of AAV species which contain rep and cap genes on the same DNA molecule. Therefore, the lower amount between the quantities of rep and cap sequences is an indicator of the maximum possible number of pseudo-wild type rcAAV. Thus, where either the rep-rcAAV or cap-rcAAV is in excess, it is likely that the amount of pseudo-type rcAAV is lower than if the levels of rep-rcAAV and cap-rcAAV were equal. The lower amount between the quantities of rep and cap sequences as a proportion of the upper amount between the quantities of rep and cap sequences is an indicator of the proportion of rcAAV which is pseudo-wild type rcAAV. As an example, if there are 40 times more cap sequences than rep sequences, then the proportion of rcAAV which is pseudo-wild type rcAAV may be 1/40. In the rcAAV produced, the proportion of rcAAV which is pseudo-wild type rcAAV may be less than 1/5, less than 1/10, less than 1/25, less than 1/30, or less than 1/35.

The quantity of cap and rep sequences may be measured by qPCR. Optionally, the level of rep-rcAAV is the level of rep-rcAAV detected by qPCR using primers binding to rep68 exon 1. Optionally, the level of rep-rcAAV is the level of rep-rcAAV detected by qPCR using the forward primer CACGTGCATGTGGAAGTAG (SEQ ID NO: 10) and the reverse primer CGACTTTCTGACGGAATGG (SEQ ID NO: 11). Optionally, the level of cap-rcAAV is the level of cap-rcAAV detected by qPCR using primers binding to a sequence encoding VP3. Optionally, the level of cap-rcAAV is the level of cap-rcAAV detected by qPCR using the forward primer TACTGAGGGACCATGAAGAC (SEQ ID NO: 12) and the reverse primer GTTTACGGACTCGGAGTATC (SEQ ID NO: 13).

Optionally, an excess of rep-rcAAV or cap-rcAAV indicates that the level of pseudo wild-type rcAAV is reduced and/or minimised. Optionally, the excess is at least 5 times, at least 10 times, at least 25 times, at least 30 times, or at least 35 times excess of rep-rcAAV or cap-rcAAV. Optionally, the excess is between at least 5 times and at least 100 times, or between at least 25 times and at least 50 times excess of rep-rcAAV or cap-rcAAV. Optionally, the low level of rcAAV comprises an at least 5 times, at least 10 times, at least 25 times, at least 30 times, or at least 35 times excess of cap-rcAAV, i.e. the rcAAV may comprise at least 5 times, at least 10 times, at least 25 times, at least 30 times, or at least 35 times more cap sequences than rep sequences. Optionally, the low level of rcAAV comprises between an at least 5 times and an at least 100 times, or between an at least 25 times and an at least 50 times excess of cap-rcAAV, i.e. the low level of rcAAV may comprise between at least 5 times and at least 100 times, or between at least 25 times and at least 50 times more cap sequences than rep sequences. Where the level of cap-rcAAV is in excess, then the level of rep-rcAAV must be lower. A lower level of rep-rcAAV indicates a low level of pseudo wild-type rcAAV (because pseudo-wild type rcAAV comprises the rep gene). Optionally, the low level of rcAAV comprises an at least 5 times, at least 10 times, at least 25 times, at least 30 times, or at least 35 times excess of rep-rcAAV, i.e. the rcAAV may comprise at least 5 times, at least 10 times, at least 25 times, at least 30 times, or at least 35 times more rep sequences than cap sequences. Optionally, the low level of rcAAV comprises between an at least 5 times and an at least 100 times, or between an at least 25 times and an at least 50 times excess of rep-rcAAV, i.e. the low level of rcAAV may comprise between at least 5 times and at least 100 times, or between at least 25 times and at least 50 times more rep sequences than cap sequences. Where the level of rep-rcAAV is in excess, then the level of cap-rcAAV must be lower. A lower level of cap-rcAAV indicates a low level of pseudo wild-type rcAAV (because pseudo-wild type rcAAV comprises the cap gene). Optionally, the excess is greater than the excess produced using a two-plasmid system comprising a plasmid comprising both at least one rep gene and at least one cap gene. The two-plasmid system comprising a plasmid comprising both at least one rep gene and at least one cap gene is also referred to herein as the two-plasmid system used for comparison.

Optionally, the quantity of cap and rep sequences in an rAAV preparation is measured following at least one round of amplification. For example, the quantity of cap and rep sequences in an rAAV preparation may be measured following two rounds of amplification. To perform a round of amplification, optionally, suitable host cells (such as HEK293 cells) are infected with the rAAV preparation, co-infected with adenovirus and incubated under conditions suitable for the production of rAAV. The cells may be lysed and the quantity of cap and rep sequences in the lysate measured. Optionally, the lysate may be used for a second round of amplification. For example, the lysate may be used to infect further suitable host cells (such as HEK293 cells) which are also co-infected with adenovirus and incubated under conditions suitable for the production of rAAV. The cells may be lysed and the quantity of cap and rep sequences in the lysate measured.

### Transfecting, culturing and harvesting

The methods of the invention may comprise steps of obtaining the two-plasmid system or the helper plasmid of the invention, transfecting a host cell with the two-plasmid system, or the helper plasmid, and culturing the host cell in suspension under conditions suitable for recombinant AAV production.

Transfecting a host cell with the two-plasmid system, or the helper plasmid, may comprise exposing the host cell to the two-plasmid system, or the helper plasmid in conditions suitable for transfection. For example, the user of the method may add a transfection agent (addition of a transfection agent would be considered to be a condition suitable for transfection). Alternatively, calcium phosphate transfection, electroporation or cationic liposomes could be used. Optionally, the step of transfecting the host cell takes place when the host cell has grown to confluence.

Culturing the host cell under conditions suitable for rAAV production refers to culturing the host cell under conditions at which it can grow and AAV can replicate. For example, the host cell may be cultured at a temperature between 32°C and 40°C, between 34°C and 38°C, between 35°C and 38°C, or around 37°C. Optionally, the host cell may be cultured in the presence of a complete cell culture medium such as Dulbecco's Modified Eagle's Medium (DMEM). A complete cell culture medium is a medium that provides all the essential nutrients required for growth of the host cell. Optionally, the complete cell culture medium is supplemented with serum, such as fetal bovine serum or bovine serum albumin.

Optionally, the host cell is a host cell such as those defined above under the heading *"host cell".* Optionally, the host cell is selected from the group consisting of a HEK293T cell, a HEK293 cell, a HEK293EBNA cell, a CAP cell, a CAP-T cell, an AGE1.CR cell, a PerC6 cell, a C139 cell, and an EB66 cell. Optionally, the host cell is a cell that expresses a functional E1A/B protein.

The helper plasmid and two-plasmid system may be used to produce AAV in any suitable cell culture system. In a particularly preferred aspect of the invention, culturing the host cell under conditions suitable for recombinant AAV production comprises culturing the host cell using a suspension system.

For the purposes of the present invention, the term *"suspension system"* refers to a system suitable for suspension cell culture, i.e. a system which allows cells to grow free-floating in culture medium. Cells in a suspension system may form aggregates, or may be suspended in medium as single cells. For the purposes of the present invention, the term *"adherent system"* refers to a system suitable for adherent cell culture, i.e. for cells to be cultured whilst anchored to a substrate. Optionally, an adherent system refers to a flask or fermenter which forms a container to which cells can bind, and optionally is specifically treated to allow cell adhesion and spreading. Alternatively, an adherent system may be a *"carrier system",* in which the container contains an additional carrier such as a bead or a fibre to which the cells can adhere. In such *"carrier"* adherent systems, the cells tend to adhere less tightly and to have a morphology that is more similar to the morphology of cells grown using suspension systems compared to cells grown in conventional adherent systems.

The method may further comprise a step of purifying the rAAV. In general, a step of purifying the rAAV will involve increasing the concentration of the rAAV compared to other components of the preparation. Optionally, the step of purifying the rAAV results in a concentrated rAAV preparation. Optionally, the step of purifying the rAAV results in an isolated rAAV.

Any suitable purification method may be used. Optionally, the step of purifying the rAAV comprises a technique selected from the group consisting of gradient density centrifugation (such as CsCI or lodixanol gradient density centrifugation), filtration, ion exchange chromatography, size exclusion chromatography, affinity chromatography and hydrophobic interaction chromatography. Optionally, the step of purifying the rAAV is carried out using a technique selected from the group consisting of gradient density centrifugation (such as CsCI or lodixanol gradient density centrifugation), filtration, ion exchange chromatography, size exclusion chromatography, affinity chromatography and hydrophobic interaction chromatography.

Optionally, the method comprises further concentrating the rAAV using ultracentrifugation, tangential flow filtration, or gel filtration.

Optionally, the method comprises formulating the rAAV with a pharmaceutically acceptable excipient. The pharmaceutically acceptable excipients may comprise carriers, diluents and/or other medicinal agents, pharmaceutical agents or adjuvants, etc. Optionally, the pharmaceutically acceptable excipients comprise saline solution. Optionally, the pharmaceutically acceptable excipients comprise human serum albumin.

### Recombinant AAV (rAAV) preparations

The present invention further provides a recombinant AAV (rAAV) preparation obtainable by a method of the invention. The invention further provides recombinant AAV preparation obtained by a method of the invention.

The recombinant AAV preparations obtainable or obtained by the methods of the invention are advantageous, as they generally comprise a low level of rcAAV, and/or have a desired ratio of full to total particles.

| Sequence identity number | Sequence |
|---|---|
| 1 | Genome of AAV2 (AF043303.1) |
| 2 | Genome of AdV5 (AC_000008.1) |
| 3 | Genome of AdV2 (AC_000007.1) |
| 4 | Helper gene region |
| 5 | Alternative AdV5 genome (AY339865.1) |
| 6 | Rep binding site |
| 7 | Rep gene + p5 promoter region of Rep78pos helper plasmid |
| 8 | Rep 78 gene region of Rep78pos helper plasmid (321-2186 of AAV2) |
| 9 | Rep gene region of Rep78pos helper plasmid (321-2252 of AAV2) |
| 10 | rcAAV qPCR rep forward primer |
| 11 | rcAAV qPCR rep reverse primer |
| 12 | rcAAV qPCR cap forward primer |
| 13 | rcAAV qPCR cap reverse primer |
| 14 | Rep gene common region Rep78pos helper plasmid) (993-1710 of AAV2) |
| 15 | Rep78 protein |
| 16 | Amino acid sequence corresponding to common portion |
| 17 | E4 amino acid sequence encoded by ORFs 6/7 (nucleotides 32914-33192 and 33904-34077 of SEQ ID NO: 2) |
| 18 | E2A amino acid sequence encoded by nucleotides 22443-24032 of SEQ ID NO: 2 |

Based on the disclosure provided herein, those skilled in the art will recognize, or be able to ascertain using no more than routine experimentation, many equivalents to the specific embodiments of the invention described herein. Such equivalents are intended to be encompassed by the following numbered aspects and embodiments.

Specifically, the present disclosure provides the following numbered aspects, advantageous features and specific embodiments, respectively alone or in combination:
1. A helper plasmid which does not comprise a cap gene encoding a functional set of Cap proteins and which does comprise at least one rep Adeno-associated Virus (AAV) gene and at least one helper virus gene, wherein the at least one rep gene comprises a rep 78 cassette comprising a nucleotide sequence encoding a functional Rep 78 protein and a transcription regulatory element or a rep 78 cassette comprising a nucleotide sequence encoding a functional Rep 78 protein and a functional Rep 68 protein and a transcription regulatory element, and wherein said rep 78 cassette comprises an ATG start codon at the beginning of the nucleotide sequence encoding a functional Rep 78 protein or both a functional Rep 78 protein and a functional Rep 68 protein.
2. A helper plasmid which does not comprise a cap gene encoding a functional set of Cap proteins and which does comprise at least one rep Adeno-associated Virus (AAV) gene and at least one helper virus gene, wherein the at least one rep gene comprises a rep 78 cassette comprising a nucleotide sequence encoding a functional Rep 78 protein and a transcription regulatory element, and wherein said rep 78 cassette comprises an ATG start codon at the beginning of the nucleotide sequence encoding said functional Rep 78 protein.
3. A helper plasmid according to aspect 1 which does not comprise a cap gene encoding a functional set of Cap proteins and which does comprise at least one rep gene and at least one helper virus gene, wherein the at least one rep gene comprises a rep 78 cassette comprising a nucleotide sequence encoding a functional Rep 78 protein and a transcription regulatory element or a rep 78 cassette comprising a nucleotide sequence encoding a functional Rep 78 protein and a functional Rep 68 protein and a transcription regulatory element, and wherein said rep 78 cassette comprises an ATG start codon at the beginning of the nucleotide sequence encoding a functional Rep 78 protein or both a functional Rep 78 protein and a functional Rep 68 protein, and wherein said helper plasmid promotes production of recombinant AAV at a higher yield compared to a helper plasmid comprising at least one rep gene which does not encode a Rep 78 protein.
4. A helper plasmid according to aspect 2 which does not comprise a cap gene encoding a functional set of Cap proteins and which does comprise at least one rep gene and at least one helper virus gene, wherein the at least one rep gene comprises a rep 78 cassette comprising a nucleotide sequence encoding a functional Rep 78 protein and a transcription regulatory element, and wherein said rep 78 cassette comprises an ATG start codon at the beginning of the nucleotide sequence encoding a functional Rep 78 protein, and wherein said helper plasmid promotes production of recombinant AAV at a higher yield compared to a helper plasmid comprising at least one rep gene which does not encode a Rep 78 protein.
5. A helper plasmid according to aspect 2 or 4, which does not comprise a cap gene encoding a functional set of Cap proteins and which does comprise at least one rep gene and at least one helper virus gene, wherein the at least one rep gene comprises a rep 78 cassette comprising a nucleotide sequence encoding a functional Rep 78 protein and a transcription regulatory element, and wherein the rep 78 cassette is under the control of a wild type AAV p5 promoter preferably an AAV2 p5 promoter, and comprises an ATG start codon at the beginning of the nucleotide sequence encoding a functional Rep 78 protein, and wherein said helper plasmid promotes production of recombinant AAV at a higher yield compared to a helper plasmid comprising at least one rep gene which does not encode a Rep 78 protein.
6. A helper plasmid which does not comprise a cap gene encoding a functional set of Cap proteins and which does comprise at least one rep gene and at least one helper virus gene, wherein the at least one rep gene comprises a rep 78 cassette comprising a nucleotide sequence encoding a functional Rep 78 protein and a transcription regulatory element or a rep 78 cassette comprising a nucleotide sequence encoding a functional Rep 78 protein and a functional Rep 68 protein and a transcription regulatory element, and wherein said rep 78 cassette comprises an ATG start codon at the beginning of the nucleotide sequence encoding a functional Rep 78 protein or both a functional Rep 78 protein and a functional Rep 68 protein, and wherein said helper plasmid promotes production of recombinant AAV comprising a lower level of nucleic acid impurities compared to a helper plasmid comprising at least one rep gene which does not encode a functional Rep 78 protein.
7. A helper plasmid which does not comprise a cap gene encoding a functional set of Cap proteins and which does comprise at least one rep gene and at least one helper virus gene, wherein the at least one rep gene comprises a rep 78 cassette comprising a nucleotide sequence encoding a functional Rep 78 protein and a transcription regulatory element, and wherein said rep 78 cassette comprises an ATG start codon at the beginning of the nucleotide sequence encoding a functional Rep 78 protein and wherein said helper plasmid promotes production of recombinant AAV comprising a lower level of nucleic acid impurities compared to a helper plasmid comprising at least one rep gene which does not encode a functional Rep 78 protein.
8. The helper plasmid of any of the preceding aspects, wherein said nucleic acid impurities comprise (a) a lower level of plasmid backbone impurities; and/or (b) a lower level of helper plasmid backbone impurities; and/or (c) a lower level of host cell derived impurities
9. The helper plasmid of any one of the preceding aspects, wherein the start codon at the beginning of the nucleotide sequence encoding a functional Rep 78 protein or a both a functional Rep 78 protein and a functional Rep 68 protein is a start codon in a position corresponding to nucleotides 321 to 323 of SEQ ID NO: 1.
10. The helper plasmid of any one of the preceding aspects 3, wherein the start codon at the beginning of the nucleotide sequence encoding a functional Rep 78 protein is a start codon in a position corresponding to nucleotides 321 to 323 of SEQ ID NO: 1.
11. The helper plasmid of any one of the preceding aspects, wherein the nucleotide sequence encoding a functional Rep 78 protein has at least 90%, at least 95%, at least 98%, at least 99%, or 100% identity to the full length or a fragment of at least 1500, at least 1600, at least 1700, at least 1800, or at least 1850 nucleotides of SEQ ID NO: 15.
12. The helper plasmid of any one of the preceding aspects, wherein the nucleotide sequence encoding a functional Rep 78 protein has at least 98% identity to a fragment of at least 1800 nucleotides of SEQ ID NO: 15.
13. The helper plasmid of any one of the preceding aspects, wherein the nucleotide sequence encoding a functional Rep 78 protein has at least 99% identity to a fragment of at least 1850 nucleotides of SEQ ID NO: 15.
14. The helper plasmid of any one of the preceding aspects, wherein the nucleotide sequence encoding a functional Rep 78 protein has at least 98%, or at least 99% identity to SEQ ID NO: 15.
15. The helper plasmid of any one of the preceding aspects, wherein the at least one rep gene further comprises a gene encoding:
   (a) a functional Rep 52 protein;
   (b) a functional Rep 40 protein; and/or
   (c) a functional Rep 68 protein.
16. The helper plasmid of any one of the preceding aspects, wherein the at least one rep gene further comprises a gene encoding a functional Rep 52 protein, a gene encoding a functional Rep 40 protein, and a gene encoding a functional Rep 68 protein.
17. The helper plasmid of aspect 16, wherein the gene encoding a functional Rep 52 protein, the gene encoding a functional Rep 40 protein, the gene encoding a functional Rep 68 protein, and the gene encoding a functional Rep 78 protein all contain a common portion.
18. The helper plasmid of aspect 17, wherein the common portion comprises a sequence having at least 90%, at least 95%, at least 98%, at least 99%, or 100% identity to the full length or a fragment of at least 500, at least 550, at least 600, at least 650, or at least 700 nucleotides of SEQ ID NO: 14.
19. The helper plasmid of aspect 17 or 18, wherein the common portion encodes an amino acid sequence having at least 90%, at least 95%, at least 98%, at least 99%, or 100% identity to the full length or a fragment of at least 150, at least 210, at least 220, at least 230, or at least 238 amino acids of SEQ ID NO: 16.
20. The helper plasmid of any one of the preceding aspects, wherein the rep 78 cassette comprises fewer than 2400, fewer than 2300, fewer than 2200, or fewer than 2100 nucleotides, and encodes a functional Rep 52 protein, a functional Rep 40 protein, a functional Rep 68 protein and a functional Rep 78 protein.
21. The helper plasmid of any one of the preceding aspects, wherein the at least one rep gene comprises a nucleic acid sequence having at least 95%, at least 98%, at least 99%, or 100% identity to the full length or to a fragment of at least 1000, at least 1400, at least 1500, or at least 1600 nucleotides in length of a stretch of nucleotides corresponding to nucleotides 321-2252 of SEQ ID NO: 1, or to corresponding stretches of nucleotides in a different serotype of AAV.
22. The helper plasmid of any one of the preceding aspects, wherein the at least one rep gene comprises a nucleic acid sequence having at least 95%, at least 98%, at least 99%, or 100% identity to the full length or to a fragment of at least 1700, at least 1800, or at least 1900 nucleotides in length of a stretch of nucleotides corresponding to nucleotides 321-2252 of SEQ ID NO: 1, or to corresponding stretches of nucleotides in a different serotype of AAV.
23. The helper plasmid of any one of the preceding aspects, wherein the at least one rep gene does not comprise a functional internal p40 promoter.
24. The helper plasmid of any one of the preceding aspects, wherein the at least one helper virus gene is an adenovirus gene.
25. The helper plasmid of any one of the preceding aspects, wherein the at least one helper virus gene is an Adenovirus 5 or Adenovirus 2 gene.
26. The helper plasmid of any one of the preceding aspects, wherein the least one helper virus gene comprises:
   (a) a VA (viral associated) nucleic acid encoding functional VA RNA I and II;
   (b) an E2A gene encoding a functional E2A protein; and/or
   (c) an E4 gene encoding a functional E4 protein.
27. The helper plasmid of aspect 26, wherein the at least one helper virus gene comprises a VA nucleic acid, an E2A gene and an E4 gene.
28. The helper plasmid of aspect 27, wherein the E4 gene is not located between the VA nucleic acid and the E2A gene.
29. The helper plasmid of any one of the preceding aspects, wherein the at least one helper virus gene is comprised on a contiguous stretch of the plasmid having at least 95%, at least 98%, at least 99%, or 100% identity to the full length or to a fragment at least 6000, at least 7000, or at least 8000 nucleotides in length of SEQ ID NO: 4.
30. The helper plasmid of any one of the preceding aspects, wherein the helper plasmid is less than 20000 bp, less than 15000 bp, less than 14000 bp, less than 13000bp, between 10000 bp and 20000 bp, between 11000 bp and 15000 bp, between 12000 bp and 13000 bp, around 12941 bp, or around 12668 bp in length.
31. The helper plasmid of any one of the preceding aspects, wherein the helper plasmid does not comprise a gene encoding a functional adenoviral E1A/B protein.
32. The helper plasmid of any one of the preceding aspects, wherein the helper plasmid does not comprise a contiguous sequence of at least 2000, at least 2500, at least 3000, or 3427 nucleotides of a contiguous stretch of nucleotides of equivalent length comprised within nucleotides 194-3620 of SEQ ID NO: 2, or a corresponding stretch of nucleotides in a different serotype of adenovirus.
33. The helper plasmid of any one of the preceding aspects, wherein the helper plasmid does not comprise a contiguous sequence of at least 50, at least 60, or 69 nucleotides of a contiguous stretch of nucleotides of equivalent length comprised within nucleotides 4032-4100 of SEQ ID NO: 2, or a corresponding stretch of nucleotides in a different serotype of adenovirus.
34. The helper plasmid of any one of the preceding aspects, wherein the helper plasmid does not comprise a contiguous sequence of at least 200, at least 300, at least 350, or 363 nucleotides of a contiguous stretch of nucleotides of equivalent length comprised within nucleotides 4051-4413 of SEQ ID NO: 1, or a corresponding stretch of nucleotides in a different serotype of AAV.
35. The helper plasmid of any one of the preceding aspects, wherein the helper plasmid does not comprise a contiguous sequence of at least 200, at least 300, at least 350, or 361 nucleotides of a contiguous stretch of nucleotides of equivalent length comprised within nucleotides 4051-4411 of SEQ ID NO: 1, or a corresponding stretch of nucleotides in a different serotype of AAV.
36. The helper plasmid of any one of the preceding aspects, wherein the helper plasmid does not comprise a contiguous sequence of at least 400, at least 500, at least 600, or 647 nucleotides of a contiguous stretch of nucleotides of equivalent length comprised within nucleotides 2301-2947 of SEQ ID NO: 1, or a corresponding stretch of nucleotides in a different serotype of AAV.
37. The helper plasmid of any one of the preceding aspects, wherein the VA nucleic acid has an activity level which has at least 75%, at least 80%, at least 90%, at least 95%, or between 95% and 100% of the activity of a wild type VA nucleic acid from Adenovirus 5.
38. The helper plasmid of aspect 31, wherein the activity level of the VA nucleic acid is determined by measuring recombinant AAV yield.
39. The helper plasmid of any one of the preceding aspects, wherein the VA nucleic acid comprises a contiguous sequence that is at least 95%, at least 98%, or 100% identical to a stretch of at least 15 nucleotides, at least 20 nucleotides, or 25 nucleotides of nucleotides 10595-10619 of SEQ ID NO: 2, or a corresponding stretch of nucleotides in a different serotype of adenovirus.
40. The helper plasmid of any one of aspects the preceding aspects, wherein the E2A gene is operably linked to a promoter which comprises a contiguous sequence at least 96%, at least 98%, or 100% identical to a stretch of at least 60, at least 70, at least 80, or 100 nucleotides of nucleotides 27037-27136 of SEQ ID NO: 2, or a corresponding stretch of nucleotides in a different serotype of adenovirus.
41. The helper plasmid of any one of the preceding aspects, wherein the at least one helper virus gene comprises a VA nucleic acid, an E2A gene and an E4 gene, and wherein the VA nucleic acid, the E2A gene and the E4 gene are comprised within a contiguous portion of fewer than 15000, fewer than 12000, fewer than 10000, or fewer than 9000 nucleotides.
42. The helper plasmid of any one of the preceding aspects, wherein the helper plasmid does not comprise a contiguous sequence of at least 15000, at least 20000, at least 22000, or 22137 nucleotides of a contiguous stretch of nucleotides of equivalent length comprised within nucleotides 10619-32755 of SEQ ID NO: 2, or a corresponding stretch of nucleotides in a different serotype of adenovirus.
43. The helper plasmid of any one of the preceding aspects wherein the E4 gene is operably linked to an E4 promoter that has at least 50%, at least 70%, or at least 90% of the activity of a wild type promoter from Adenovirus 5.
44. The helper plasmid of aspect 43, wherein the E4 promoter comprises a sequence of at least 30, at least 40, or 55 nucleotides corresponding to nucleotides 35793-35848 of SEQ ID NO: 2, or a corresponding stretch of nucleotides in a different serotype of adenovirus.
45. The helper plasmid of any one of the preceding aspects, wherein the helper plasmid does not comprise an artificial Rep binding site.
46. The helper plasmid of any one of the preceding aspects, wherein the helper plasmid comprises a plasmid backbone, and the plasmid backbone does not comprise an artificial Rep binding site.
47. The helper plasmid of any one of the preceding aspects, wherein the helper plasmid does not comprise a contiguous stretch of exclusively cap gene sequence of more than 400, more than 250 nucleotides, more than 100 nucleotides, or more than 60 nucleotides.
48. The helper plasmid of aspect 47, wherein the helper plasmid does not comprise a contiguous stretch of exclusively cap gene sequence of more than 60 nucleotides.
49. The helper plasmid of any one of the preceding aspects, wherein the helper plasmid comprises a portion of cap gene sequence, and the portion of cap gene sequence does not encode a functional set of Cap proteins.
50. A plasmid system comprising the helper plasmid of any one of the preceding aspects.
51. A plasmid system, preferably a three-plasmid system comprising the helper plasmid of any one of the preceding aspects
52. A two-plasmid system according to aspect 50comprising the helper plasmid of any one of the preceding aspects and a vector plasmid.
53. The two-plasmid system of aspect 52, wherein the two-plasmid system comprises a molar excess of helper plasmid compared to vector plasmid.
54. The two-plasmid system of aspect 52 or 53, wherein the ratio of helper plasmid to vector plasmid is between 1:10 to 10:1, between 1:3 and 7:3, between 1:3 and 6:3, preferably around 1:3 or around 2:3 or around 3:3 or around 4:3 or around 5:3, or around 6:3.
55. The two-plasmid system of any one of aspects 52 to 54, wherein the vector plasmid comprises:
   (a) a cap gene encoding at least one functional Cap protein; or
   (b) at least one cap gene promoter, a cloning site operably linked to the cap gene promoter, and an expression cassette flanked on at least one side by an ITR;
   wherein the vector plasmid does not comprise a rep gene encoding a functional Rep protein and the expression cassette comprises a transgene operably linked to at least one regulatory control element.
56. The two-plasmid system of aspect 55, wherein the expression cassette is less than 5.0 kbp, less than 4.9 kbp, less than 4.8 kbp, less than 4.75 kbp, less than 4.7 kbp, or less than 4.5 kbp.
57. The two-plasmid system of aspect 55 or 56, wherein the expression cassette is between 2.0 kbp and 5.5 kbp, between 2.0 kbp and 4.75 kbp, between 2.0 kbp and 4.7 kbp, between 2.1 kbp and 4.9 kbp, between 2.2 kbp and 4.8 kbp or between 2.3 kbp and 4.5 kbp.
58. The two-plasmid system of any one of aspects 52 to 57, wherein the vector plasmid does not comprise an artificial Rep binding site.
59. The two-plasmid system of any one of aspects 52 to 58, wherein the vector plasmid comprises a plasmid backbone, and the plasmid backbone does not comprise an artificial Rep binding site.
60. The two-plasmid system of any one of aspects 52 to 59, wherein the vector plasmid comprises a cap gene operably linked to at least one cap gene promoter.
61. The two-plasmid system of any one of aspects 52 to 60, wherein the vector plasmid comprises a cap gene and further comprises an expression cassette flanked on at least one side by an ITR, optionally wherein the expression cassette comprises a transgene operably linked to at least one regulatory control element.
62. The two-plasmid system of any one of aspects 52 to 61, wherein the vector plasmid comprises a cap gene and the cap gene encodes a VP1, a VP2, and/or a VP3 protein.
63. The two-plasmid system of any one of aspects 52 to 62, wherein the vector plasmid comprises a cap gene and the cap gene encodes a Cap protein selected from the group of AAV serotypes consisting of serotypes 1, 2, 3A, 3B, 4, 5, 6, 7, 8, 9, 10, 11, 12, or 13, LK03, rh74, rh10.
64. The two-plasmid system of any one of aspects 52 to 63, wherein the vector plasmid comprises a cap gene and the cap gene encodes a Cap protein selected from the group of AAV serotypes consisting of serotypes 2, 5, 8, 9.
65. The two-plasmid system of any one of aspects 52 to 64, wherein the vector plasmid comprises an at least one cap gene promoter, which is a native cap gene promoter.
66. The two-plasmid system of any one of aspects 52 to 65, wherein the vector plasmid comprises an at least one cap gene promoter, which comprises an AAV p40 promoter, a p5 promoter, and/or a p19 promoter.
67. The two-plasmid system of aspect 66, wherein the at least one cap gene promoter comprises a p40 promoter.
68. The two-plasmid system of aspect 66 or 67, wherein the at least one cap gene promoter comprises a p40 promoter, a p5 promoter, and a p19 promoter.
69. The two-plasmid system of any one of aspects 52 to 68, wherein the transgene encodes an enzyme, a metabolic protein, a signalling protein, an antibody, an antibody fragment, an antibody-like protein, an antigen, or a non-translated RNA such as an miRNA, siRNA, snRNA, or antisense RNA.
70. The two-plasmid system of any one of aspects 52 to 69, wherein the cloning site is a multicloning site (MCS).
71. The two-plasmid system of any one of aspects 52 to 70, wherein the vector plasmid does not comprise any dispensable translation initiation codons.
72. The two-plasmid system of aspect 71, wherein the vector plasmid comprises a promoter region comprising one or more promoters, and the promoter region does not comprise ATG or GTG codons.
73. The two-plasmid system of aspect 72, wherein the promoter region comprises p5, p19 and p40 promoters, and wherein ATG or GTG codons at one or more positions corresponding to positions (a) 321-323, (b) 766-768, (c) 955-957, (d) 993-995 and (e) 1014-1016 of SEQ ID NO: 1 are absent or mutated.
74. The two-plasmid system of aspect 73, wherein in the promoter region:
   (a) nucleotides corresponding to nucleotides 321-323 of SEQ ID NO: 1 are absent;
   (b) nucleotides corresponding to nucleotides 766-768 of SEQ ID NO: 1 are not ATG and are optionally ATT;
   (c) nucleotides corresponding to nucleotides 955-957 of SEQ ID NO: 1 are absent;
   (d) nucleotides corresponding to nucleotides 993-995 of SEQ ID NO: 1 are absent; and/or
   (e) nucleotides corresponding to nucleotides 1014-1016 of SEQ ID NO: 1 are absent.
75. The two-plasmid system of any one of aspects 52 to 74, wherein the vector plasmid comprises a backbone less than 4000 nucleotides, less than 3500 nucleotides, less than 3000 nucleotides, or less than 2500 nucleotides in length.
76. The two-plasmid system of any one of aspects 52 to 75, wherein the vector plasmid does not comprise any spacers.
77. A host cell comprising the helper plasmid or two-plasmid system of any one of the preceding aspects.
78. The host cell of aspect 77, wherein said cell is cultured in suspension,
79. Use of the helper plasmid or the two-plasmid system of any one of aspects 1 to 76 for producing a recombinant AAV preparation in suspension cells.
80. A method for producing a recombinant AAV preparation comprising:
   (a) obtaining the helper plasmid or the two-plasmid system of any one of aspects 1 to 76;
   (b) transfecting a host cell with the helper plasmid or the two-plasmid system of any one of aspects 1 to 76; and
   (c) culturing the host cell in suspension under conditions suitable for recombinant AAV production.
81. Use of the helper plasmid or the two-plasmid system of any one of aspects 1 to 76 for:
   (i) increasing, optimising and/or maximising the yield of recombinant AAV produced during recombinant AAV production in suspension cells;
   (ii) increasing the yield of recombinant AAV produced during recombinant AAV production in suspension cells compared to an equivalent use of a rep 78 negative helper plasmid or a two-plasmid system comprising a rep 78 negative helper plasmid;
   (iii) controlling and/or maximising the ratio of full to total particles produced during recombinant AAV production;
   (iv) reducing and/or minimising the level of nucleic acid impurities (normalised to vector genome level) produced during recombinant AAV production in suspension cells;
   (v) reducing the level of nucleic acid impurities (normalised to vector genome level) produced during recombinant AAV production in suspension cells compared to an equivalent use of a rep 78 negative helper plasmid or a two-plasmid system comprising a rep 78 negative helper plasmid;
   (vi) increasing the potency of recombinant AAV produced during recombinant AAV production in suspension cells; and/or
   (vii) increasing the potency of recombinant AAV produced during recombinant AAV production in suspension cells compared to an equivalent use of a rep 78 negative helper plasmid or a two-plasmid system comprising a rep 78 negative helper plasmid.
82. A method for:
   (i) increasing, optimising and/or maximising the yield of recombinant AAV produced during recombinant AAV production in suspension cells;
   (ii) increasing the yield of recombinant AAV produced during recombinant AAV production in suspension cells compared to an equivalent method using a two-plasmid system comprising a rep 78 negative helper plasmid or a rep 78 negative helper plasmid;
   (iii) controlling and/or maximising the ratio of full to total particles produced during recombinant AAV production in suspension cells;
   (iv) reducing and/or minimising the level of nucleic acid impurities (normalised to vector genome level) produced during recombinant AAV production in suspension cells;
   (v) reducing the level of nucleic acid impurities (normalised to vector genome level) produced during recombinant AAV production in suspension cells compared to an equivalent method using a rep 78 negative helper plasmid or a two-plasmid system comprising a rep 78 negative helper plasmid;
   (vi) increasing the potency of recombinant AAV produced during recombinant AAV production in suspension cells; and/or
   (vii) increasing the potency of recombinant AAV produced during recombinant AAV production in suspension cells compared to an equivalent method of a two-plasmid system comprising a a rep 78 negative helper plasmid or rep 78 negative helper plasmid,
   comprising:
   a) obtaining the helper plasmid or the two-plasmid system of any one of aspects 1 to 76;
   (b) transfecting a host cell with the helper plasmid or the two-plasmid system of any one of aspects 1 to 76; and
   (c) culturing the host cell in suspension under conditions suitable for recombinant AAV production.
83. The use of aspect 79 or 81, wherein the use comprises transfecting a host cell with the two-plasmid system or helper plasmid of any one of aspects 1 to 76 and culturing the host cell in suspension under conditions suitable for recombinant AAV production.
84. The use or method of any one of aspects 79 to 83, wherein the use or method is a use or method for producing a recombinant AAV preparation in suspension at a high or desired yield.
85. The use or method of any one of aspects 79 to 84, wherein the use or method is a use or method for producing a recombinant AAV preparation at a higher yield than the yield obtained using an equivalent method or use using a a rep 78 negative helper plasmid or a two-plasmid system comprising a rep 78 negative helper plasmid.
86. The use or method of aspect 85, wherein the higher yield is at least 1.03, at least 1.04, at least 1.05, at least 1.1 fold, at least 1.2 fold, at least 1.25 fold, at least 1.5 fold, at least 1.6 fold, at least 1.7 fold, at least 2 fold, between 1.1 fold and 5 fold, between 1.2 fold and 3 fold, or between 1.2 fold and 2.5 fold higher than the yield obtained using an equivalent method or use using a rep 78 negative helper plasmid or a two-plasmid system comprising a rep 78 negative helper plasmid.
87. The use or method of any one of aspects 79 to 86, wherein the use or method is a use or method for increasing, optimising and/or maximising the yield of recombinant AAV produced during recombinant AAV production.
88. The use or method of any one of aspects 79 to 87, wherein the use or method is a use or method for increasing the yield of recombinant AAV produced during recombinant AAV production compared to an equivalent use of a rep 78 negative helper plasmid or a two-plasmid system comprising a rep 78 negative helper plasmid.
89. The use or method of aspect 88, wherein the use or method is a use or method for increasing the yield of recombinant AAV produced during recombinant AAV production compared to an equivalent use of a two-plasmid system comprising a rep 78 negative helper plasmid by at least 1.03, at least 1.04, at least 1.05, at least 1.1 fold, at least 1.2 fold, at least 1.25 fold, at least 1.5 fold, at least 1.6 fold, at least 1.7 fold, at least 2 fold, between 1.1 fold and 5 fold, between 1.2 fold and 3 fold, or between 1.2 fold and 2.5 fold.
90. The use or method of any one of aspects 79 to 89, comprising a step of harvesting the recombinant AAV to provide a recombinant AAV preparation comprising a high or desired yield of recombinant AAV.
91. The use or method of any one of aspects 79 to 90, comprising a step of harvesting the recombinant AAV to provide a recombinant AAV preparation comprising a yield of recombinant AAV that is higher than the yield obtained using an equivalent method or use using a rep 78 negative helper plasmid or atwo-plasmid system comprising a rep 78 negative helper plasmid.
92. The use or method of aspect 91, wherein the recombinant AAV preparation comprises a yield of recombinant AAV that is at least 1.03, at least 1.04, at least 1.05, at least 1.1 fold, at least 1.2 fold, at least 1.25 fold, at least 1.5 fold, at least 1.6 fold, at least 1.7 fold, at least 2 fold, between 1.1 fold and 5 fold, between 1.2 fold and 3 fold, or between 1.2 fold and 2.5 fold higher than the yield of recombinant AAV obtained using an equivalent method or use using a rep 78 negative helper plasmid or a two-plasmid system comprising a rep 78 negative helper plasmid.
93. The use or method of any one of aspects 79 to 92, wherein the use or method is a use or method for producing a recombinant AAV preparation with a low level of nucleic acid impurities (normalised to vector genome level).
94. The use or method of any one of aspects 79 to 93, wherein the use or method is a use or method for producing a recombinant AAV preparation with a lower level of nucleic acid impurities (normalised to vector genome level) than the level of nucleic acid impurities obtained using an equivalent method or use using a rep 78 negative helper plasmid or a two-plasmid system comprising a rep 78 negative helper plasmid.
95. The use or method of aspect 93 or 94, wherein the low or lower level of nucleic acid impurities is a low or lower level of vector plasmid impurities.
96. The use or method of aspect 95, wherein the low or lower level of vector plasmid impurities is a low or lower level of cap gene and/or vector plasmid backbone impurities.
97. The use or method of aspect 96, wherein the low or lower level of cap gene impurities is a level of cap gene impurities that is less than 0.8 fold, less than 0.7 fold, less than 0.65 fold, less than 0.55 fold, less than 0.5 fold, less than 0.25 fold, less than 0.2 fold, between 0.1 fold and 0.8 old, between 0.15 fold and 0.7 fold, between 0.15 fold and 0.65 fold, between 0.15 fold and 0.55 fold, between 0.15 fold and 0.5 fold, or between 0.15 fold and 0.2 fold of the level of cap gene impurities produced using an equivalent method or use using a two-plasmid system comprising a rep 78 negative helper gene.
98. The use or method of any one of aspects 93 to 97 wherein the level of vector plasmid impurities or cap gene impurities is determined by qPCR or ddPCR using primers that are complementary to a portion of the cap gene.
99. The use or method of aspect 98, wherein the low or lower level of vector plasmid backbone impurities is a level of vector plasmid backbone impurities that is less than 0.6 fold, less than 0.5 fold, less than 0.45 fold, less than 0.4 fold, less than 0.35 fold, less than 0.2 fold, less than 0.15 fold, between 0 fold and 0.6 fold, between 0.05 fold and 0.5 fold, between 0.1 fold and 0.45 fold, between 0.1 fold and 0.4 fold, between 0.1 fold and 0.35 fold, between 0.1 fold and 0.2 fold, or between 0.1 fold and 0.15 fold of the level of vector plasmid backbone impurities obtained using an equivalent method or use using a two-plasmid system comprising a rep 78 negative helper gene.
100. The use or method of aspect 93 to 99, wherein the level of vector plasmid impurities or vector plasmid backbone impurities is determined by qPCR or ddPCR using primers that are complementary to a portion of the vector plasmid backbone, optionally an antibiotic resistance gene such as kanamycin R.
101. The use or method of aspect 99 or 100, wherein the low or lower level of nucleic acid impurities comprises a low or lower level of plasmid backbone impurities.
102. The use of method of aspect 99, 100, or 101, wherein the low or lower level of nucleic acid impurities comprises a low or lower level of helper plasmid impurities.
103. The use or method of aspect 101 or 102, wherein the low or lower level of plasmid backbone impurities and/or helper plasmid impurities comprises a level of plasmid backbone impurities and/or helper plasmid impurities that is less than 0.6 fold, less than 0.5 fold, less than 0.45 fold, less than 0.4 fold, less than 0.35 fold, less than 0.2 fold, less than 0.15 fold, between 0 fold and 0.6 fold, between 0.05 fold and 0.5 fold, between 0.1 fold and 0.45 fold, between 0.1 fold and 0.4 fold, between 0.1 fold and 0.35 fold, between 0.1 fold and 0.2 fold, or between 0.1 fold and 0.15 fold of the level of plasmid backbone impurities and/or helper plasmid impurities obtained using an equivalent method or use using a rep 78 negative helper plasmid or a two-plasmid system comprising a rep 78 negative helper gene.
104. The use or method of any one of aspects 101 to 103, wherein the level of plasmid backbone impurities or helper plasmid impurities is determined by qPCR or ddPCR using primers that are complementary to a portion of the helper plasmid backbone, optionally an antibiotic resistance gene such as kanamycin R.
105. The use or method of any one of aspects 93 to 104, wherein the low or lower level of nucleic acid impurities comprises a low or lower level of host cell derived impurities.
106. The use or method of aspect 105, wherein the low or lower level of host cell derived impurities comprises a low or lower level of 18S rRNA gene impurities.
107. The use or method of aspect 105 or 106, wherein the level of host cell-derived or 18S rRNA gene impurities is determined by ddPCR using primers and a probe that are complementary to a portion of the host cell genome, optionally an 18S rRNA gene.
108. The use or method of any one of aspects 79 to 107, wherein the use or method is a use or method for producing a recombinant AAV preparation comprising recombinant AAV having high potency.
109. The use or method of any one of aspects 79 to 108, wherein the use or method is a use or method for producing a recombinant AAV preparation comprising recombinant AAV having higher potency compared to the potency of recombinant AAV obtained using an equivalent method or use using a two-plasmid system comprising a rep 78 negative helper plasmid.
110. The use or method of aspect 109, wherein the higher potency is at least 1.03, at least 1.04, at least 1.05, at least 1.1 fold, at least 1.15 fold, between 1.1 fold and 5 fold, or between 1.15 fold and 3 fold higher than the potency of recombinant AAV obtained using an equivalent method or use using a rep 78 negative helper plasmid or a two-plasmid system comprising a rep 78 negative helper plasmid.
111. The use or method of any one of aspects 79 to 1101, wherein the use or method is a use or method for increasing the potency of recombinant AAV produced during recombinant AAV production.
112. The use or method of any one of aspects 79 to 111, wherein the use or method is a use or method for increasing the potency of recombinant AAV produced during recombinant AAV production compared to an equivalent use of a rep 78 negative helper plasmid or a two-plasmid system comprising a rep 78 negative helper plasmid.
113. The use or method of any one of aspects 79 to 112, wherein the use or method is a use or method for increasing the potency of recombinant AAV produced during recombinant AAV production compared to an equivalent use of a a rep 78 negative helper plasmid or a two-plasmid system comprising a rep 78 negative helper plasmid by at least 1.03, at least 1.04, at least 1.05, at least 1.1 fold, at least 1.15 fold, between 1.1 fold and 5 fold, or between 1.15 fold and 3 fold.
114. The use or method of any one of aspects 79 to 113, comprising a step of harvesting the recombinant AAV to provide a recombinant AAV preparation comprising high potency recombinant AAV.
115. The use or method of any one of aspects 79 to 114, comprising a step of harvesting the recombinant AAV to provide a recombinant AAV preparation comprising recombinant AAV that has higher potency than the recombinant AAV obtained using an equivalent method or use using a rep 78 negative helper plasmid or a two-plasmid system comprising a rep 78 negative helper plasmid.
116. The use or method of aspect 115, wherein the recombinant AAV preparation comprises recombinant AAV having a potency at least 1.03, at least 1.04, at least 1.05, at least 1.1 fold, at least 1.15 fold, between 1.1 fold and 5 fold, or between 1.15 fold and 3 fold higher than the potency of recombinant AAV obtained using an equivalent method or use using a a rep 78 negative helper plasmid or two-plasmid system comprising a rep 78 negative helper plasmid.
117. The use or method of any one of aspects 79 to 1167, wherein the ratio of helper plasmid to vector plasmid is adjusted to:
   (i) increase, optimise and/or maximise the yield of recombinant AAV produced during recombinant AAV production;
   (ii) control and/or maximise the ratio of full to total particles produced during recombinant AAV production;
   (iii) obtain the high or desired yield;
   (iv) obtain a desired ratio of full to total particles; and/or
   (v) achieve a balanced yield versus full to total particle ratio.
118. The use or method of any one of aspects 79 to 117, comprising a step of selecting a ratio of helper plasmid to vector plasmid.
119. The use or method of aspect 118, wherein the step of selecting a ratio of helper plasmid to vector plasmid is carried out in order to:
   (i) increase, optimise and/or maximise the yield of recombinant AAV produced during recombinant AAV production;
   (ii) control and/or maximise the ratio of full to total particles produced during recombinant AAV production;
   (iii) obtain the high or desired yield;
   (iv) obtain a desired ratio of full to total particles; and/or
   (v) achieve a balanced yield versus full to total particle ratio.
120. The use or method of any one of aspects 79 to 119 wherein the use or method is a use or method for producing a recombinant AAV preparation having a desired ratio of full to total particles.
121. The use or method of any one of aspects 79 to 120, comprising a step of harvesting the recombinant AAV to provide a recombinant AAV preparation comprising a desired ratio of full to total particles.
122. The use or method of any one of aspects 117 to 121, wherein the desired ratio of full to total particles is at least 2.5%, at least 5%, at least 7.5%, at least 8%, between 2.5% and 10%, between 5% and 9%, between 7.5% and 9%, or between 8% and 9%.
123. The use or method of any one of aspects 117to 123, wherein the balanced yield versus full to total particle ratio is a ratio that achieves a maximum yield of recombinant AAV with the minimum yield of empty particles achievable at such maximum yield of recombinant AAV.
124. The use or method of any one of aspects 79 to 123, wherein the ratio of helper plasmid to vector plasmid comprises a molar excess of helper plasmid.
125. The use or method of any one of aspects 79 to 124, wherein the ratio of helper plasmid to vector plasmid is between 1:10 to 10:1, between 1:3 and 7:3, between 1:3 and 6:3, preferably around 1:3 or around 2:3 or around 3:3 or around 4:3 or around 5:3, or around 6:3.
126. The use or method of any one of aspects 79 to 125, wherein the ratio of helper plasmid to vector plasmid is adjusted to and/or the ratio of helper plasmid to vector plasmid is selected to be between 1:10 to 10:1, between 1:3 and 7:3, between 1:3 and 6:3, preferably around 1:3 or around 2:3 or around 3:3 or around 4:3 or around 5:3 , or around 6:3.
127. The use or method of any one of aspects 79 to 126, wherein the high or desired yield is a yield that is at least at least 1.05 fold, at least 1.1 fold, 1.2 fold, at least 1.3 fold, at least 1.4 fold, at least 1.6 fold, at least 1.8 fold, or at least 2 fold higher than the yield achieved using an equivalent method or use with a ratio of helper plasmid to vector plasmid of 1:3.
128. The use or method of any one of aspects 79 to 127, wherein the desired ratio of full to total particles is a ratio of full to total particles that is at least at least 1.05 fold, at least 1.1 fold, 1.2 fold, at least 1.3 fold, at least 1.4 fold, at least 1.6 fold, at least 1.8 fold, at least 2 fold, at least 3 fold, or at least 3.5 fold higher than the ratio of full to total particles achieved using an equivalent method with a ratio of helper plasmid to vector plasmid of 1:3.
129. The use or method of any one of aspects 79 to 128, wherein the ratio of full to total particles is measured by calculating the number of vector genomes using qPCR or ddPCR to quantify the number of nucleic acid sequences comprising a cassette comprising a promoter sequence (vector genomes; vg); measuring the total number of particles using an immunoassay preferably a capsid-specific ELISA or a sandwich immunometric assay; and calculating the ratio using the formula vg/ml ÷ particles/ml × 100.
130. The use or method of any one of aspects 79 to 129, wherein the yield is determined by using qPCR or ddPCR to quantify the number of nucleic acid sequences comprising a cassette comprising a promoter sequence (vg).
131. The use or method of any one of aspects 79 to 130, wherein the capsid yield is determined by measuring the total number of particles using an immunoassay preferably a capsid-specific ELISA or a sandwich immunometric assay
132. A method for reducing and/or minimising the level of replication competent AAV (rcAAV) produced during recombinant AAV production comprising:
   (d) obtaining the two-plasmid system, or the helper plasmid as defined in any one of aspects 1-76;
   (e) transfecting a host cell with the two-plasmid system, or the helper plasmid as defined in any one of aspects 1-76; and
   (f) culturing the host cell in suspension under conditions suitable for recombinant AAV production.
133. A method for reducing and/or minimising the level of pseudo-wild type replication competent AAV (rcAAV) produced during recombinant AAV production comprising:
   (d) obtaining the two-plasmid system, or the helper plasmid as defined in any one of aspects 1-76;
   (e) transfecting a host cell with the two-plasmid system, or the helper plasmid as defined in any one of aspects 1-76; and
   (f) culturing the host cell in suspension under conditions suitable for recombinant AAV production.
134. A method for producing recombinant AAV comprising a reduced and/or minimised level of replication competent AAV (rcAAV) or pseudo-wild type replication competent AAV (rcAAV).
135. The method of aspect 1334or 134, wherein an excess of rep-rcAAV or cap-rcAAV indicates that the level of pseudo wild-type rcAAV is reduced or minimised.
136. The method of aspect 135, wherein the excess is at least 5 times, at least 10 times, at least 25 times, at least 30 times, or at least 35 times excess of rep-rcAAV or cap-rcAAV.
137. The method of aspect 135 or 136, wherein the excess is greater than the excess produced using a two-plasmid system comprising a plasmid comprising both at least one rep gene and at least one cap gene or a rep 78 negative two-plasmid system.
138. The method of any one of aspects 135 to 137, wherein the level of rep-rcAAV is the level of rep-rcAAV detected by qPCR using primers binding to rep68 exon 1.
139. The method of any one of aspects 135 to 139, wherein the level of rep-rcAAV is the level of rep-rcAAV detected by qPCR using the forward primer CACGTGCATGTGGAAGTAG (SEQ ID NO: 10) and the reverse primer CGACTTTCTGACGGAATGG (SEQ ID NO: 11).
140. The method of any one of aspects 135 to 129, wherein the level of cap-rcAAV is the level of cap-rcAAV detected by qPCR using primers binding to a sequence encoding VP3.
141. The method of any one of aspects 135 to 140, wherein the level of cap-rcAAV is the level of cap-rcAAV detected by qPCR using the forward primer TACTGAGGGACCATGAAGAC (SEQ ID NO: 12) and the reverse primer GTTTACGGACTCGGAGTATC (SEQ ID NO: 13).
142. The method of any one of aspects 135 to 141, further comprising a step of harvesting the recombinant AAV to provide a recombinant AAV preparation.
143. The method of aspect 142, wherein the recombinant AAV preparation comprises a reduced or minimised level of rcAAV.
144. The method of any one of aspects 135 to 143, wherein the reduced or minimised level of rcAAV comprises a low level of rep-rcAAV.
145. The method of any one of aspects 135 to 144, wherein the reduced or minimised level of rcAAV comprises a lower level of rep-rcAAV compared to the level of rep-rcAAV produced using a two-plasmid system comprising a plasmid comprising both at least one rep gene and at least one cap gene or a rep 78 negative two-plasmid system.
146. The method of any one of aspects 135 to 145, wherein the reduced or minimised level of rcAAV comprises an at least 5 times, at least 10 times, at least 25 times, at least 30 times, or at least 35 times excess of rep-rcAAV or cap-rcAAV.
147. The method of any one of aspects 135 to 146, wherein the reduced or minimised level of rcAAV comprises a proportion of less than 1/5 rep-rcAAV, less than 1/10 rep-rcAAV, less than 1/25 rep-rcAAV, less than 1/30 rep-rcAAV, or less than 1/35 rep-rcAAV.
148. The method of any one of aspects 135 to 147, wherein the reduced or minimised level of rcAAV comprises a proportion of rep-rcAAV that is less than the proportion produced using a two-plasmid system comprising a plasmid comprising both at least one rep gene and at least one cap gene or a rep 78 negative two-plasmid system.
149. The method of aspect 148, wherein the proportion of rep-rcAAV is less than 90%, less than 75%, less than 60%, less than 50%, less than 25%, less than 20%, less than 17%, or less than 15% of the proportion produced using the two-plasmid system comprising a plasmid comprising both at least one rep gene and at least one cap gene or a rep 78 negative two-plasmid system.
150. The method of any one of aspects 135 to 149, wherein the reduced or minimised level of rcAAV comprises a low level of pseudo-wild type rcAAV.
151. The method of any one of aspects 135 to 150, wherein the reduced or minimised level of rcAAV comprises less than 1/5 pseudo-wild type rcAAV, less than 1/10 pseudo-wild type rcAAV, less than 1/25 pseudo-wild type rcAAV, less than 1/30 pseudo-wild type rcAAV, or less than 1/35 pseudo-wild type rcAAV.
152. The method of any one of aspects 137 to 151, wherein the level of rep-rcAAV is the level of rep-rcAAV detected by qPCR using primers binding to rep68 exon 1.
153. The method of any one of aspects 137 to 152, wherein the level of rep-rcAAV is the level of rep-rcAAV detected by qPCR using the forward primer CACGTGCATGTGGAAGTAG (SEQ ID NO: 10) and the reverse primer CGACTTTCTGACGGAATGG (SEQ ID NO: 11).
154. The method of any one of aspects 137 to 153, wherein the level of cap-rcAAV is the level of cap-rcAAV detected by qPCR using primers binding to a sequence encoding VP3.
155. The method of any one of aspects 137 to 154, wherein the level of cap-rcAAV is the level of cap-rcAAV detected by qPCR using the forward primer TACTGAGGGACCATGAAGAC (SEQ ID NO: 12) and the reverse primer GTTTACGGACTCGGAGTATC (SEQ ID NO: 13).
156. The method of any one of aspects 137 to 155, wherein the level of rcAAV is measured to be less than 1 rcAAV in 10⁷ recombinant AAV, less than 1 rcAAV in 10⁹ recombinant AAV, or less than 1 rcAAV in 10¹⁰ recombinant AAV.
157. The method of any one of aspects 137 to 156, wherein the level of rcAAV is less than the level of rcAAV produced using an equivalent method except that the vector plasmid comprises both at least one rep gene and at least one cap gene or a rep 78 negative two-plasmid system.
158. The method of any one of aspects 137 to 157, wherein the level of rcAAV is measured by using qPCR to measure the number of recombinant AAV particles that comprise a rep gene.
159. The use or method of any one of aspects 79 to 158, wherein the host cell is selected from the group consisting of a HEK293T cell, a HEK293 cell, a HEK293EBNA cell, a CAP cell, a CAP-T cell, an AGE1.CR cell, a PerC6 cell, a C139 cell, and an EB66 cell.
160. The use or method of any one of aspects 79 to 159, wherein culturing the host cell in suspension under conditions suitable for recombinant AAV production does not comprise culturing the host cell using a suspension system.
161. The use or method of any one of aspects 79 to 160, wherein the host cell is a cell that expresses a functional E1A/B protein.
162. The use or method of any one of aspects 79 to 161, further comprising a step of purifying the recombinant AAV.
163. The use or method of aspect 162, wherein the step of purifying the recombinant AAV comprises using a technique selected from the group consisting of gradient density centrifugation (such as CsCI or lodixanol gradient density centrifugation), filtration, ion exchange chromatography, size exclusion chromatography, affinity chromatography and hydrophobic interaction chromatography.
164. The use or method of any one of aspects 79 to 163, comprising further concentrating the recombinant AAV using ultracentrifugation, tangential flow filtration, or gel filtration.
165. The use or method of any one of aspects 79 to 164, comprising formulating the recombinant AAV with a pharmaceutically acceptable excipient.
166. A recombinant AAV preparation obtainable by the method of any one of aspects 79 to 165.
167. A recombinant AAV preparation obtained by the method of any one of aspects 79 to 165

The following examples illustrate the invention.

### Examples

### Example 1: Construction of helper plasmid of Rep78neq two-plasmid system

### Rep78neg helper plasmid

In order to construct the Rep78neg helper plasmid, nucleotides 200-4497 of wild type AAV2 (Genbank accession number AF043303; SEQ ID NO: 1) containing the rep and cap genes were cloned into the pUC19 (Yanisch-Perron et al (1985), Gene, 33:103-119). Next, AAV2 nucleotides 4461-4497 were deleted to minimise sequence homology with the vector plasmid (the construction of which is described below). To prevent Rep 78 expression while maintaining Rep 68 expression, the intron within the cloned rep gene was deleted. In order to provide for expression of Rep 52 following deletion of the intron, AAV2 nucleotides corresponding to rep 52 including the p19 promoter were cloned immediately 3' of the intron-less rep 68 gene. The majority of cap gene sequences were then deleted.

The two p40 promoters (one in each of the Rep 68- and Rep 52-encoding rep gene duplications) were rendered non-functional by ablation of the TATA boxes (mutation of the T corresponding to AAV2 position 1823 and AAG corresponding to AAV2 positions 1826-1828 to C and CTC respectively).

The resulting 'rep cassette' was then cloned into a plasmid comprising an 8342-nucleotide stretch comprising functional VA RNA I and II, E2A and E4 genes from adenovirus (i.e. helper virus) serotype 5 (SEQ ID NO: 4). The plasmid backbone, containing kanamycin resistance gene and bacterial origin of replication, was about 2.2kb in length, resulting in a helper plasmid of 14021 nucleotides.

Figure 8 is a schematic of the Rep78neg helper plasmid showing the main features. For the 'rep cassette' the portions of AAV2 sequence, by reference to the nucleotide positions of SEQ ID NO: 1, are indicated.

### Example 2: Construction of vector plasmid of the Rep78neq two-plasmid system

In order to construct the vector plasmid, nucleotides 200-4497 of wild type AAV2 (Genbank accession number AF043303; SEQ ID NO: 1) containing the rep and cap genes were cloned into pUC19. Two portions of rep gene sequence, between the p5 and p19 and between the p19 and p40 promoters respectively, were then deleted to prevent Rep protein expression whilst maintaining the downstream cap gene under the regulation of the three native promoters. As for the helper plasmid described above, AAV2 nucleotides 4461-4497 were deleted to minimise sequence homology with the helper plasmid.

To minimise or prevent translation of undesired products from potential initiation codons within the remaining promoter region, four ATG codons and one GTG codon were removed: ATGs at positions corresponding to AAV2 nucleotides 321-323, 955-957 and 993-995, and a GTG corresponding to AAV2 nucleotides 1014-1016, were deleted, whilst ATG at AAV2 nucleotides 766-768 was mutated to ATT.

The AAV2 cap gene encoding the VPs 1, 2 and 3 immediately 3' of the above promoter region was replaced with the corresponding sequence of an engineered cap gene, which cap gene is 3 nucleotides (equating to one additional encoded amino acid) longer than the AAV2 cap gene. The resulting 'promoter-cap' cassette was cloned into a plasmid backbone containing kanamycin resistance gene and bacterial origin of replication. Into this backbone was inserted an expression cassette, containing the transgene sequence linked to promoter and polyA transcription regulatory elements, flanked by AAV2 sequence comprising the native AAV2 ITRs (AAV2 nucleotides 1-145 and 4535-4679).

In the case of a vector plasmid comprising a 2672-nucleotide (ITR-to-ITR) Factor IX expression cassette, as used in Example 5, the plasmid backbone was approximately 2.3kb in length, resulting in an 8268-nucleotide vector plasmid. In the case of a vector plasmid comprising a 2297-nucleotide (ITR-to-ITR) alpha-Galactosidase A expression cassette, as used in Example 5, the plasmid backbone was approximately 2.3kb in length, resulting in a 7893-nucleotide vector plasmid. In the case of a vector plasmid comprising a 3046-nucleotide (ITR-to-ITR) beta-Glucocerebrosidase expression cassette, as used in Example 5, the plasmid backbone was approximately 2.3kb in length, resulting in an 8642-nucleotide vector plasmid. In the case of a vector plasmid comprising a 4713-nucleotide (ITR-to-ITR) Factor VIII expression cassette, as used in Examples 5 and 7, the plasmid backbone was approximately 2.3kb in length, resulting in a 10309-nucleotide vector plasmid. In the case of a vector plasmid comprising a 2672-nucleotide (ITR-to-ITR) Factor IX expression cassette, as used in Example 6, the plasmid backbone was approximately 2.6kb in length, resulting in an 8525-nucleotide vector plasmid. Figure 9 is a schematic of the vector plasmid showing the main features. For the 'promoter-cap' cassette, the portions of AAV2 sequence, by reference to the nucleotide positions of SEQ ID NO: 1, are indicated.

### Example 3: Construction of helper plasmid of two plasmid system comprising an entire Rep gene under the control of an ATG start codon (Rep78pos two plasmid system)

### Rep78pos helper plasmid

In order to construct the Rep78pos helper plasmid comprising an entire rep gene under the control of the native start codon (ATG), nucleotides 200-4460 of wild type AAV2 (Genbank accession number AF043303; SEQ ID NO: 1) containing the rep and cap genes were cloned into pUC19 (Yanisch-Perron et al (1985), Gene, 33:103-119). Next, AAV2 nucleotides 2301-4410 were deleted to remove the majority of the cap gene sequences. Rep78/Rep68 expression is driven by the native ATG start codon in the rep gene.

The p40 promoter (contained within the rep gene) was rendered non-functional by ablation of the TATA box (mutation of the T corresponding to AAV2 position 1823 and AAG corresponding to AAV2 positions 1826-1828 to C and CTC respectively).

The resulting 'rep cassette' was then cloned into a plasmid comprising an 8342-nucleotide stretch comprising functional VA RNA I and II, E2A and E4 genes from adenovirus (i.e. helper virus) serotype 5 (SEQ ID NO:4). The plasmid backbone, containing kanamycin resistance gene and bacterial origin of replication, was about 2.2kb in length, resulting in a helper plasmid of 12668 nucleotides.

Figure 10 is a schematic of the Rep78pos helper plasmid comprising an entire rep gene under the control of the native start codon ATG showing the main features. For the 'rep cassette' the portions of AAV2 sequence, by reference to the nucleotide positions of SEQ ID NO: 1, are indicated.

### Example 4: Construction of AAV expression cassette/vector plasmid of the Rep78pos two-plasmid system

In order to construct the AAV expression cassette/capsid plasmid, nucleotides 200-4497 of wild type AAV2 (Genbank accession number AF043303; SEQ ID NO: 1) containing the rep and cap genes were cloned into pUC19. Two portions of rep gene sequence, between the p5 and p19 and between the p19 and p40 promoters respectively, were then deleted to prevent Rep protein expression whilst maintaining the downstream cap gene under the regulation of the three native promoters. As for the helper plasmid described above, AAV2 nucleotides 4461-4497 were deleted to minimise sequence homology with the different helper plasmids.

To minimise or prevent translation of undesired products from potential initiation codons within the remaining promoter region, four ATG codons and one GTG codon were removed: ATGs at positions corresponding to AAV2 nucleotides 321-323, 955-957 and 993-995, and a GTG corresponding to AAV2 nucleotides 1014-1016, were deleted, whilst ATG at AAV2 nucleotides 766-768 was mutated to ATT.

The AAV2 cap gene encoding the VPs 1, 2 and 3 immediately 3' of the above promoter region was replaced with the corresponding sequence of the AAV9 cap gene (Genbank accession number AY530579), which cap gene is 3 nucleotides (equating to one additional encoded amino acid) longer than the AAV2 cap gene. The resulting 'promoter-cap' cassette was cloned into a plasmid backbone of approximately 2.1kb in length containing kanamycin resistance gene and bacterial origin of replication. Into this backbone was inserted a 2985-nucleotide (ITR-to-ITR) AAV expression cassette, containing a secreted alkaline phosphatase (SEAP) transgene sequence linked to enhancer, promoter, intron and polyA regulatory elements, flanked by AAV2 sequence comprising the native AAV2 ITRs (AAV2 nucleotides 1-145 and 4535-4679). Overall, this resulted in an 8581-nucleotide AAV expression cassette/capsid plasmid.

### Example 5: General methods

### Cell Cultivation

HEK293 cells were maintained in suspension culture at 37 °C, 5% CO₂, and 120 rpm in BalanCD HEK293 medium supplemented with 4 mM L-glutamine. Cells were passaged every 3-4 days with a seeding density of 1.0 × 10⁶ or 0.5 × 10⁶ viable cells/mL.

### Transfection of HEK293 suspension cells in an AMBR^{®}15 bioreactor system and preparation of cell lysates

HEK293 suspension cells in BalanCD HEK293 medium supplemented with 4 mM L-glutamine from a 1-day culture were seeded with a seeding density of 2.5 × 10⁶ viable cells/mL (NudeoCounter^{®} NC-202^{™}, Chemometec) in a total volume of 12 mL. Transient transfections of HEK293 suspension cells were performed 2h after inoculation of the Ambr^{®}15 bioreactor vessels.

HEK293 suspension cells were transfected with a molar ratio between the respective DNA molecules of the split 2-plasmid system in the range of 1:3 to 6:3 (rep/AdV helper: AAV expression cassette/capsid plasmid). A DNA concentration of 0.52 or 1.0 pg/vc and a PElpro^{®}:DNA ratio of 2:1 or 1:1 were applied. DNA and PElpro^{®} were diluted separately in fresh, supplemented BalanCD HEK293 medium. The PElpro^{®}-mix was added to the DNA-mix and incubated for 20 ± 2 min at room temperature. The transfection mix was added per bioreactor vessel with a final volume of 5 or 10 % of the total volume.

Cells were cultured until day 3 post transfection to allow for rAAV production, harvested in the medium and lysed by three freeze-thaw cycles at -80°C and 37°C, respectively. Cell debris was removed by centrifugation for 30 min at 3700 x g and 4 °C or 10 min at 4700 x g and room temperature before transferring the supernatants to new vials. The clarified freeze-thaw lysates were subjected to one-step affinity purification (following nuclease treatment to remove non-packed vector genomes) or used directly for subsequent analytical procedures.

### Transfection of HEK293 suspension cells in a Biostat^{®} STR 50 L bioreactor system and preparation of cell lysates

HEK293 suspension cells in BalanCD HEK293 medium supplemented with 4 mM L-glutamine were seeded with a seeding density of 7.0 × 10⁵ viable cells/mL (Vi-Cell Blu cell counter, Beckman-Coulter). The growth phase of the cells in the controlled environment in the 50 L bioreactor lasts for 24 h starting from the inoculation. HEK293 suspension cells were transfected with a molar ratio between the respective DNA molecules of the split 2-plasmid system of 4:3 (rep/AdV helper: AAV expression cassette/capsid plasmid). A DNA concentration of 0.83 pg/vc and a PElpro^{®}:DNA ratio of 2:1 were applied. DNA and PEIpro^{®} were diluted separately in fresh, supplemented BalanCD HEK293 medium. The PEIpro^{®}-mix was added to the DNA-mix and incubated for 20 ± 2 min at room temperature. The transfection mix was added to the bioreactor vessel with a final volume of 5 % of the total volume. The rAAV production phase lasted 72 h starting from transfection. A glucose addition back to 4 g/L was performed 48 h post transfection. Harvest was performed in the medium and cells were lysed using the CF2 Cell Disruptor ("microfluidizer", Constant Systems Ltd., Daventry, England).

### Transfection of HEK293 suspension cells in a Biostat^{®} STR 200 L bioreactor system and preparation of cell lysates

HEK293 suspension cells in BalanCD HEK293 medium supplemented with 4 mM L-glutamine were seeded with a seeding density of 7.0 × 10⁵ viable cells/mL (Vi-Cell Blu cell counter, Beckman-Coulter). The growth phase of the cells in the controlled environment in the 200 L bioreactor lasts for 24 h starting from the inoculation with an expected cell count between 1.1 - 1.5 x 10⁶ vc/mL at the time point of transfection. HEK293 suspension cells were transfected with a molar ratio between the respective DNA molecules of the split 2-plasmid system of 4:3 (rep/AdV helper: AAV expression cassette/capsid plasmid). A DNA concentration of 0.83 pg/vc and a PEtpro^{®}:DNA ratio of 2:1 were applied. DNA and PElpro^{®} were diluted separately in fresh, supplemented BalanCD HEK293 medium. The PElpro^{®}-mix was added to the DNA-mix and incubated for 20 ± 2 min at room temperature. The transfection mix was added to the bioreactor vessel with a final volume of 5 % of the total volume. The rAAV production phase lasted 72 h starting from transfection. A glucose addition back to 4 g/L was performed 48 h post transfection. Harvest was performed in the medium and cells were lysed using the CF2 Cell Disruptor ("microfluidizer", Constant Systems Ltd., Daventry, England).

### Differential harvest of samples from AMBR^{®}15 bioreactor system

One millilitre harvest from an AMBR^{®}15 production was centrifuged for 5 min at 150 x g and room temperature. The supernatant was transferred to a new vial ("supernatant" fraction). The pellet after centrifugation was washed with 1 mL of supplemented BalanCD HEK293 medium and centrifuged for 5 min at 150 x g and room temperature. The supernatant ("wash fraction") was discarded. The pellet after centrifugation was resuspended in 1 mL of supplemented BalanCD HEK293 medium ("cell-associated" fraction) and subjected to cell lysis by freeze-thaw.

### Quantification of rAAV vector genomes

The AAV vector genome assay is based on a quantitative polymerase chain reaction (qPCR) specific for a sequence of the rAAV expression cassette.

Cell lysate test samples were subjected to a nuclease treatment procedure to remove non-packed vector genomes prior to performing the qPCR. To that aim, denarase (c-LEcta GmbH, Leipzig, Germany) was added to the samples with a final concentration of 100 U/mL. Incubation was performed for 2 h at 37°C. Afterwards, the samples were centrifuged for 5 min at 4500 x g and room temperature. The samples were filtered using polyethersulfone (PES) 0.45 µm membrane filters. The samples were either stored at -80°C until performance of the qPCR or used directly for the analytical procedure.

Per sample, 10 µl 2x iTaq Universal SYBR Green Supermix (Biorad) were mixed with 0.80 µl of qPCR primer working stock solution (containing 10 µM of each primer) and filled up to a volume of 15 µl with nuclease-free water. 5 µl of pre-treated cell lysate or purified virus test sample were added to the mix (total reaction volume 20 µl, final primer concentration in the reaction 400 nM each) and qPCR was performed in a CFX 96 Touch Real Time PCR cycler (Bio-Rad Laboratories Inc., Hercules, USA) with following program steps: 95°C 5 min; 40 cycles (95°C 5 s, 60°C 30 s, plate read); 95°C 10 sec; 60-95°C (+0.5°C/step), 10 sec; plate read. To control for the quality of the qPCR, a trending control with known rAAV vector genome titre was measured in parallel. To check for contaminations, a no template control (NTC, 5 µl H₂O) was also included. Standard row, test samples and controls were measured in triplicates for each dilution. Denarase treated cell lysate test samples, purified virus test samples and trending control were generally measured in three dilutions in EB buffer (10 mM Tris-Cl, pH 8.5). Data were analysed using the CFX Maestro^{™} Software 2.0 (Bio-Rad Laboratories Inc.). Melting curve analysis confirmed the presence of only one amplicon. Amplification results in nascent double stranded DNA amplicons detected with the fluorescent intercalator SYBR Green to monitor the PCR reaction in real time. Known quantities of the genetic material, in the form of a linearised plasmid, were serially diluted to create a standard curve and sample vector genome titre was interpolated from the standard curve.

### Quantification of rAAV vector genomes by ddPCR

The AAV vector genome assay is based on a droplet digital PCR (ddPCR) specific for a transgene sequence of the rAAV expression cassette.

Cell lysate test samples were subjected to a nuclease treatment procedure to remove non-packed vector genomes prior to performing the ddPCR. To that aim, denarase (c-LEcta GmbH, Leipzig, Germany) was added to the samples with a final concentration of 20 U/mL. Incubation was performed over night with shaking at room temperature. Afterwards, the samples were centrifuged for 30 min at 3700 x g and room temperature or for 10 min at 4700 x g and room temperature. The samples were either stored at -80°C until performance of the ddPCR or used directly for the analytical procedure. To control for the quality of the ddPCR, a trending control with known rAAV vector genome titre was measured in parallel. To check for contaminations, a no template control (NTC, EB) was also included.

Per reaction, 10 µl ddPCR^{™} EvaGreen Supermix (Bio-Rad Laboratories Inc., Hercules, USA) were mixed with 1 µL of primer mix (containing 2 µM of each primer) resulting in a reaction master mix volume of 11 µL per reaction. For the test samples, the optimal copy number per reaction is between 10000 and 40000. Thus, samples and trending control were diluted accordingly in elution buffer. The specific working range of this method was determined to be 150 - 120000 copies per reaction. Three dilutions each (in duplicates) were prepared for each sample and trending control using EB buffer (10 mM Tris-Cl, pH 8.5). Pre reaction, 9 µL of test sample dilution or trending control were mixed with 11 µl of the reaction master mix to result in the total reaction volume of 20 µL. Samples were partitioned with droplet generator oil (Bio-Rad Laboratories Inc., Hercules, USA) using a droplet generator (Bio-Rad Laboratories Inc., Hercules, USA) according to manufacturer's instructions.

PCR reaction was performed in a C1000 Touch Thermal Cycler 96 Deep Well (Bio-Rad Laboratories Inc., Hercules, USA) with following program steps: 95°C 5 min; 40 cycles (95°C 30 s, 60°C 1min); 4°C 5 min; 90°C 5 min; 8°C 30 min. A ramp rate of 2°C per second was applied. Droplet readout was performed using a QX200 or QX600 droplet reader (Bio-Rad Laboratories Inc., Hercules, USA). The cycled droplets are analysed using microfluidics to identify droplets which are positive and negative for the respective template/fluorophore (end point assay). From the overall droplet number and the positive and negative droplet numbers the Bio-Rad QX Manager software calculates the initial DNA concentration based on Poisson statistics.

### Quantification of rAAV capsids by AAV9 Titration ELISA

The AAV9 Titration ELISA method is a measure of total AAV particles (capsids) and is based on a commercially available kit (Progen^{™}, Heidelberg, Germany; catalogue number PRAAV9). This sandwich immunometric technique utilises a monoclonal antibody specific for a conformational epitope on assembled AAV9 capsids for both capture and detection. The AAV9 Titration ELISA kit was used to quantify total AAV particles in cell lysates and purified virus preparations according to the manufacturer's instructions. In brief, 100 µl diluted AAV9 Kit Control, test samples, or trending control of capsid with known total particle titre were added per well of a microtiter plate coated with monoclonal antibody specific for a conformational epitope on assembled AAV9 capsids and incubated for 1 h at 37°C. Standard row, test samples and controls were measured in duplicates for each dilution. In a second step, 100 µl of pre-diluted biotin-conjugated monoclonal antibody (1:20 in Assay Buffer [ASSB]) were added and incubated for 1 h at 37°C. Then, 100 µl of a pre-diluted streptavidin peroxidase conjugate (1:20 in ASSB) were added and incubated for 1 h at 37°C. 100 µl substrate solution (TMB [Tetramethylbenzidine]) were added and after incubation for 15 min, the reaction was stopped using 100 µl stop solution. The absorbance was measured photochemically at 450 nm using the SpectraMax M3 microplate reader (Molecular Devices, San Jose, USA). Data was analysed with the SoftMax Pro 7.0 Software (Molecular Devices).

### Quantification of rAAV capsids by Gyrolab xPlore

rAAV capsids were quantified using the automated immunoassay platform Gyrolab xPlore^{™} (Gyros Protein Technologies AB, Uppsala, Sweden) in conjunction with the Gyrolab^{®} AAVX Titer Kit for AAV serotypes 1-8 or the Gyrolab^{®} AAV9 Titer Kit for AAV9. The sandwich immunometric technique of the generic Gyrolab^{®} AAVX Titer Kit used to capture and detect AAV capsids of serotypes 1-8 is based on the Thermo Scientific^{™} CaptureSelect^{™} Biotin Anti-AAVX Conjugate and Thermo Scientific CaptureSelect Alexa Fluor^{™} 647 Anti-AAVX Conjugate from Thermo Fisher Scientific (Waltham, Massachusetts, USA). The sandwich immunometric technique of the Gyrolab^{®} AAV9 Titer Kit used to capture and detect AAV9 is based on the highly selective Thermo Scientific^{™} CaptureSelect^{™} Biotin Anti-AAV9 Conjugate and Thermo Scientific CaptureSelect Alexa Fluor^{™} 647 Anti-AAV9 Conjugate from Thermo Fisher Scientific (Waltham, Massachusetts, USA). A serial dilution of control capsids (in-house for AAV3 or commercially available empty capsids for AAV1, 2, 5, 6, 8 and 9 [(Progen^{™}, Heidelberg, Germany, catalogue numbers: 66V010, 66V020, 66V050, 66V060, 66V080, 66V090]) was applied to interpolate AAV capsid concentrations in the test samples. A negative control was included to check for contamination of reagents. Denarase^{®} treated cell lysates, purified virus test samples and AAV control serial dilution were diluted in Gyrolab^{®} AAVX Titer Sample Dilution Buffer. All samples and controls were tested in duplicate. Data were analyzed using the Gyrolab^{®} Evaluator Software Version 3.7.2.5976.

### Vector genome to total particle ratio

The ratio of vector genomes to total AAV particles is expressed as a percentage. This is based on the vector genome titre (determined by qPCR or ddPCR, as described above) and the number of total AAV particles (determined by AAV9 Titration ELISA or Gyrolab xPlore, as described above).

### Quantification of plasmid-derived impurities by qPCR

Prokaryotic DNA sequences, such as antibiotic resistance genes or parts of them originating from the bacterial backbone of the producer plasmids, can be packaged into the rAAV particles, constituting product-related impurities. Plasmid-derived impurity quantification is based on experiments using qPCR techniques specific for defined sequences of the kanamycin resistance gene (kanR), present on both helper and vector plasmid, and the AAV cap gene, which is present on the vector plasmid. Plasmid-derived impurity qPCRs were performed on one-step, spin-protocol based affinity chromatography purified rAAV material. The spin-procedure was performed using AVB Sepharose HP (Cytiva, Marlborough, Massachusetts, USA) or POROS^{™} CaptureSelect^{™} AAVX Affinity Resin (Thermo Fisher Scientific, Waltham, Massachusetts, USA). The plasmid-derived impurity qPCRs were performed according to the method for *"quantification of rAAV vector genomes by qPCR"* outlined above with appropriate modifications to reflect the impurity templates, such as the primer pairs, standards and controls. Specifically, modifications were made to the impurity specific primer pairs, the linearized plasmid standards, the trending controls, sample dilution and the annealing temperatures (57°C for kanR) in the qPCR setup.

### Quantification of plasmid-derived impurities by ddPCR

Prokaryotic DNA sequences, such as antibiotic resistance genes or parts of them originating from the bacterial backbone of the producer plasmids, can be packaged into the rAAV particles, constituting product-related impurities. Plasmid-derived impurity quantification is based on experiments using droplet digital PCR (ddPCR) techniques specific for defined sequences of the kanamycin resistance gene (kanR), present on both helper and vector plasmid, and the AAV cap gene, which is present on the vector plasmid. Plasmid-derived impurity ddPCRs were performed on one-step, spin-protocol based affinity chromatography purified rAAV material. The spin-procedure was performed using POROS^{™} CaptureSelect^{™} AAVX Affinity Resin (Thermo Fisher Scientific, Waltham, Massachusetts, USA). Duplex droplet digital PCR (2D ddPCR) enables the simultaneous quantification of plasmid-derived AAV cap gene impurities and kanamycin resistance gene (kanR) impurities as well as the calculation of percentage cap/kanR impurities based on the respective vector genome titres of purified AAV samples. Per reaction, 10 µL 2x ddPCR Supermix for Probes (no UTP) (Bio-Rad Laboratories Inc., Hercules, USA) were mixed with 1 µL of primer mix A (containing 18 µM of each primer), 1 µL of primer mix B (containing 18 µM of each primer), 1 µL of probe A (containing 5 µM of a FAM fluorophore-labelled probe) and 1 µL of probe B (containing 5 µM of a HEX fluorophore-labelled probe) resulting in a reaction master mix volume of 14 µL per reaction. To control for the quality of the ddPCR, a trending control with known rAAV vector genome titre was measured in addition to the test samples. To check for contaminations, a no template control (NTC, EB) was also included. For the test samples, the optimal copy number per reaction is between 20000 and 40000. Thus, samples and trending control were diluted accordingly in elution buffer. The specific working range of this method was determined to be 189 - 120000 copies per reaction for AAV cap and 83 - 120000 copies per reaction for kanR. Three dilutions each (in duplicates) were prepared for each sample and trending control using EB buffer (10 mM Tris-Cl, pH 8.5). Pre reaction, 6 µL of test sample dilution or trending control were mixed with 14 µl of the reaction master mix to result in the total reaction volume of 20 µL. Samples were partitioned with droplet generator oil (Bio-Rad Laboratories Inc., Hercules, USA) using a droplet generator (Bio-Rad Laboratories Inc., Hercules, USA) according to manufacturer's instructions. PCR reaction was performed in a C1000 Touch Thermal Cycler 96 Deep Well (Bio-Rad Laboratories Inc., Hercules, USA) with following program steps: 95°C 10 min; 40 cycles (94°C 30 s, 60°C 1min); 98°C 10 min. A ramp rate of 2°C per second was applied. Droplet readout was performed using the QX200 or QX600 droplet reader (Bio-Rad Laboratories Inc., Hercules, USA). The cycled droplets are analysed using microfluidics to identify droplets which are positive and negative for the respective template/fluorophore (end point assay). From the overall droplet number and the positive and negative droplet numbers the Bio-Rad QX Manager software calculates the initial DNA concentration based on Poisson statistics.

### Quantification of host cell-derived DNA impurities by ddPCR

DNA sequences, such as nucleic acid material from the genome of host cells in which AAV are produced, can be packaged into rAAV particles, constituting product-related impurities. Host cell-derived impurity quantification was carried out using droplet digital polymerase chain reaction (ddPCR) specific for defined sequences of the 18S rRNA gene locus of the 45S ORF present in HEK293 cells. Host cell-derived impurity ddPCR was performed on one-step, spin-protocol based affinity chromatography purified rAAV material. The spin-procedure was performed using AVB Sepharose HP (Cytiva, Marlborough, Massachusetts, USA) or POROS ^{™} CaptureSelect^{™} AAVX Affinity Resin (Thermo Fisher Scientific, Waltham, Massachusetts, USA). The test samples were diluted in EB (10mM Tris-Cl, pH 8.5) in a range of 1:10 to 1:20. The reaction mixtures were prepared using ddPCR Supermix for Probes (Bio-Rad Laboratories Inc., Hercules, USA) with 0.9 µM primers specific for the 18S rRNA gene locus and 0.25 µM probe specifically binding to the 18S rRNA gene locus and 8 µL sample dilution in a final volume of 20 µL as described in the manufacturer's protocol. To control for the quality of the ddPCR, a trending control was measured in parallel. To check for contaminations, a no template control (NTC, H₂O or EB) was also included. Test samples and controls were measured in duplicates for each dilution. Samples were partitioned with droplet generator oil for probes (Bio-Rad Laboratories Inc., Hercules, USA) using a QX-200 droplet generator (Bio-Rad Laboratories Inc., Hercules, USA) according to manufacturer's instructions. PCR amplification of the droplets was performed using a thermal cycler with the following parameters for a short amplicon: 95°C 10 min, 40 cycles (94°C 30 s, 59°C 1 min), 98°C 10 min. PCR amplification of the droplets was performed using a thermal cycler with the following parameters for a larger amplicon: 95°C 10 min, 51 cycles (94°C 30 s, 59°C 1 min 5 sec), 98°C 10 min. Droplet read-out of the PCR plate wells was carried out on the QX200 droplet reader (Bio-Rad Laboratories Inc., Hercules, USA) and data were analyzed with QX Manager software (Bio-Rad Laboratories Inc., Hercules, USA). The cycled droplets are analysed using microfluidics to identify droplets which are positive and negative for the respective template/fluorophore (end point assay). From the overall droplet number and the positive and negative droplet numbers the Bio-Rad QX Manager software calculates the initial DNA concentration based on Poisson statistics.

### 96-well absolute potency assay

Transduction was performed in a 96-well tissue culture plate seeded with HuH-7 cells at 2.5 x 10⁴ vc/well (manual cell count using a "Neubauer" chamber; vc = viable cells). The HuH-7 cells were transduced with one-step, spin-protocol based affinity chromatography purified rAAV comprising an expression cassette encoding an appropriate marker which had been prepared using the control Rep78neg or the Rep78pos two plasmid system of the invention, as set out above. Transduction occurred 5 h after the cells were seeded. Transduction was performed at a multiplicity of infection (MOI) of 1.17 × 10⁴ with three replicates per sample and a no transduction control (no virus). The MOI was based on vector genome titre, which was determined as set out above under the heading *"Quantification of rAAV vector genomes by qPCR".* After a production phase of 72 h, the supernatant was harvested. The marker activity in the cell supernatant was determined by a fluorogenic activity assay.

### Example 6: Rep78pos two plasmid system: comparison of helper:vector plasmid ratios

HEK293 cells were cultivated according to the method for *"cell cultivation"* outlined above. Transfection of HEK293 suspension cells in an AMBR^{®}15 bioreactor system was performed according to the method for *"Transfection of HEK293 suspension cells in an AMBR^{®}15 bioreactor system and preparation of cell lysates"* using five molar plasmid ratios in the range of 1:3 to 6:3 [helper:vector]. The vector plasmid encoded for the AAV9 capsid and contained a vector genome of approx. 3kb encoding for a gene marker.

The products of the transfection step were assayed as described in *"Quantification of rAAV vector genomes by qPCR", "Quantification of rAAV capsids by AAV9 Titration ELISA", "Vector genome to total particle ratio", "Quantification of plasmid-derived impurities by qPCR", "Quantification of host cell-derived DNA impurities by ddPCR".*

### Results

As apparent from Figure 1A, increasing the portion of the helper plasmid led to higher vector genome yields with the highest vector genome yields at ratios of 5:3 and 6:3 [helper:vector]. As apparent from Figure 1B, no relevant changes in the capsid yields were observed by increasing the portion of the helper plasmid, which is expected as the portion of the vector plasmid encoding for the AAV capsid gene remained constant. As apparent from Figure 1C, increasing the portion of the helper plasmid led to higher vector genome to total capsid ratios with the highest ratio at a plasmid ratio of 6:3 [helper:vector]. As apparent from Figures 1D and 1E, increasing the portion of the helper plasmid led to decreasing levels of plasmid derived impurities (both cap and kanR impurities) with the lowest mispackaging at a ratio of 6:3 [helper:vector]. Figure 1F shows that the level of host cell DNA derived impurities did not change significantly by increasing the portion of the helper plasmid.

### Example 7: Comparison of a Rep78neg two plasmid system to a Rep78pos two plasmid system

HEK293 cells were cultivated according to the method for *"cell cultivation"* outlined above. Transfection of HEK293 suspension cells in an AMBR^{®}15 bioreactor system was performed according to the method for *"Transfection of HEK293 suspension cells in an AMBR^{®}15 bioreactor system and preparation of cell lysates"* using the either the control Rep78neg two-plasmid system or the Rep78pos two plasmid system of the invention. A molar ratio of 1:3 [helper:vector] or 4:3 [helper:vector] was applied for the control plasmid system or the Rep78pos two plasmid system. The vector plasmid encoded for the AAV9 capsid and contained a vector genome of approx. 3kb encoding for a gene marker.

The products of the transfection step were assayed as described in *"Quantification of rAAV vector genomes by qPCR", "Quantification of rAAV capsids by AAV9 Titration ELISA", "Vector genome to total particle ratio", "Quantification of plasmid-derived impurities by qPCR", "Quantification of host cell-derived DNA impurities by ddPCR".*

### Results

As shown in Figure 2A, no significant difference in vector genome yield was observed between the Rep78neg plasmid system and the Rep78pos plasmid system of the invention. As apparent from Figure 2B, a Rep78neg plasmid system demonstrated increased capsid yields in comparison to a Rep78pos plasmid system. However, as it is apparent from Figure 2C, the Rep78pos two plasmid system demonstrated increased vector genome to total capsid ratio. Figure 2D to Figure 2F show that the level of plasmid-derived and host cell DNA derived impurities was reduced for the Rep78pos plasmid system compared to a Rep78neg plasmid system, signifying a beneficial performance for the Rep78pos system of the invention.

### Example 8: Rep78pos two plasmid system in a variety of AAV serotypes

HEK293 cells were cultivated according to the method for *"cell cultivation"* outlined above. Transfection of HEK293 suspension cells in an AMBR^{®}15 bioreactor system was performed according to the method for *"Transfection of HEK293 suspension cells in an AMBR^{®}15 bioreactor system and preparation of cell lysates"* using the split 2-plasmid system with a variety of vector plasmids encoding in total seven AAV capsids [AAV1, AAV2, AAV3, AAV5, AAV6, AAV8 and AAV9]. The same vector genome of approximately 3kb encoding the same gene marker was applied for all setups. A molar ratio of 4:3 [helper:vector] was applied. In addition to the sample harvest according to the method for *"Transfection of HEK293 suspension cells in an AMBR^{®}15 bioreactor system and preparation of cell lysates",* a differential harvest of samples was performed according to the method for *"Differential harvest of samples from AMBR^{®}15 bioreactor system"* as outlined above.

The products of the transfection step were assayed as described in *"Quantification of rAAV vector genomes by ddPCR", "Quantification of rAAV capsids by Gyrolab xPlore", "Vector genome to total particle ratio".*

### Results

As apparent from Figure 3A (vector genome yields) and Figure 3B (capsid yields), rAAV could be efficiently produced across serotypes using the Rep78pos two plasmid system of the invention. Figure 3C shows that comparable levels of vector genome to total particle ratios were obtained across the different serotypes. Figure 3D and 3E show that the AAV particle distribution based on a rAAV production using the Rep78pos two plasmid system did not differ from the observations previously made for the different serotypes.

### Example 9: Comparison of a Rep78neq two plasmid system to the Rep78pos two plasmid system using different transgene sizes

HEK293 cells were cultivated according to the method for *"cell cultivation"* outlined above. Transfection of HEK293 suspension cells in an AMBR^{®}15 bioreactor system was performed according to the method for *"Transfection of HEK293 suspension cells in an AMBR^{®}15 bioreactor system and preparation of cell lysates"* using either a Rep78neg or a Rep78pos two plasmid system. A molar ratio of 1:3 [helper:vector] or 4:3 [helper:vector] was applied for the Rep78neg or the Rep78pos two plasmid system. The vector plasmid encoded for the AAV9 capsid and contained either a vector genome of approx. 3kb or 4.7kb encoding for the same gene marker without or with an additional stuffer sequence.

The products of the transfection step were assayed as described in *"Quantification of rAAV vector genomes by ddPCR", "Quantification of rAAV capsids by Gyrolab xPlore", "Vector genome to total particle ratio", "Quantification of plasmid-derived impurities by ddPCR", "Quantification of host cell-derived DNA impurities by ddPCR".*

### Results

As shown in Figure 4A, an increase in vector genome yield was observed when using the Rep78 pos two plasmid system compared to a Rep78neg plasmid system. As apparent from Figure 4B, no significant difference was observed for the capsid yield comparing the Rep78pos two plasmid system with the Rep78neg control system. Additionally, as shown in Figure 4C, use of the Rep78pos two plasmid system led to increased vector genome to total capsid ratio. Figure 4D to Figure 4F show that the level of plasmid-derived and host cell DNA derived impurities were significantly reduced for the Rep78pos two plasmid system in comparison to a Rep78neg control plasmid system.

### Example 10: Rep78pos two plasmid system- Comparison of potency

### Preparation of the rAA V for transduction

rAAV for transduction were prepared using the method according to *"Transfection of HEK293 suspension cells in an AMBR^{®}15 bioreactor system and preparation of cell lysates"* using the Rep78neg two plasmid system as a control and the Rep78pos two plasmid system of the invention. In particular, the rAAV were generated using a vector plasmid with an approx. 3kb vector genome encoding for an appropriate gene marker. A molar ratio of 1:3 [helper:vector] or 4:3 [helper:vector] was used for the Rep78neg two plasmid system or the Rep78pos two plasmid system.

The products of the transfection step were nuclease treated and then purified by an AVB-spin (batch) procedure using AVB Sepharose HP (Cytiva, Marlborough, Massachusetts, USA). Buffer exchange was then performed in a buffer that was suitable for cell-based assays. The rAAV were then used in the 96-well absolute potency assay according to *"96-well absolute potency assay".*

### Results

As apparent in Figure 5, application of the Rep78pos two plasmid system resulted in an increased transgene activity compared to the control Rep78neg plasmid system.

### Example 11: Scale-up to larger production formats for rAAV production using the Rep78pos two plasmid system

HEK293 cells were cultivated according to the method for *"cell cultivation"* outlined above. Transfection of HEK293 suspension cells in a Biostat^{®} STR 50 L bioreactor system and Biostat^{®} STR 200 L bioreactor system was performed according to the methods for *"Transfection of HEK293 suspension cells in a Biostat^{®} STR 50 L bioreactor system and preparation of cell lysates"* and *Transfection of HEK293 suspension cells in a Biostat^{®} STR 200 L bioreactor system and preparation of cell lysates"* using the Rep78pos two plasmid system. A molar ratio of 4:3 [helper:vector] was applied for the Rep78pos two plasmid system. The vector plasmid encoded for the AAV9 capsid and contained a vector genome of approx. 3kb encoding for a gene.

The products of the transfection step were assayed as described in *"Quantification of rAAV vector genomes by ddPCR", "Quantification of rAAV capsids by Gyrolab xPlore", "Vector genome to total particle ratio", "Quantification of plasmid-derived impurities by ddPCR", "Quantification of host cell-derived DNA impurities by ddPCR".*

As apparent in Figure 6A and Figure 6B, rAAV can be effectively produced in 50 Litres and 200 Litres stirred-tank bioreactor format using the Rep78pos two plasmid system of the invention, with the highest yields obtained for the 200 Litres bioreactor format. As apparent in Figure 6C, no relevant difference was observed between the 50 Litres and 200 Litres bioreactor format concerning the vector genome to total particle ratio using the Rep78pos plasmid system. As apparent in Figure 6D and Figure 6E, no relevant differences in plasmid-derived impurity levels were observed in the 50 Litres and 200 Llitres bioreactor based rAAV productions using the Rep78pos two plasmid system. Figure 6F shows, that host cell DNA derived impurities are in a comparable range for rAAV productions in 50 Litres and 200 Litres bioreactors.

## Claims

1. A helper plasmid which does not comprise a cap gene encoding a functional set of Cap proteins and which does comprise at least one Adeno-associated virus (AAV) rep gene and at least one helper virus gene, wherein the at least one rep gene comprises a rep 78 cassette comprising a nucleotide sequence encoding a functional Rep 78 protein and a transcription regulatory element or a rep 78 cassette comprising a nucleotide sequence encoding a functional Rep 78 protein and a functional Rep 68 protein and a transcription regulatory element, and wherein said rep 78 cassette comprises an ATG start codon at the beginning of the nucleotide sequence encoding a Rep 78 protein or both a Rep 78 and a Rep 68 protein.

2. A helper plasmid according to claim 1 which does not comprise a cap gene encoding a functional set of Cap proteins and which does comprise at least one rep gene and at least one helper virus gene, wherein the at least one rep gene comprises a rep 78 cassette comprising a nucleotide sequence encoding a functional Rep 78 protein and a transcription regulatory element or a rep 78 cassette comprising a nucleotide sequence encoding a functional Rep 78 protein and a functional Rep 68 protein and a transcription regulatory element, and wherein said rep 78 cassette comprises an ATG start codon at the beginning of the nucleotide sequence encoding a functional Rep 78 protein or both a Rep 78 and a Rep 68 protein, **characterised in that** said helper plasmid promotes production of recombinant AAV at a higher yield compared to a helper plasmid comprising at least one rep gene which does not encode a functional Rep 78 protein or both a Rep 78 and a Rep 68 protein, and/or at a lower level of nucleic acid impurities (normalised to vector genome level) than the level of nucleic acid impurities obtained compared to a helper plasmid comprising at least one rep gene which does not encode a functional Rep 78 protein or both a Rep 78 and a Rep 68 protein.

3. The helper plasmid of claim 1 or 2, wherein the nucleotide sequence encoding a functional Rep 78 protein:
(i) has at least 90%, at least 95%, at least 98%, at least 99%, or 100% identity to the full length or a fragment of at least 1500, at least 1600, at least 1700, at least 1800, or at least 1850 nucleotides of SEQ ID NO: 8; and/or
(ii) encodes a protein that has at least 90%, at least 95%, at least 98%, at least 99%, or 100% identity to the full length or a fragment of at least 500, at least 550, at least 560, at least 570, or at least 575 amino acids of SEQ ID NO: 15.

4. The helper plasmid of any one of the preceding claims, wherein the rep 78 cassette comprises a nucleotide sequence that has at least 90%, at least 95%, at least 98%, at least 99%, or 100% identity to the full length or a fragment of at least 1500, at least 1600, at least 1700, at least 1800, or at least 1900 nucleotides of SEQ ID NO: 9.

5. The helper plasmid of any one of the preceding claims, wherein the at least one rep gene further comprises a gene encoding a functional Rep 52 protein, a gene encoding a functional Rep 40 protein, and a gene encoding a functional Rep 68 protein, optionally wherein the gene encoding a functional Rep 52 protein, the gene encoding a functional Rep 40 protein, the gene encoding a functional Rep 68 protein, and the gene encoding a functional Rep 78 protein all contain a common portion, optionally wherein:
(i) the common portion comprises a sequence having at least 90%, at least 95%, at least 98%, at least 99%, or 100% identity to the full length or a fragment of at least 500, at least 550, at least 600, at least 650, or at least 700 nucleotides of SEQ ID NO: 14; and/or
(ii) the common portion encodes an amino acid sequence having at least 90%, at least 95%, at least 98%, at least 99%, or 100% identity to the full length or a fragment of at least 150, at least 210, at least 220, at least 230, or at least 238 amino acids of SEQ ID NO: 16.

6. The helper plasmid of any one of the preceding claims, wherein the at least one helper virus gene:
(i) comprises a VA nucleic acid, an E2A gene and an E4 gene; and/or
(ii) is comprised on a contiguous stretch of the plasmid having at least 95%, at least 98%, at least 99%, or 100% identity to the full length or to a fragment at least 6000, at least 7000, or at least 8000 nucleotides in length of SEQ ID NO: 4.

7. The helper plasmid of any one of the preceding claims, wherein the helper plasmid is less than 20000 bp, less than 15000 bp, less than 14000 bp, less than 13000bp, between 10000 bp and 20000 bp, between 11000 bp and 15000 bp, between 12000 bp and 13000 bp in length.

8. The helper plasmid of any one of the preceding claims, wherein said lower level of nucleic acid impurities is
(i) a lower level of vector plasmid impurities; and/or
(ii) a lower level of cap gene and/or vector plasmid backbone impurities; and/or
(iii) a level of cap gene impurities; and/or
(iv) a lower level of plasmid backbone impurities; and/or
(v) a lower level of helper plasmid backbone impurities; and/or
(vi) a lower level of host cell derived impurities,

9. A plasmid system, preferably a two-plasmid system comprising the helper plasmid of any one of the preceding claims and a vector plasmid.

10. The two-plasmid system of claim 9, wherein the ratio of helper plasmid to vector plasmid is between 1:10 to 10:1, between 1:3 and 7:3, between 1:3 and 6:3, preferably around 1:3 or around 2:3 or around 3:3 or around 4:3 or around 5:3, or around 6:3.

11. The two-plasmid system of claim 9 or 10, wherein the vector plasmid comprises:
(a) a cap gene encoding at least one functional Cap protein; or
(b) at least one cap gene promoter, a cloning site operably linked to the cap gene promoter, and an expression cassette flanked on at least one side by an ITR;
wherein the vector plasmid does not comprise a rep gene encoding a functional Rep protein and the expression cassette comprises a transgene operably linked to at least one regulatory control element

12. The two-plasmid system of claim 11, wherein the vector plasmid comprises a cap gene and further comprises an expression cassette flanked on at least one side by an ITR, optionally wherein the expression cassette comprises a transgene operably linked to at least one regulatory control element, preferably the expression cassette being less than 5.0 kbp, less than 4.9 kbp, less than 4.8 kbp, less than 4.5 kbp, between 2.0 kbp and 5.5 kbp, between 2.1 kbp and 4.9 kbp, between 2.2 kbp and 4.8 kbp or between 2.3 kbp and 4.5 kbp.

13. A method for producing a recombinant AAV preparation with a lower level of nucleic acid impurities (normalised to vector genome level) than the level of nucleic acid impurities obtained using an equivalent method using a two-plasmid system comprising a rep 78 negative helper plasmid, said method comprising:
(a) obtaining the helper plasmid or the two-plasmid system of any one of claims 1 to 11;
(b) transfecting a host cell with the helper plasmid or the two-plasmid system of any one of claims 1 to 11; and
(c) culturing the host cell in suspension under conditions suitable for recombinant AAV production.

14. The method of claim 13, wherein the lower level of nucleic acid impurities:
(i) is a lower level of vector plasmid impurities; and/or
(ii) is a lower level of cap gene and/or vector plasmid backbone impurities; and/or
(iii) is a level of cap gene impurities that is less than 0.8 fold, less than 0.7 fold, less than 0.65 fold, less than 0.55 fold, less than 0.5 fold, less than 0.25 fold, less than 0.2 fold, between 0.1 fold and 0.8 fold, between 0.15 fold and 0.7 fold, between 0.15 fold and 0.65 fold, between 0.15 fold and 0.55 fold, between 0.15 fold and 0.5 fold, or between 0.15 fold and 0.2 fold of the level of cap gene impurities produced using an equivalent method using a two-plasmid system comprising a rep 78 negative helper gene; and/or
(iv) is a level of vector plasmid backbone impurities that is less than 0.6 fold, less than 0.5 fold, less than 0.45 fold, less than 0.4 fold, less than 0.35 fold, less than 0.2 fold, less than 0.15 fold, between 0 fold and 0.6 fold, between 0.05 fold and 0.5 fold, between 0.1 fold and 0.45 fold, between 0.1 fold and 0.4 fold, between 0.1 fold and 0.35 fold, between 0.1 fold and 0.2 fold, or between 0.1 fold and 0.15 fold of the level of vector plasmid backbone impurities obtained using an equivalent method using a two-plasmid system comprising a rep 78 negative helper gene,
optionally, wherein the level of vector plasmid impurities or cap gene impurities is determined by qPCR or ddPCR using primers that are complementary to a portion of the cap gene, or the level of vector plasmid impurities or vector plasmid backbone impurities is determined by qPCR or ddPCR using primers that are complementary to a portion of the vector plasmid backbone, optionally an antibiotic resistance gene such as kanamycin R.

15. The method of claim 13 or 14, wherein the lower level of nucleic acid impurities comprises (a) a lower level of plasmid backbone impurities; and/or (b) a lower level of helper plasmid backbone impurities; and/or (c) a lower level of host cell derived impurities,
optionally wherein:
(i) the lower level of plasmid backbone impurities and/or helper plasmid backbone impurities comprises a level of plasmid backbone impurities and/or helper plasmid backbone impurities that is less than 0.6 fold, less than 0.5 fold, less than 0.45 fold, less than 0.4 fold, less than 0.35 fold, less than 0.2 fold, less than 0.15 fold, between 0 fold and 0.6 fold, between 0.05 fold and 0.5 fold, between 0.1 fold and 0.45 fold, between 0.1 fold and 0.4 fold, between 0.1 fold and 0.35 fold, between 0.1 fold and 0.2 fold, or between 0.1 fold and 0.15 fold of the level of plasmid backbone impurities and/or helper plasmid backbone impurities obtained using an equivalent method using a two-plasmid system comprising a rep 78 negative helper gene; or
(ii) the level of vector plasmid impurities and/or vector plasmid backbone impurities is determined by qPCR or ddPCR using primers that are complementary to a portion of the vector plasmid backbone, optionally an antibiotic resistance gene such as kanamycin R; or
(iii) the lower level of host cell derived impurities comprises a lower level of 18S rRNA gene impurities; or
(iv) the level of host cell-derived or 18S rRNA gene impurities is determined by ddPCR using primers and a probe that are complementary to a portion of the host cell genome, optionally an 18S rRNA gene.

16. The method of any one of claims 13 to 15, wherein:
(a) the ratio of helper plasmid to vector plasmid comprises a molar excess of helper plasmid; and/or
(b) the ratio of helper plasmid to vector plasmid is between 1:10 to 10:1, between 1:3 and 7:3, between 1:3 and 6:3, preferably around 1:3 or around 2:3 or around 3:3 or around 4:3 or around 5:3, or around 6:3.

17. The method of any one of claims 13 to 16, wherein culturing the host cell under conditions suitable for recombinant AAV production comprises culturing the host cell using a suspension system.

18. The method of any one of claims 13 to 17, wherein the method:
(a) further comprises a step of purifying the recombinant AAV, optionally wherein the step of purifying the recombinant AAV comprises using a technique selected from the group consisting of gradient density centrifugation (such as CsCI or lodixanol gradient density centrifugation), filtration, ion exchange chromatography, size exclusion chromatography, affinity chromatography and hydrophobic interaction chromatography; and/or
(b) comprises concentrating the recombinant AAV using ultracentrifugation, tangential flow filtration, or gel filtration; and/or
(c) comprises formulating the recombinant AAV with a pharmaceutically acceptable excipient.

19. A recombinant AAV preparation obtainable or obtained by the method of any one of claims 13 to 18.

20. Use of the helper plasmid or the two-plasmid system of any one of claims 1 to 12 for:
(i) producing a recombinant AAV preparation; and/or
(ii) increasing, optimising and/or maximising the yield of recombinant AAV produced during recombinant AAV production; and/or
(iii) increasing the yield of recombinant AAV produced during recombinant AAV production compared to an equivalent use of a two-plasmid system comprising a rep 78 negative helper plasmid; and/or
(iv) controlling and/or maximising the ratio of full to total particles produced during recombinant AAV production; and/or
(v) reducing and/or minimising the level of nucleic acid impurities (normalised to vector genome level) produced during recombinant AAV production; and/or
(vi) reducing the level of nucleic acid impurities (normalised to vector genome level) produced during recombinant AAV production compared to an equivalent use of a two-plasmid system comprising a rep 78 negative helper plasmid; and/or
(vii) increasing the potency of recombinant AAV produced during recombinant AAV production; and/or
(viii) increasing the potency of recombinant AAV produced during recombinant AAV production compared to an equivalent use of a two-plasmid system comprising a rep 78 negative helper plasmid.

21. A method for:
(i) producing a recombinant AAV preparation comprising:
(a) obtaining the helper plasmid or the two-plasmid system of any one of claims 1 to 12;
(b) transfecting a host cell with the helper plasmid or the two-plasmid system of any one of claims 1 to 12; and
(c) culturing the host cell in suspension under conditions suitable for recombinant AAV production; or
(ii) increasing, optimising and/or maximising the yield of recombinant AAV produced during recombinant AAV production;
(iii) increasing the yield of recombinant AAV produced during recombinant AAV production compared to an equivalent method using a two-plasmid system comprising a rep 78 negative helper plasmid;
(iv) controlling and/or maximising the ratio of full to total particles produced during recombinant AAV production;
(v) reducing and/or minimising the level of nucleic acid impurities (normalised to vector genome level) produced during recombinant AAV production;
(vi) reducing the level of nucleic acid impurities (normalised to vector genome level) produced during recombinant AAV production compared to an equivalent method using a two-plasmid system comprising a rep 78 negative helper plasmid;
(vii) increasing the potency of recombinant AAV produced during recombinant AAV production; or
(vii) increasing the potency of recombinant AAV produced during recombinant AAV production compared to an equivalent method of a two-plasmid system comprising a rep 78 negative helper plasmid,
comprising:
a) obtaining the helper plasmid or the two-plasmid system of any one of claims 1 to 12;
(b) transfecting a host cell with the helper plasmid or the two-plasmid system of any one of claims 1 to 12; and
(c) culturing the host cell under conditions suitable for recombinant AAV production.
